# EUROPEAN PATENT SPECIFICATION

(11) **EP 2 912 462 B1**
(45) Date of publication and mention of the grant of the patent: **07.08.2019**
(21) Application number: 13848471.2
(22) Date of filing: 23.10.2013
(51) Int. Cl.: C12Q 1/68, G01N 33/53, G01N 33/68, C07K 14/705, G01N 33/72, G01N 33/74

(54) **BIOMARKER FOR USE IN TREATING ANEMIA**
BIOMARKER ZUR VERWENDUNG BEI DER BEHANDLUNG VON ANÄMIE
BIOMARQUEUR UTILISABLE DANS LE TRAITEMENT DE L'ANÉMIE

(30) Priority: 24.10.2012 US 201261718126 P
(43) Date of publication of application: 02.09.2015
(60) Divisional application: 19184182.4
(73) Proprietor: Celgene Corporation, Summit, NJ 07901 (US)
(72) Inventor: SUNG, Victoria, San Francisco, CA 94114 (US); CHOPRA, Rajesh, London SW15 1LE (GB); HERMINE, Olivier, 91120 Palaiseau (FR); CRUZ MOURA, Ivan, 75015 Paris (FR); DUSSIOT, Michael, 92320 Chatillon (FR); TROVATI MACIEL, Thiago, 75020 Paris (FR); FRICOT, Aurelie, 93100 Montreuil (FR)
(74) Representative: Jones Day
(86) International application number: PCT/US2013/066350
(87) International publication number: WO 2014/066486

(56) References cited:
- EP-A1- 2 446 896
- WO-A1-2011/020045
- WO-A2-2009/158025
- US-A- 5 914 234
- US-A1- 2002 150 577
- US-A1- 2010 008 918
- US-A1- 2010 015 144
- US-A1- 2011 020 372
- US-A1- 2011 038 831
- US-A1- 2011 092 670
- US-A1- 2011 281 796
- US-A1- 2012 015 877
- US-A1- 2012 094 908
- US-A1- 2012 121 576
- US-A1- 2012 237 521
- US-A1- 2012 252 884
- US-B2- 8 058 229
- DATABASE BIOSIS [Online] BIOSCIENCES INFORMATION SERVICE, PHILADELPHIA, PA, US; 16 November 2012 (2012-11-16), CARRANCIO SORAYA ET AL: "Sotatercept, an Activin Receptor IIa Ligand Trap, Acts Through Bone Marrow Accessory Cells to Promote Late-Stage Erythropoiesis and a Rapid Induction of Red Blood Cell Number and Hemoglobin", XP002757056, Database accession no. PREV201300232414
- TANNO ET AL.: 'High levels of GDF15 in thalassemia suppress expression of the iron regulatory protein hepcidin.' NAT MED. vol. 13, no. 9, 01 September 2007, pages 1096 - 1101, XP055256006
- MCPHERRON: 'Metabolic functions of myostatin and GDF11.' IMMUNOL. ENDOCR. METAB. AGENTS MED. CHEM. vol. 10, no. 4, 2010, pages 217 - 231, XP055200150
- LI ET AL.: 'Transgenic overexpression of bone morphogenetic protein 11 propeptide in skeleton enhances bone formation.' BBRC vol. 416, no. 3-4, 18 October 2011, pages 289 - 292, XP028342313
- SAKO ET AL.: 'Characterization of the ligand binding functionality of the extracellular domain of activin receptor type IIb.' J BIOL CHEM. vol. 285, no. 27, 02 July 2010, pages 21037 - 21048, XP008148640
- 'A Phase 2, Open-Label, Pharmacodynamic Study to Evaluate the Effect of Sotatercept (ACE-011) on Red Blood Cell Mass and Plasma Volume in Subjects With Solid Tumors.' NCT01190644 14 August 2012, XP055257547 Retrieved from the Internet: <URL:http://clinicaltrials.gov/archive/NCT0 1190644/2012_08_14> [retrieved on 2013-12-17]
- 'A Phase-2, Prospective, Open-Label Study to Determine the Safety and Efficacy of Sotatercept (ACE-011) in Subjects With Myeloproliferative Neoplasm (MPN) - Associated Myelofibrosis and Anemia' CLINICAL TRIALS, NCT01712308, [Online] 22 October 2012, XP055265729 Retrieved from the Internet: <URL:HTTP://CLINICALTRIALS.GOV/ARCHIVE/NCT0 1712308/2012_10_22> [retrieved on 2013-12-17]
- BARON ET AL.: 'Update on Bone Anabolics in Osteoporosis Treatment: Rationale, Current Status, and Perspectives.' JOURNAL OF CLINICAL ENDOCRINOLOGY & METABOLISM vol. 97, no. 2, February 2012, pages 311 - 325, XP055256085
- MAKANJI ET AL.: 'Generation of a Specific Activin Antagonist by Modification of the Activin A Propeptide.' ENDOCRINOLOGY vol. 152, no. 10, October 2011, pages 3758 - 3768, XP055256115
- STEINBICKER ET AL.: 'Inhibition of bone morphogenetic protein signaling attenuates anemia associated with inflammation.' BLOOD vol. 117, no. 18, 05 May 2011, pages 4915 - 4923, XP055256123
- MARKS-BLUTH ET AL.: 'Cell signalling pathways that mediate haematopoietic stem cell specification.' INT J BIOCHEM CELL BIOL. vol. 44, no. 12, 17 September 2012, pages 2175 - 2184, XP055256134
- MULIVOR ET AL.: 'RAP-011, a soluble activin receptor type IIa murine IgG-Fc fusion protein, prevents chemotherapy induced anemia' BLOOD vol. 114, no. 22, 2009, page 161, XP055265735 Retrieved from the Internet: <URL:https://ash.confex.com/ash/2009/webpro gram/Paper23536.html> [retrieved on 2012-12-18]
- LONGO ET AL.: 'Therapeutic approaches to myeloma bone disease: an evolving story.' CANCER TREAT REV. vol. 38, no. 6, 09 April 2012, pages 787 - 797, XP028511955
- YAMAWAKI ET AL.: 'Adult-Specific Systemic Over-Expression Reveals Novel In Vivo Effects of the Soluble Forms of ActRIIA, ActRIIB and BMPRII.' PLOS ONE vol. 8, no. 10, 21 October 2013, page E78076, XP055265740
- CARRANCIO ET AL.: 'SOTATERCEPT, AN ACTIVIN RECEPTOR IIA LIGAND TRAP, ACTS THROUGH BONE MARROW ACCESSORY CELLS TO PROMOTE LATE-STAGE ERYTHROPOIESIS AND A RAPID INDUCTION OF RED BLOOD CELL NUMBER AND HEMOGLOBIN' 54TH ASH ANNUAL MEETING AND EXPOSITION 10 December 2012, ATLANTA, GA, USA, XP055265762 Retrieved from the Internet: <URL:https://ash.confex.com/ash/2012/webpro gram/Paper49571.html> [retrieved on 2013-12-17]
- CHEN ET AL.: 'EXPOSURES AND ERYTHROPOIETIC RESPONSES TO SOTATERCEPT (ACE-011) IN HEALTHY VOLUNTEERS AND CANCER PATIENTS: IMPLICATIONS FOR MECHANISM OF ACTION' 54TH ASH ANNUAL MEETING AND EXPOSITION 10 December 2012, XP055265770 Retrieved from the Internet: <URL:https://ash.confex.com/ash/2012/webpro gram/Paper52788.html> [retrieved on 2012-12-18]

## Description

### 1. INTRODUCTION

Provided herein is an activin receptor type II inhibitor for use in methods of treating diseases associated with anemia or ineffective erythropoiesis by using the level and/or activity of a ligand of an activin type II receptor, in particular, Growth differentiation factor 11 (GDF11), as an indicator of responsiveness of a patient to the treatment, efficacy of the treatment, or appropriate dosage for the treatment with an activin type II receptor inhibitor. For example, the methods could be determining the level and/or activity of GDF11, selecting patients having elevated level and/or activity of GDF11, and treating patients having elevated level and/or activity of GDF11 with an activin type II receptor inhibitor. Further the methods comprise determining the level and/or activity of GDF11 in a patient treated with an activin type II receptor inhibitor, and administering the activin type II receptor inhibitor in a dose amount depending on the level and/or activity of GDF11 in the patient. Such activin type II receptor inhibitor can be an inhibitor of ActRIIA and / or ActRIIB signaling (e.g., a humanized fusion-protein consisting of the extracellular domain of activin-receptor type IIA and the human IgG1 Fc domain).

### 2. BACKGROUND

Anemia is a decrease in number of red blood cells or less than the normal quantity of hemoglobin in the blood. Anemia can also be caused by decreased oxygen-binding ability of the hemoglobin. Anemia is the most common disorder of the blood.

Anemia can be caused by ineffective erythropoiesis. Ineffective erythropoiesis is present if active erythropoiesis takes place but mature red blood cells fail to develop at the proper rate. Progenitor cells undergo apoptosis before the stage of mature red blood cells is reached.

Thalassemia is a form of ineffective erythropoiesis. In thalassemia, ineffective erythropoiesis is characterized by apoptosis of the maturing nucleated erythroid cells. Specifically beta-thalassemia is a disease characterized by defects in Hemoglobin (or Hgb) synthesis leading to impaired erythrocyte maturation and production. The decrease is primarily thought to be due to abnormally accelerated erythroid differentiation and apoptosis at the late basophilic/polychromatic erythroblast stage of red blood cell differentiation, resulting in an overall decrease in mature red blood cell production. beta-thalassemia is characterized by a hypercellular bone marrow compartment where abnormal erythroblasts accumulate and undergo apoptosis, resulting in systemic anemia.

The transforming growth factor beta (TGF-beta) family, which include TGF-beta, activins, bone morphogenic proteins (BMPs) and growth and differentiation factors (GDFs) are secreted proteins known to regulate numerous cellular processes during both development and tissue homeostasis. TGF-beta1, activin A, BMP-2 and BMP-4 have all been associated with the regulation of erythropoiesis in various model systems. TGF-beta1 both inhibits and promotes erythroid differentiation depending on the context, activin A was shown to be a pro-erythroid differentiation agent, while BMP-4 has been implicated in stress erythropoiesis and recovery from acute anemia in murine models. BMP-2 acts on early erythroid cells to increase colony formation in samples obtained from mobilized peripheral blood or bone marrow CD34+ cells. Abnormally high levels of some of these growth factors have been associated with various hematologic diseases. For example, high levels of GDF-15 are not usually a feature of normal erythropoiesis but in conditions of ineffective erythropoiesis, GDF-15 expression is elevated.

The TGF-beta superfamily consists of more than 30 proteins and they signal through only a limited number of receptors and signaling pathways, pointing to inherent promiscuity and redundancy in their actions. Furthermore, in any given tissue, several different ligands may be found and signaling presumably occurs via an overlapping subset of receptors complicating the ability to associate particular ligands to their function. GDF11 is a member of the GDF subfamily and shares about 90% amino acid homology with GDF8, also known as myostatin. Both can bind the activin type IIA and B receptors and activate the Smad 2/3 signaling pathway. GDF11 plays a big role in development, taking part in the formation of muscle, cartilage, bone, kidney, and nervous system while in adult tissues, GDF11 was detected in the pancreas, intestine, kidney, skeletal muscle, brain and dental pulp. Low amounts of GDF-11 can be also found in the circulation. To this date, however, there is no evidence describing a role for GDF-11 in erythropoiesis.

Type II receptors for activins can also bind GDF11. Two related type II receptors, ActRIIa and ActRIIb, have been identified (Mathews and Vale, 1991, Cell 65:973-982; Attisano et al., 1992, Cell 68: 97-108). Besides GDF11, ActRIIa and ActRIIb can biochemically interact with several other TGF-beta family proteins, including BMP7, Nodal, GDF8, and activin (Yamashita et al., 1995, J. Cell Biol. 130:217-226; Lee and McPherron, 2001, Proc. Natl. Acad. Sci. 98:9306-9311; Yeo and Whitman, 2001, Mol. Cell 7: 949-957; Oh et al., 2002, Genes Dev. 16:2749-54). ALK4 is the primary type I receptor for activins, particularly for activin A, and ALK-7 may serve as a receptor for activins as well, particularly for activin B.

WO 2011/020045 A1 discloses a combined use of GDF traps and erythropoietin receptor activators to increase red blood cell levels.

WO 2009/158025 A2 discloses methods for dosing an anti-Activin receptor IIB (ActRIIB) antagonist and monitoring of patients treated therewith.

US 2012/237521 A1 discloses the use of anti-ActRIIB antibodies for treating muscle disorders, such as muscle wasting due to a disease or disuse.

A humanized fusion-protein consisting of the extracellular domain of activin-receptor type IIA (ActRIIA) and the human IgG1 Fc (ActRIIA-hFc) is currently being evaluated in phase II clinical trials for treatment of patients with anemia and bone disorders associated with end-stage renal disease (ESRD) as well as for those patients with beta-thalassemia. In healthy postmenopausal women, ActRIIA-hFc was shown to significantly increase hematocrit (Hct) and hemoglobin (Hgb) as well as bone mineral density. However, the identity of ActRIIA ligands sequestered by ActRIIA-hFc in the bloodstream remains unknown.

### 3. SUMMARY

The invention provides an activin receptor type II inhibitor for use in a method for treatment of anemia, beta thalassemia, or for increasing red blood cell levels or orthochromatic erythroblasts (Ery-C) in a subject in need thereof, wherein the method comprises: (a) assessing the level and/or activity of GDF11 in a tissue sample of the subject, and (b) if the level and/or activity of GDF11 is elevated over the normal level and/or activity of GDF11, administering an activin receptor type II inhibitor to the subject.

The invention also provides an activin receptor type II inhibitor for use in a method for treatment of anemia, beta thalassemia, or for increasing red blood cell levels or orthochromatic erythroblasts (Ery-C) in a subject in need thereof, wherein the method comprises: (a) administering an activin receptor type II inhibitor, and (b) monitoring the level and/or activity of GDF11 in a tissue sample of the subject.

Within the boundary of the invention, the activin receptor type II inhibitor is a polypeptide comprising an amino acid sequence selected from the group consisting of:

| | |
|---|---|
| a | 90% identical to SEQ ID NO:2; |
| b | 95% identical to SEQ ID NO:2; |
| c | 98% identical to SEQ ID NO:2; |
| d | SEQ ID NO:2; |
| e | 90% identical to SEQ ID NO:3; |
| f | 95% identical to SEQ ID NO:3; |
| g | 98% identical to SEQ ID NO:3; |
| h | SEQ ID NO:3; |
| i | 90% identical to SEQ ID NO:6; |
| j | 95% identical to SEQ ID NO:6; |
| k | 98% identical to SEQ ID NO:6; |
| l | SEQ ID NO:6; |
| m | 90% identical to SEQ ID NO:7; |
| n | 95% identical to SEQ ID NO:7; |
| o | 98% identical to SEQ ID NO:7; |
| p | SEQ ID NO:7; |
| q | 90% identical to SEQ ID NO:12; |
| r | 95% identical to SEQ ID NO:12; |
| s | 98% identical to SEQ ID NO:12; |
| t | SEQ ID NO:12; |
| u | 90% identical to SEQ ID NO:17; |
| v | 95% identical to SEQ ID NO:17; |
| w | 98% identical to SEQ ID NO:17; |
| x | SEQ ID NO:17; |
| y | 90% identical to SEQ ID NO:20; |
| z | 95% identical to SEQ ID NO:20; |
| aa | 98% identical to SEQ ID NO:20; |
| bb | SEQ ID NO:20; |
| cc | 90% identical to SEQ ID NO:21; |
| dd | 95% identical to SEQ ID NO:21; |
| ee | 98% identical to SEQ ID NO:21; |
| ff | SEQ ID NO:21 |
| gg | 90% identical to SEQ ID NO:22; |
| hh | 95% identical to SEQ ID NO:22; |
| ii | 98% identical to SEQ ID NO:22; |
| JJ | SEQ ID NO:22; |
| kk | 90% identical to SEQ ID NO:23; |
| ll | 95% identical to SEQ ID NO:23; |
| mm | 98% identical to SEQ ID NO:23; |
| nn | SEQ ID NO:23; |
| oo | 90% identical to SEQ ID NO:24; |
| pp | 95% identical to SEQ ID NO:24; |
| qq | 98% identical to SEQ ID NO:24; |
| rr | SEQ ID NO:24; |
| ss | 90% identical to SEQ ID NO:25; |
| tt | 95% identical to SEQ ID NO:25; |
| uu | 98% identical to SEQ ID NO:25; and |
| vv | SEQ ID NO:25; |

or
wherein the activin receptor type II inhibitor is a polypeptide comprising the extracellular domain of activin-receptor type IIA linked to the Fc portion of an antibody;
or
wherein the activin receptor type II inhibitor is a polypeptide comprising the extracellular domain of activin-receptor type IIB linked to the Fc portion of an antibody.

Hereinafter, reference to the activin receptor type II inhibitor in the context of the invention or its embodiments means the activin receptor type II inhibitor as defined above.

In one aspect, provided herein is an activin receptor type II inhibitor for use in methods for treating anemia, or a blood disorder associated with anemia or ineffective erythropoiesis, using GDF11 as an indicator of responsiveness of a patient to the treatment, efficacy of the treatment, or appropriate dosage for the treatment of the patient with an activin type II receptor inhibitor.

In certain embodiments, the method comprises determining the level and/or activity of GDF11 in a tissue sample of a patient having anemia, or a disorder, e.g., a blood disorder associated with anemia or ineffective erythropoiesis. In some embodiments, the method comprises determining the level and/or activity of GDF11 in a tissue sample of a patient having anemia, or a disorder, e.g., a blood disorder associated with anemia or ineffective erythropoiesis, and comparing it to the normal level and/or activity of GDF11 (e.g., the average level and/or activity of GDF11 in a sample from the same tissue of a healthy subject or subjects). In some embodiments, the method comprises determining the level and/or activity of GDF11 in a tissue sample (such as serum/plasma, blood, bone marrow, spleen, or liver) of a patient having anemia, or a disorder, e.g., a blood disorder associated with anemia or ineffective erythropoiesis, and comparing it to the level and/or activity of GDF11 in a sample from a different tissue of the subject (such as a tissue in which the level of GDF11 is not elevated in patients having anemia).

In certain embodiments, provided herein is an activin receptor type II inhibitor for use in a method for treating anemia, or a blood disorder associated with anemia or ineffective erythropoiesis, wherein the method comprises: (a) assessing the level and/or activity of GDF11 in a tissue sample of the subject, and (b) if the level and/or activity of GDF11 is elevated over the normal level and/or activity of GDF11, administering an activin receptor type II inhibitor to the subject. In some embodiments, provided herein is an activin receptor type II inhibitor for use in a method for treating anemia, or a blood disorder associated with anemia or ineffective erythropoiesis, wherein the method comprises: (a) administering an activin receptor type II inhibitor, and (b) monitoring the level and/or activity of GDF11 in a tissue sample of the subject. In certain embodiments, provided herein is an activin receptor type II inhibitor for use in a method for treating anemia, or a blood disorder associated with anemia or ineffective erythropoiesis, wherein the method comprises: (a) administering a dose amount of an activin receptor type II inhibitor, (b) assessing the level and/or activity of GDF11 in a tissue sample of the subject, and (c) adjusting the dose amount of the activin receptor type II inhibitor wherein, if the level and/or activity of GDF11 is elevated over the normal level and/or activity of GDF11, the dose amount of the activin receptor type II inhibitor is increased, and wherein, if the level and/or activity of GDF11 is decreased over the normal level and/or activity of GDF11, the dose amount of the activin receptor type II inhibitor is decreased. The blood disorders associated with anemia or ineffective erythropoiesis treated in accordance with the methods described herein include, without limitation, thalassemia (e.g., beta thalassemia), myelodysplastic syndrome, chronic pernicious anemia, sickle cell anemia, Diamond Blackfan anemia, decreased red blood cell levels, or decreased orthochromatic erythroblasts (Ery-C). In a preferred embodiment, the subject being treated in accordance with the methods described herein is a human.

In certain embodiments, provided herein is an activin receptor type II inhibitor for use in a method for treating anemia in a subject in need of treatment, wherein the method comprises: (a) assessing the level and/or activity of GDF11 in a tissue sample of the subject, and (b) if the level and/or activity of GDF11 is elevated over the normal level and/or activity of GDF11, administering an activin receptor type II inhibitor to the subject.

Further, in some embodiments, provided herein is an activin receptor type II inhibitor for use in a method for treating thalassemia (e.g., beta thalassemia) in a subject in need of treatment, wherein the method comprises: (a) assessing the level and/or activity of GDF11 in a tissue sample of the subject, and (b) if the level and/or activity of GDF11 is elevated over the normal level and/or activity of GDF11, administering an activin receptor type II inhibitor to the subject. In particular embodiments, provided herein is an activin receptor type II inhibitor for use in a method for treating thalassemia (e.g., beta thalassemia) in a subject in need of treatment, wherein the method comprises: (a) administering a dose amount of an activin receptor type II inhibitor, (b) assessing the level and/or activity of GDF11 in a tissue sample of the subject, and (c) adjusting the dose amount of the activin receptor type II inhibitor wherein, if the level and/or activity of GDF11 is elevated over the normal level and/or activity of GDF11, the dose amount of the activin receptor type II inhibitor is increased, and wherein, if the level and/or activity of GDF11 is decreased over the normal level and/or activity of GDF11, the dose amount of the activin receptor type II inhibitor is decreased.

In some embodiments, provided herein is an activin receptor type II inhibitor for use in a method for increasing red blood cell levels or orthochromatic erythroblast (Ery-C) levels in a subject in need thereof, wherein the method comprises: (a) assessing the level and/or activity of GDF11 in a tissue sample of the subject, and (b) if the level and/or activity of GDF11 is elevated over the normal level and/or activity of GDF11, administering an activin receptor type II inhibitor to the subject. In other embodiments, provided herein is an activin receptor type II inhibitor for use in a method for increasing red blood cell levels or orthochromatic erythroblast (Ery-C) levels in a subject in need thereof, wherein the method comprises: (a) administering a dose amount of an activin receptor type II inhibitor, (b) assessing the level and/or activity of GDF11 in a tissue sample of the subject, and (c) adjusting the dose amount of the activin receptor type II inhibitor wherein, if the level and/or activity of GDF11 is elevated over the normal level and/or activity of GDF11, the dose amount of the activin receptor type II inhibitor is increased, and wherein, if the level and/or activity of GDF11 is decreased over the normal level and/or activity of GDF11, the dose amount of the activin receptor type II inhibitor is decreased.

In specific embodiments, administering of an activin receptor type II inhibitor in accordance with the methods described herein results in a decreased cell count of late basophilic and polychromatic erythroblasts (Ery-B) in the patient. In some embodiments, said administering results in a decreased level of bilirubin (e.g., serum level of bilirubin) in the patient. In some embodiments, said administering results in a decreased level of lactate dehydrogenase (e.g., serum level of lactate dehydrogenase) in the patient. In some embodiments, said administering results in decreased bone marrow and/or spleen cellularity in the patient. In some embodiments, said administering results in decreased reticulocyte counts in the patient. In some embodiments, said administering results in decreased transferring saturation and/or decreased systemic iron levels in the patient. In preferred embodiments, administering of an activin receptor type II inhibitor in accordance with the methods described herein results in increased red blood cell counts, increased hematocrit levels and/or increased hemoglobin levels in the patient. In particular embodiments, administering of an activin receptor type II inhibitor in accordance with the methods described herein results in increased mean corpuscular volume (MCV), mean corpuscular hemoglobin (MCH), and/or MCH concentration in the patient. In certain embodiments, such results are assessed in the subject in addition to assessment of the level and/or activity of GDF11 in the subject.

In certain embodiments, the ActRII inhibitor that can be used in the methods provided herein is a polypeptide comprising an amino acid sequence selected from the group consisting of: 90% identical to SEQ ID NO:2; 95% identical to SEQ ID NO:2; 98% identical to SEQ ID NO:2; SEQ ID NO:2; 90% identical to SEQ ID NO:3; 95% identical to SEQ ID NO:3; 98% identical to SEQ ID NO:3; SEQ ID NO:3; 90% identical to SEQ ID NO:6; 95% identical to SEQ ID NO:6; 98% identical to SEQ ID NO:6; SEQ ID NO:6; 90% identical to SEQ ID NO:7; 95% identical to SEQ ID NO:7; 98% identical to SEQ ID NO:7; SEQ ID NO:7; 90% identical to SEQ ID NO:12; 95% identical to SEQ ID NO:12; 98% identical to SEQ ID NO:12; SEQ ID NO:12; 90% identical to SEQ ID NO:17; 95% identical to SEQ ID NO:17; 98% identical to SEQ ID NO:17; SEQ ID NO:17; 90% identical to SEQ ID NO:20; 95% identical to SEQ ID NO:20; 98% identical to SEQ ID NO:20; SEQ ID NO:20; 90% identical to SEQ ID NO:21; 95% identical to SEQ ID NO:21; 98% identical to SEQ ID NO:21; and SEQ ID NO:21. In some embodiments, the ActRII inhibitor is a humanized fusion-protein consisting of the extracellular domain of ActRIIA and the human IgG1 Fc domain. In a more specific embodiment, the ActRII inhibitor is a polypeptide comprising the amino acid sequence of SEQ ID NO:7. In one embodiment, the ActRII inhibitor is administered parenterally.

In certain embodiments, the normal level and/or activity of GDF11 is an average level and/or activity of GDF11 in a tissue sample of one or more healthy subjects (e.g., healthy subjects in the same age category and/or of the same gender). In some embodiments, the level and/or activity of GDF11 in a tissue sample of the treated subject is compared to the level and/or activity of GDF11 in a sample from the same tissue of one or more healthy subjects. In some embodiments, the tissue in which the level and/or activity of GDF11 is assessed is whole blood, blood serum/plasma, bone marrow, liver, or spleen. In one embodiment, the tissue in which the level and/or activity of GDF11 is assessed is serum.

The level and/or activity of GDF11 can be measured by any method known in the art or described herein. In one embodiment, the level and/or activity of GDF11 is the protein level of GDF11. In one embodiment, the level and/or activity of GDF11 is mRNA level of GDF11.

In certain embodiments, the level and/or activity of GDF11 is deemed to be elevated over the normal level and/or activity of GDF11 when it is elevated at least 10%, at least 25%, at least 30%, at least 40%, at least 50%, at least 60%, at least 75%, at least 80%, at least 90%, at least 100%, at least 200%, at least 300%, or at least 500% over the normal level and/or activity of GDF11. In certain embodiments, the level and/or activity of GDF11 is deemed to be elevated over the normal level and/or activity of GDF11 when it is elevated at least 25%, at least 50%, at least 75%, at least 100%, or at least 200% over the normal level and/or activity of GDF11. In a particular embodiment, the level and/or activity of GDF11 is deemed to be elevated over the normal level and/or activity of GDF11 when it is elevated at least 50% over the normal level and/or activity of GDF11. In a particular embodiment, the level and/or activity of GDF11 is deemed to be elevated over the normal level and/or activity of GDF11 when it is elevated at least 100% over the normal level and/or activity of GDF11. In a particular embodiment, the level and/or activity of GDF11 is deemed to be elevated over the normal level and/or activity of GDF11 when it is elevated at least 200% over the normal level and/or activity of GDF11. In one embodiment, the level and/or activity of GDF11 is deemed to be elevated over the normal level and/or activity of GDF11 when it is elevated at least 1.5 or 2 times over the normal level and/or activity of GDF11. In some embodiments, the level and/or activity of GDF11 is deemed to be decreased over the normal level and/or activity of GDF11 when it is decreased at least 10%, at least 20%, at least 25%, at least 30%, at least 40%, at least 50%, at least 60%, at least 70%, at least 75%, at least 80%, or at least 90% over the normal level and/or activity of GDF11. In some embodiments, the level and/or activity of GDF11 is deemed to be decreased over the normal level and/or activity of GDF11 when it is decreased at least 25%, at least 50%, at least 75%, or at least 90% over the normal level and/or activity of GDF11. In one embodiment, the level and/or activity of GDF11 is deemed to be decreased over the normal level and/or activity of GDF11 when it is decreased at least 25% over the normal level and/or activity of GDF11. In one embodiment, the level and/or activity of GDF11 is deemed to be decreased over the normal level and/or activity of GDF11 when it is decreased at least 50% over the normal level and/or activity of GDF11. In one embodiment, the level and/or activity of GDF11 is deemed to be decreased over the normal level and/or activity of GDF11 when it is decreased at least 75% over the normal level and/or activity of GDF11. In one embodiment, the level and/or activity of GDF11 is deemed to be decreased over the normal level and/or activity of GDF11 when it is decreased at least 1.5 or 2 times over the normal level and/or activity of GDF11.

### 4. BRIEF DESCRIPTION OF THE FIGURES

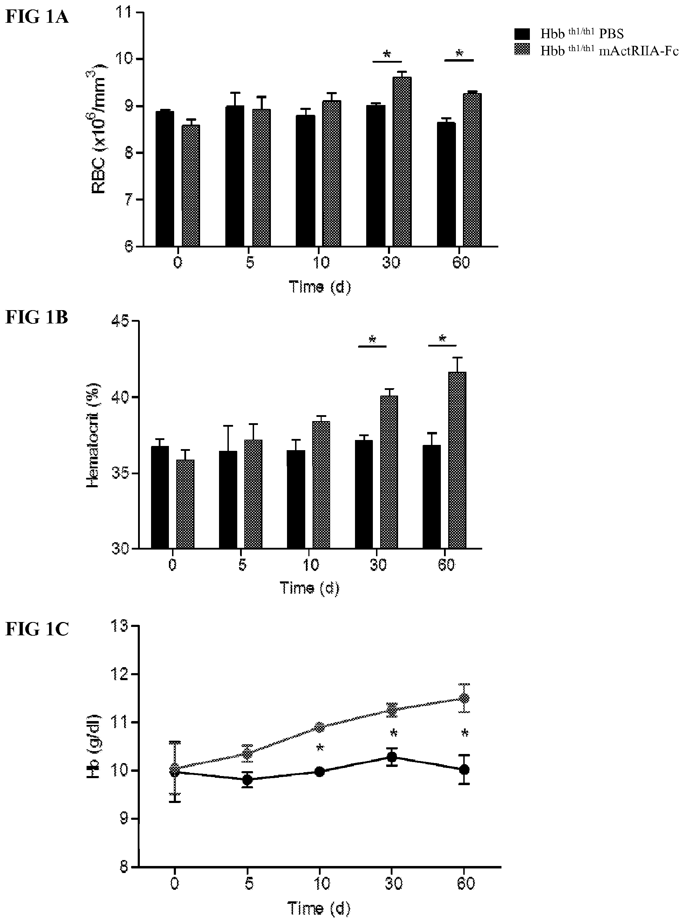

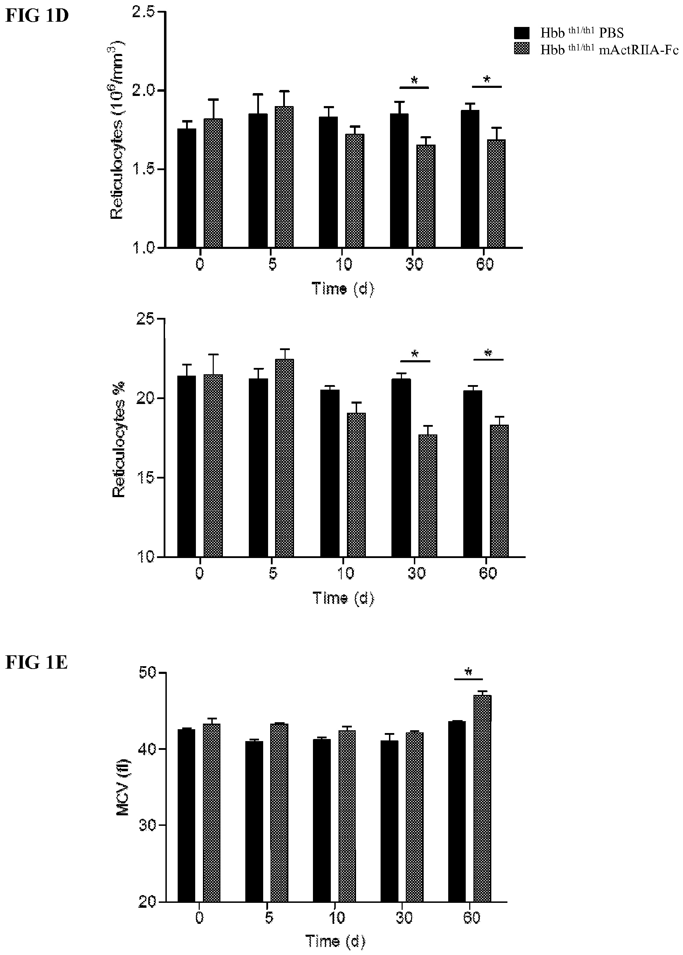

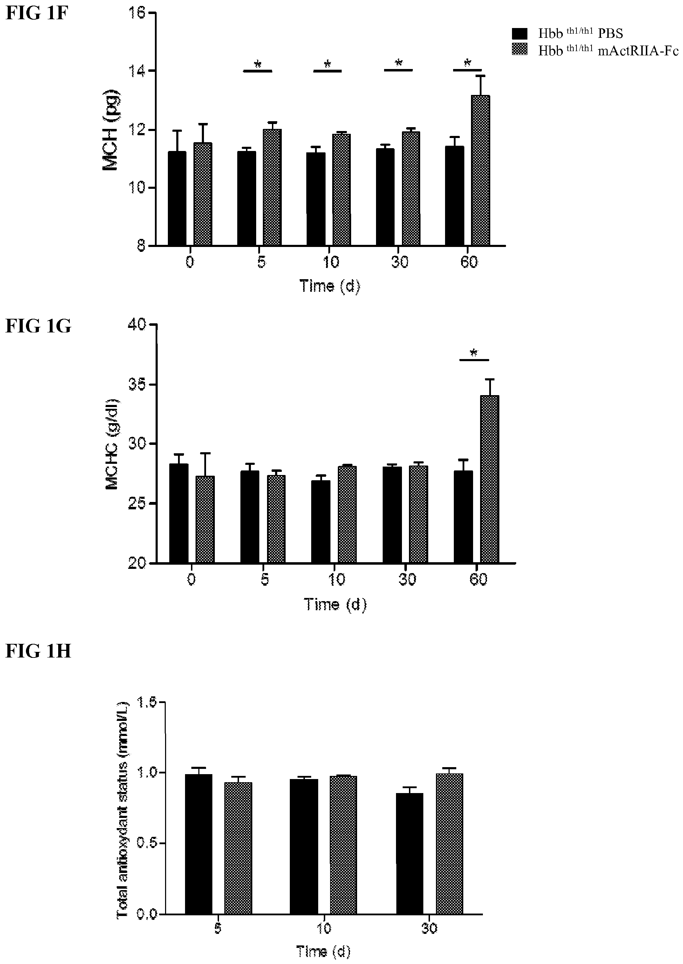

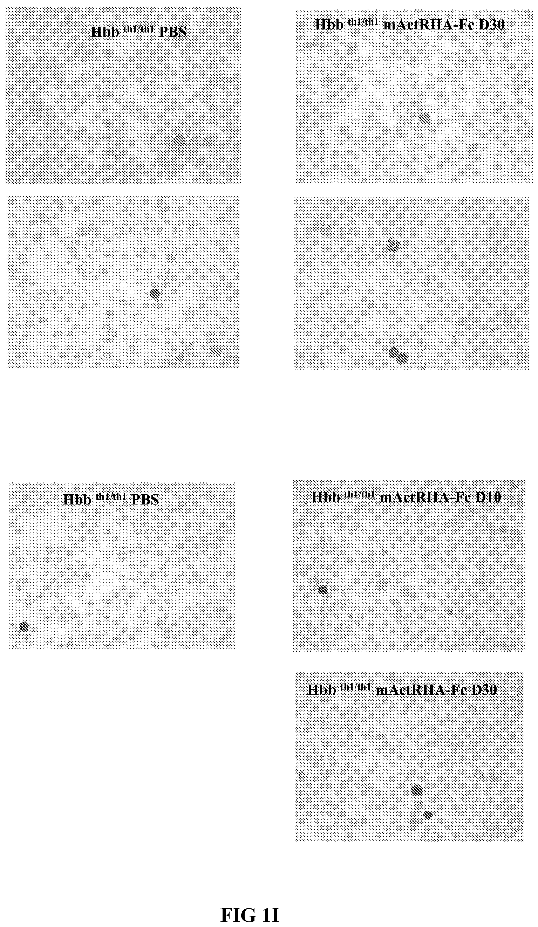

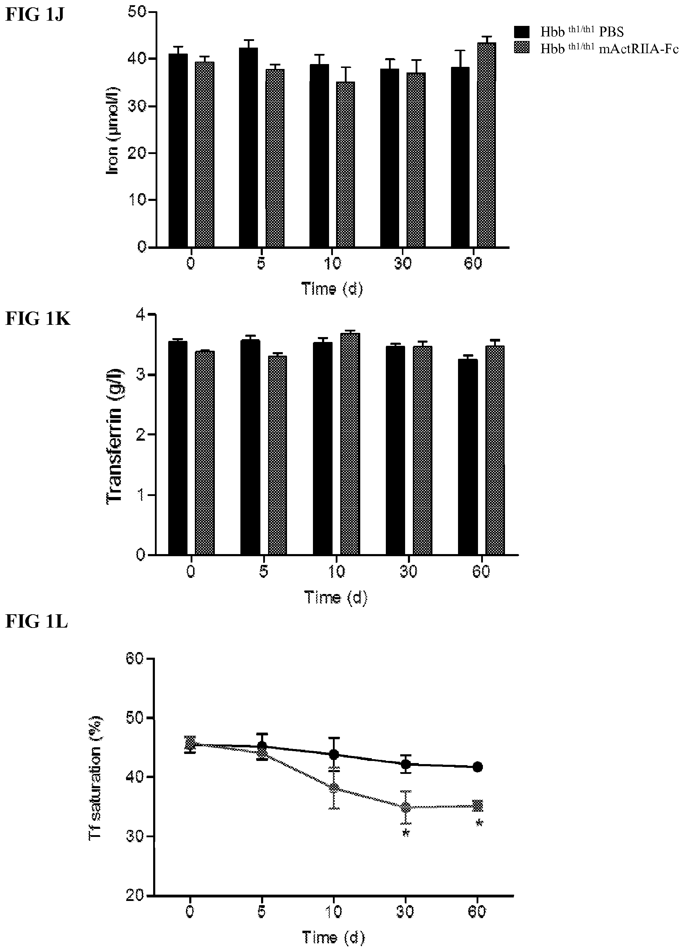

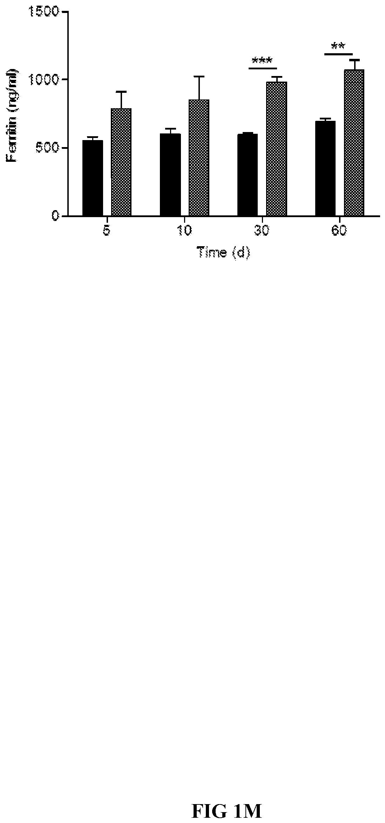

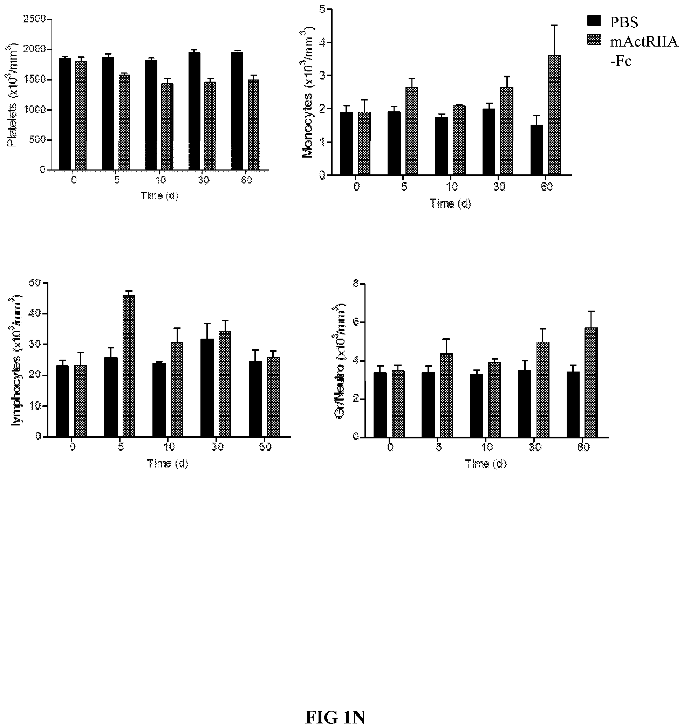

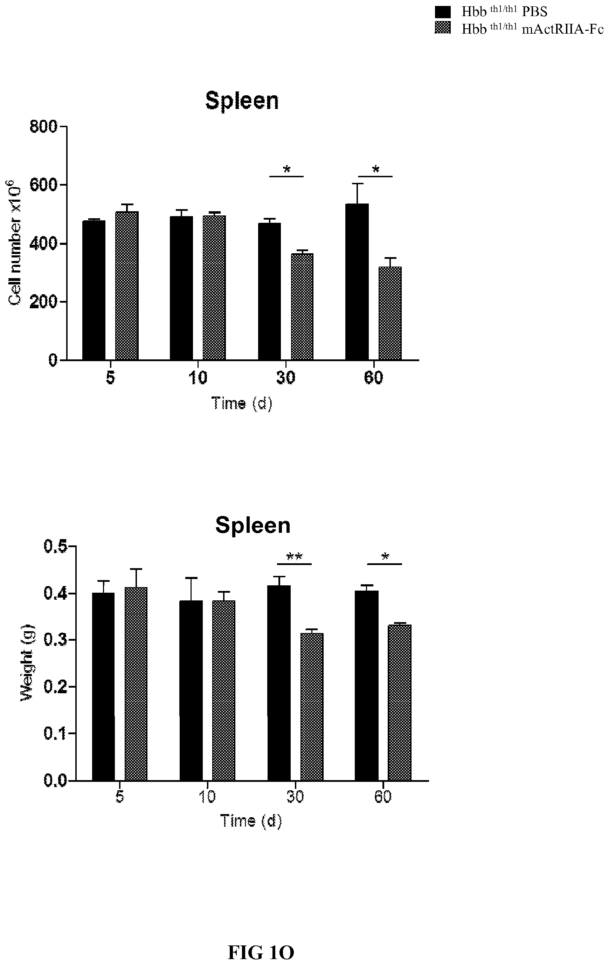

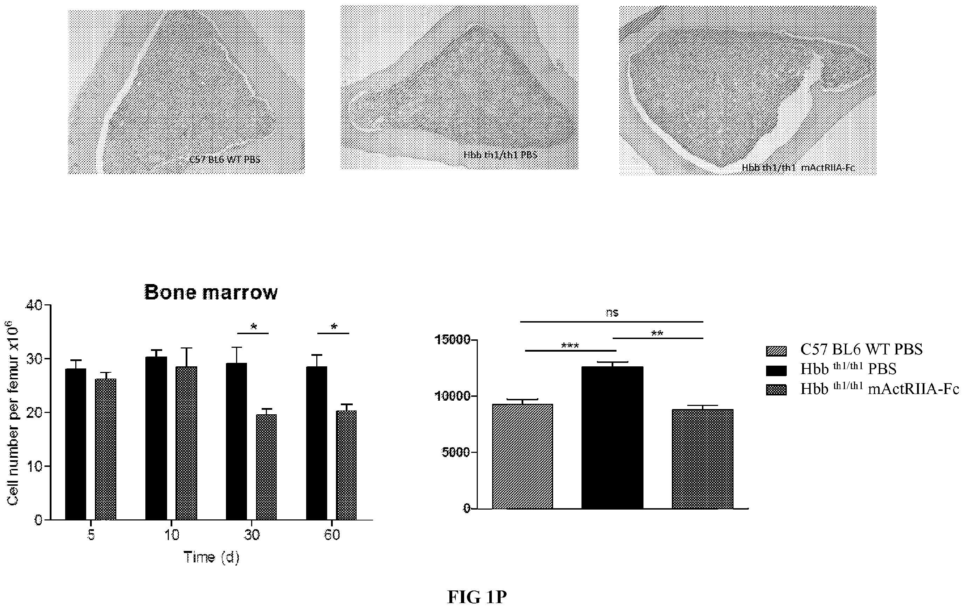

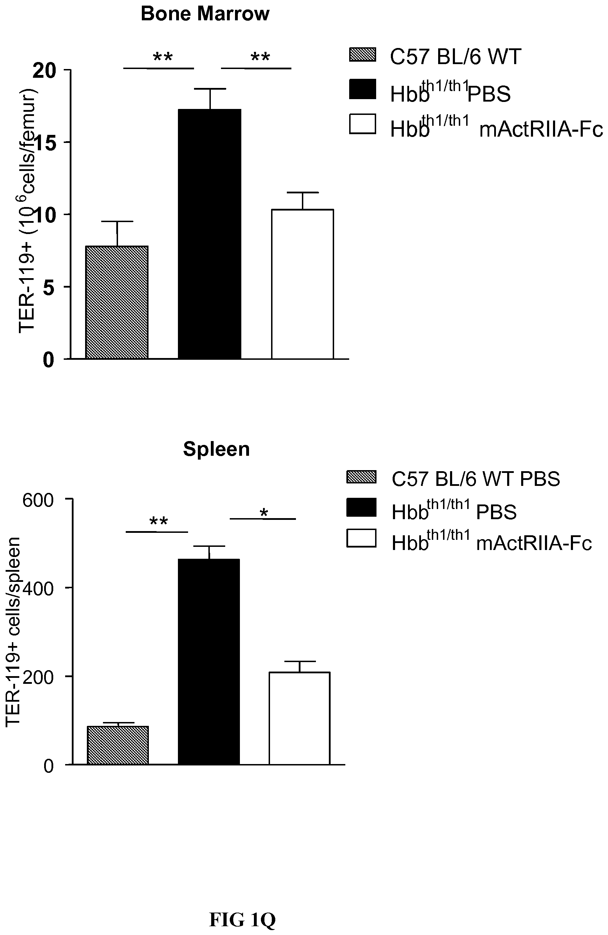

FIG. 1 illustrates that the murine counterpart of SEQ ID NO 7 (mActRIIA-Fc) ameliorates hematological parameters in β-thalassemic mice. Hbb^{th1/th1} mice (Skow LC et al., Cell 34:1043-52, 1983) were treated with PBS or mActRIIA-Fc (10 mg/Kg body weight twice a week) for 60 days. Hematological parameters were evaluated on days 5, 10, 30 and 60. Evaluation of (A) red blood cell counts, (B) hematocrit and (C) hemoglobin was associated with a decreased (D) reticulocytosis. Analysis of circulating red blood cells (RBC) parameters also show that (E) mean corpuscular volume (MCV), (F) mean corpuscular hemoglobin (MCH) and (G) MCH concentration (MCHC) increased in mice treated with mActRIIA-Fc. (H) Total antioxidant status. (I) Morphological analysis showed a reduction in anisocytosis, poikilocytosis and target cells. (J) Systemic iron levels, (K) transferrin synthesis, (L) transferrin and (M) ferritin levels saturation were also evaluated on thalassemic mice. (N) Inflammatory cell counts were also assessed. Effects of mActRIIA-Fc on splenomegaly in thalassemic mice evaluated by (O) spleen weight and total cell numbers. (P) Bone marrow erythroblasts numbers and expansion (eosin/hematoxylin staining) were also decreased in mice treated with mActRIIA-Fc. (Q) Bone marrow and spleen erythroblasts were quantified by flow cytometry by TER119 staining. * p<0.05, N=3-5 for each independent experiment.

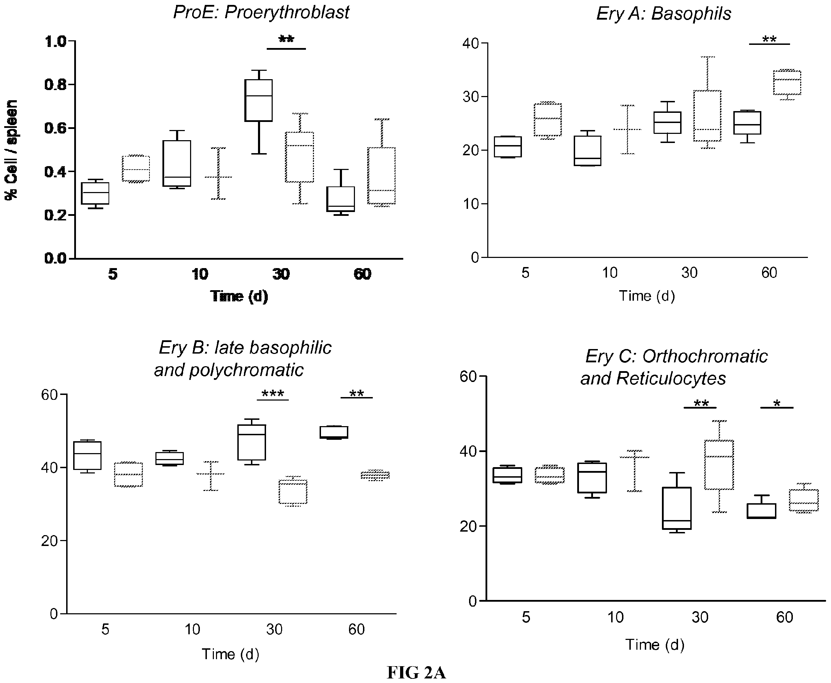

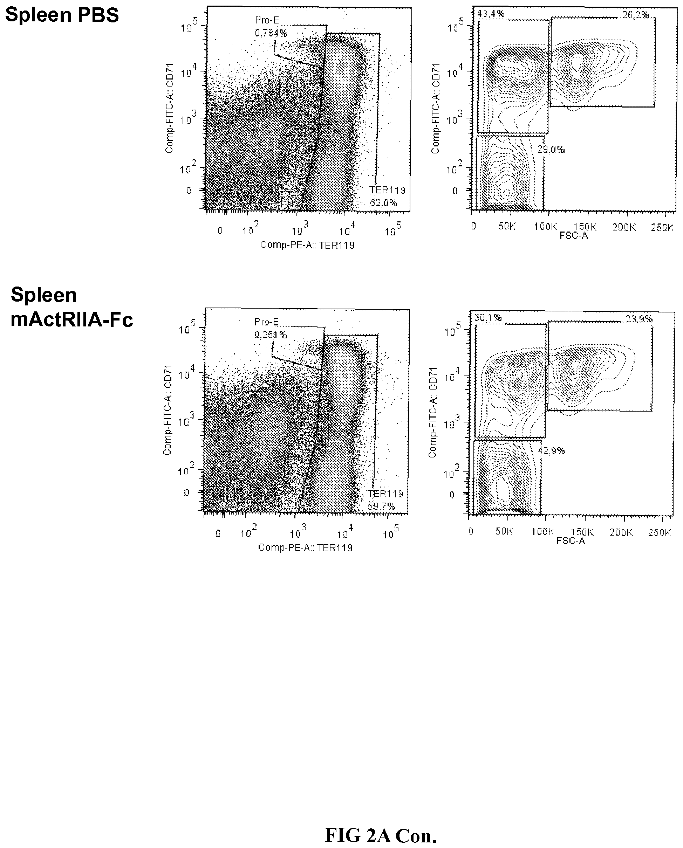

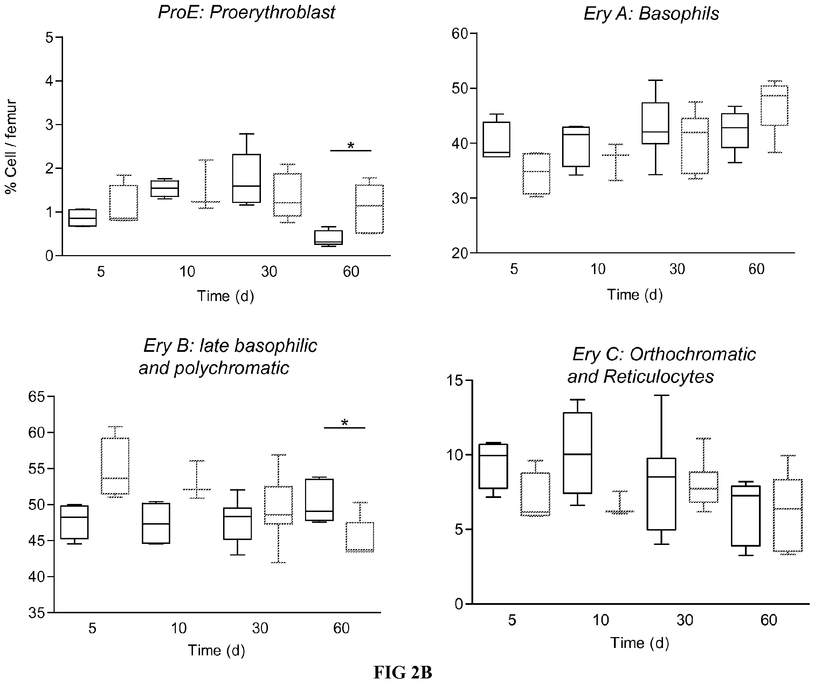

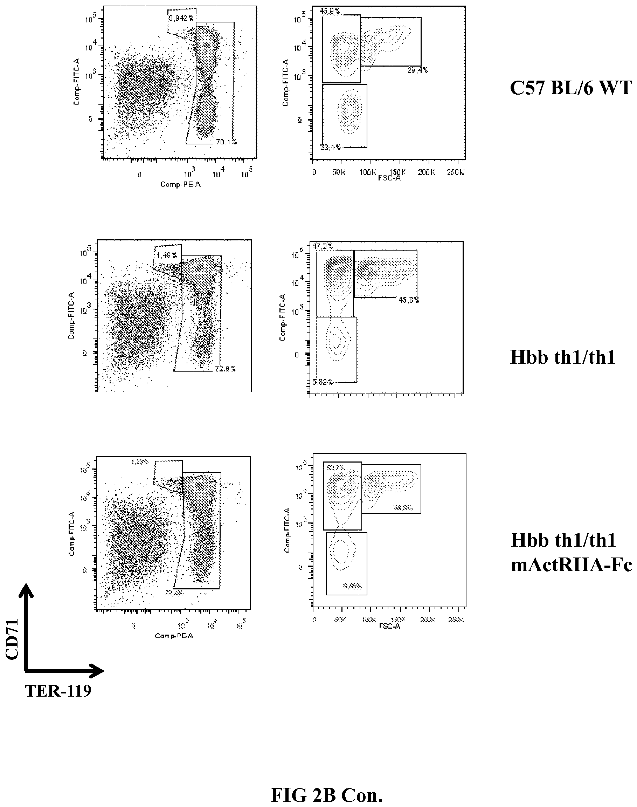

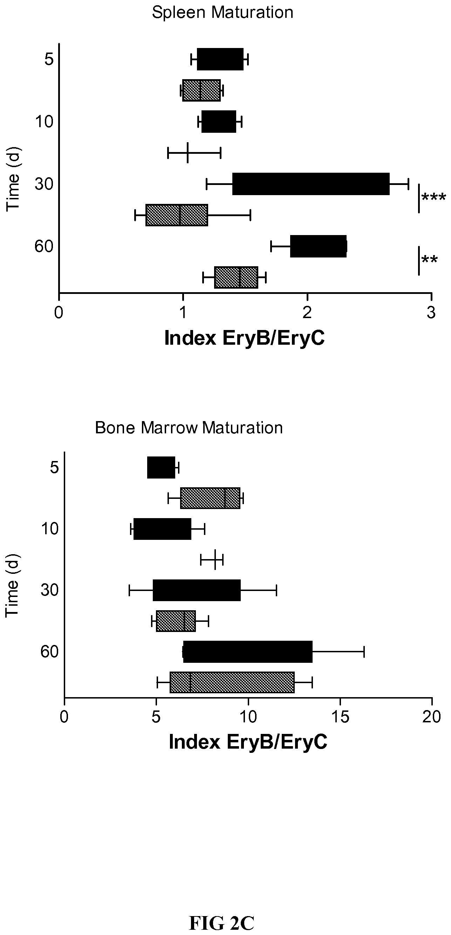

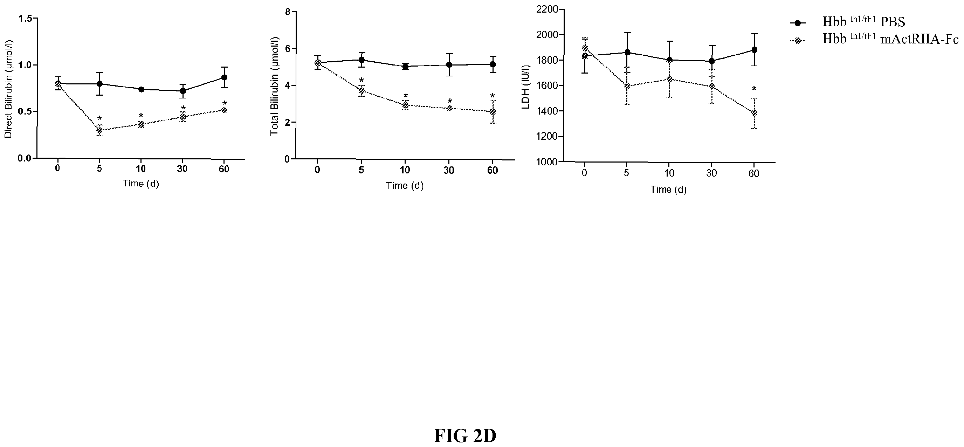

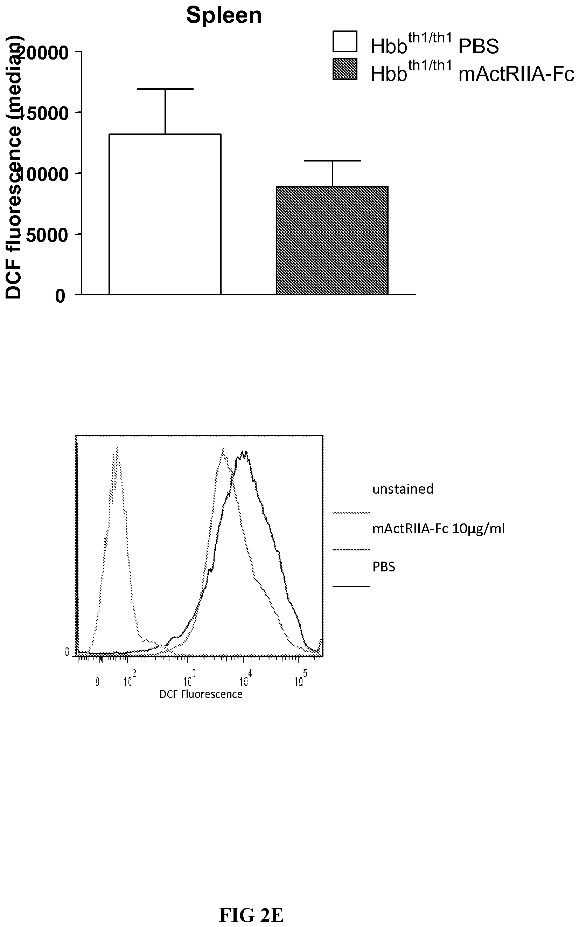

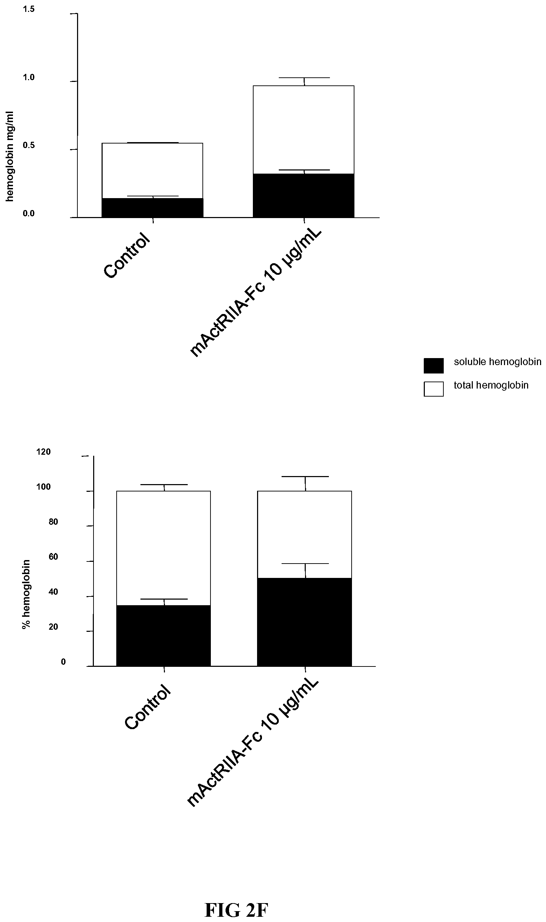

FIG. 2 illustrates that mActRIIA-Fc decreases ineffective erythropoiesis in thalassemic mice. (A-C) Bone marrow and spleen were harvested and erythroblast differentiation was evaluated by flow cytometry by CD71/TER119 staining and FSC/SSC distribution. (D) Analysis of total and direct bilirubin levels. (E) Reactive oxygen species (ROS) generation on primary pro-erythroblast differentiation was evaluated by flow cytometry using dichlorodihydrofluorescein. (F) Analysis of hemoglobin solubility on primary thalassemic pro-erythroblasts treated for 48 hours with mActRIIA-Fc or PBS. *p<0.05, **p<0.01, ***p<0.005, N=3-5 for each independent experiment.

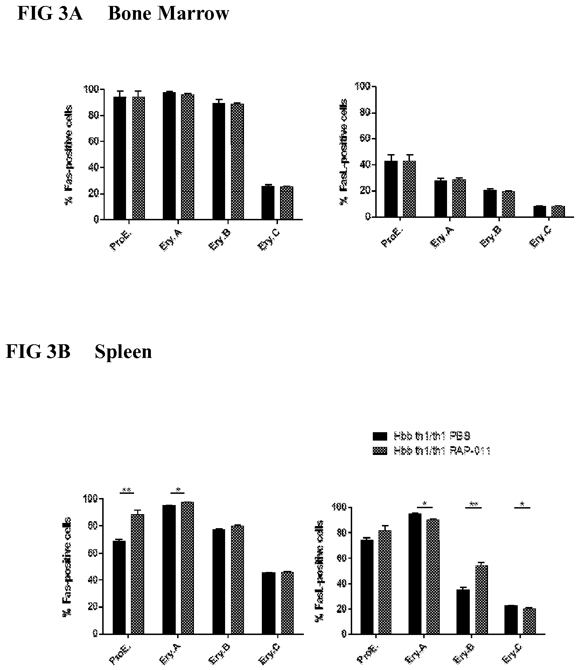

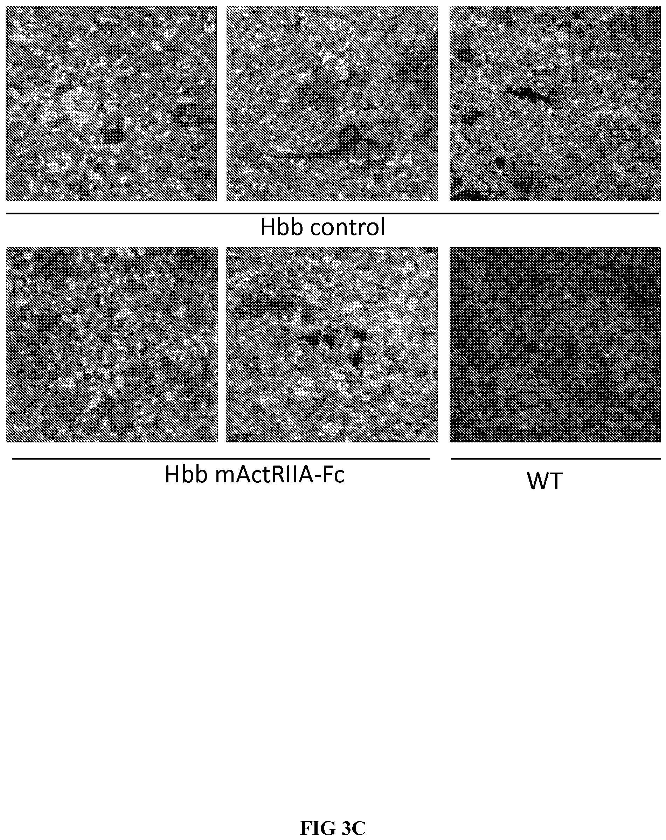

FIG. 3 illustrates the effect of mActRIIA-Fc on the apoptosis of erythroblasts from thalassemic mice. Expression of Fas-L on the bone marrow (A) and spleen (B) erythroblasts from mice treated with mActRIIA-Fc or PBS. (C) Tunel staining of erythroblasts was increased in mActRIIA-Fc-treated mice.

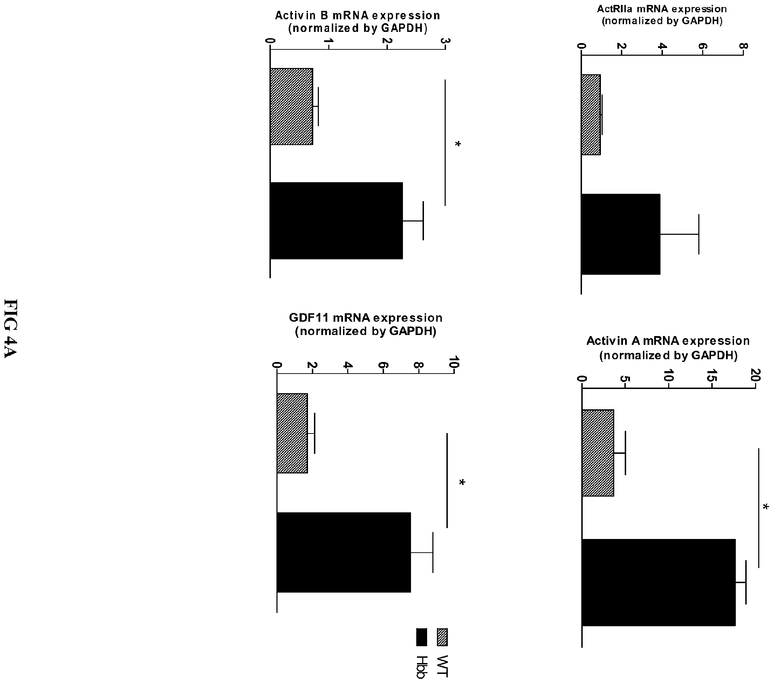

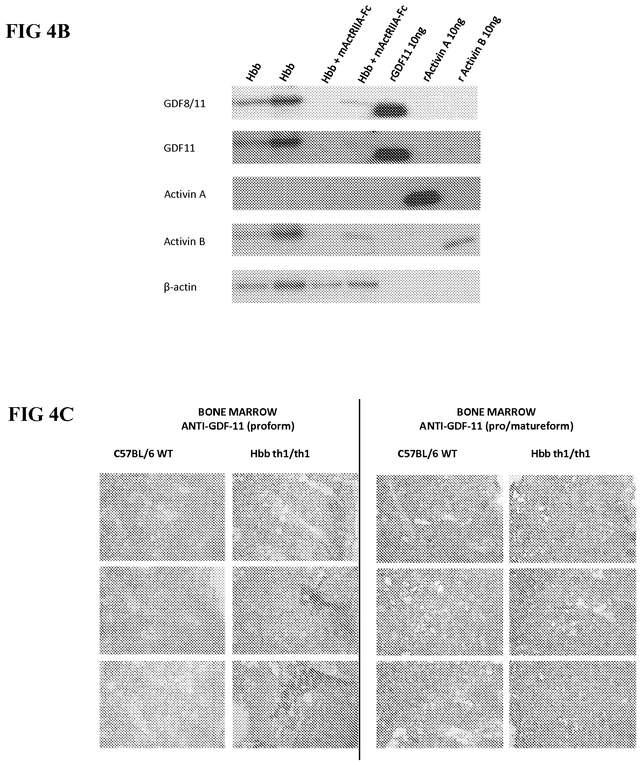

FIG. 4 illustrates the expression of ActRIIA ligands in the spleen of thalassemic mice. (A) mRNA expression levels of ActRII, activin A, activin B and GDF11 were increased in mActRIIA-Fc-treated animals. (B) Western blot analysis of GDF11 protein levels, which were decreased in mActRIIA-Fc-treated animals. (C) Immunohistochemical staining of bone marrow for GDF11 showed no change between wildtype and mice treated with mActRIIA-Fc.

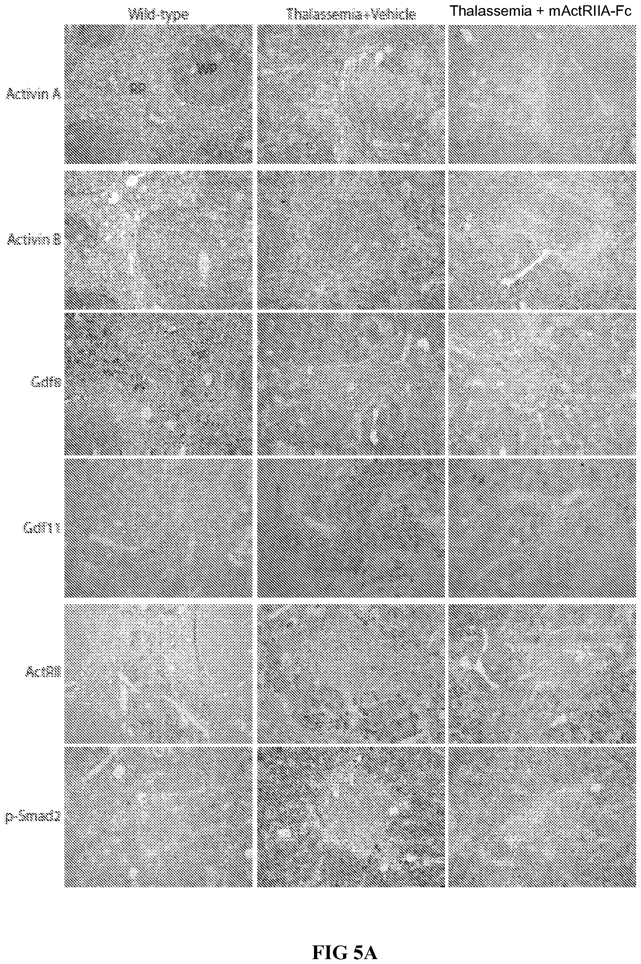

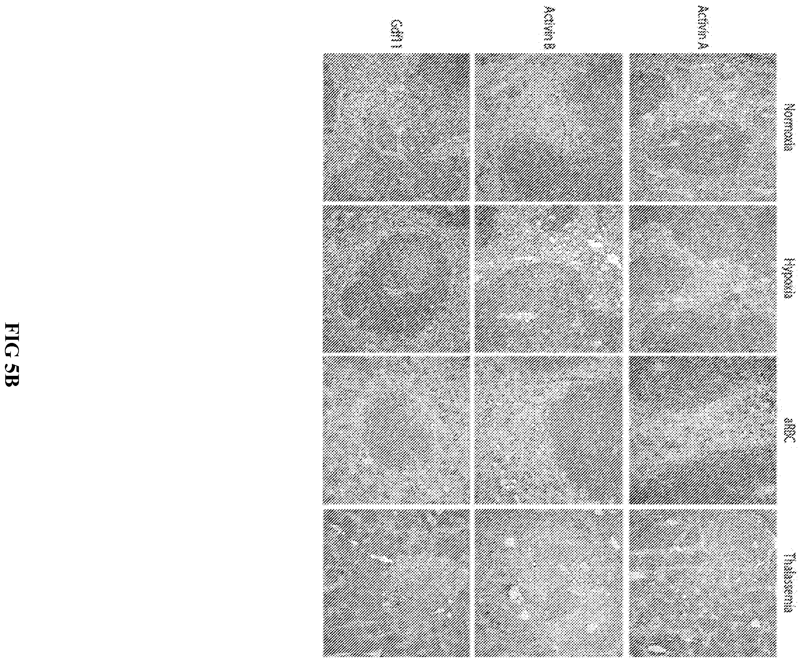

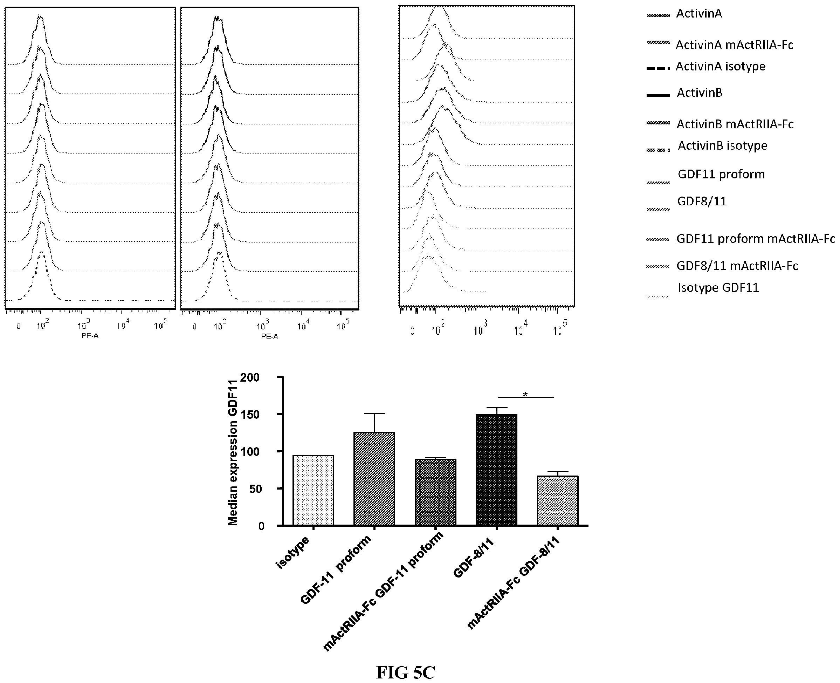

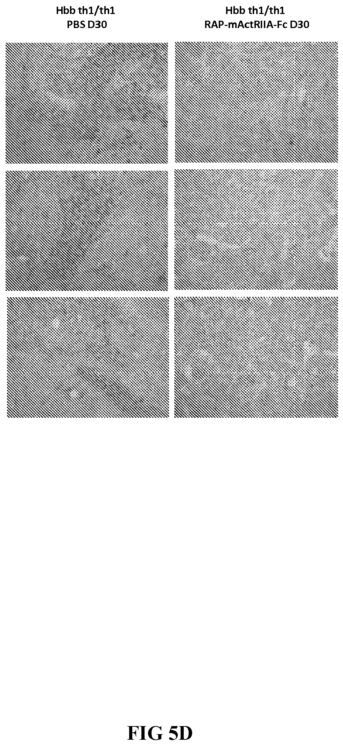

FIG. 5 illustrates the effect of mActRIIA-Fc on GDF11 expression in primary thalassemic proerythroblasts. (A) Immunohistochemical analysis of activin/GDF signaling pathway on thalassemic mice treated with PBS or mActRIIA-Fc for 30 days, showing increased levels of GDF11, ActRII and p-Smad2 on thalassemic mice. (B) Immunohistochemical analysis of activin A, activin B and GDF11 on thalassemic mice, compared to other models of anemia. (C) FACS analysis of primary thalassemic pro-erythroblasts treated with PBS or mActRIIA-Fc for 48 hours using specific antibodies against activin A, activin B, GDF11 propeptide and GDF8/GDF11 cleaved peptide. Quantification of GDF11 staining is demonstrated on the graph bars. (D) Immunohistochemical analysis of GDF11 proform expression in the spleens of thalassemic mice treated with PBS or mActRIIA-Fc. * p<0.05, N=4.

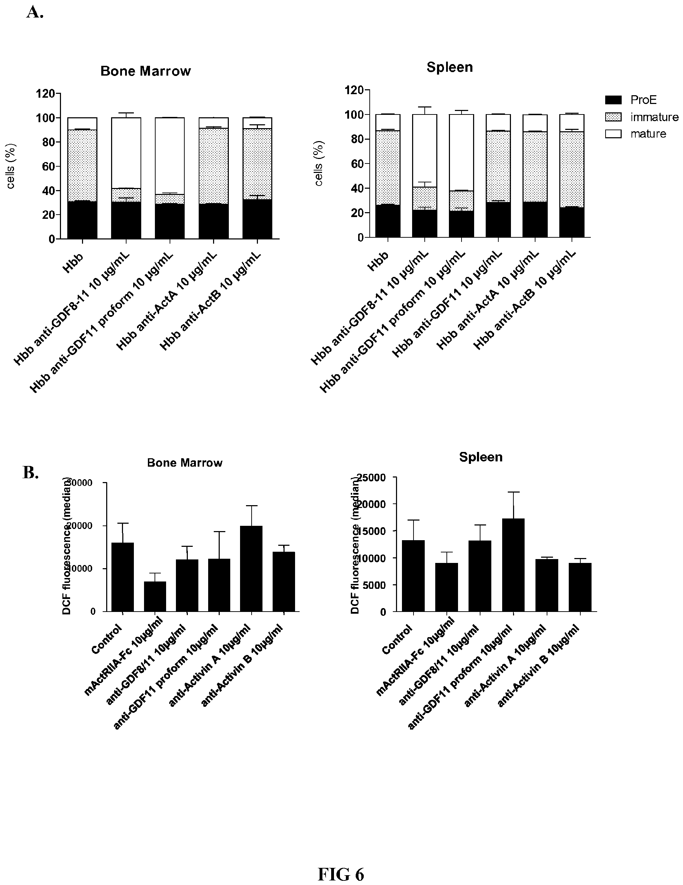

FIG. 6 illustrates that inhibition of GDF11 reduces ineffective erythropoiesis in thalassemic mice. (A) Bone marrow and spleen were harvested and primary pro-erythroblast differentiation was evaluated by flow cytometry by CD71/TER119 staining and FSC/SSC distribution. (B) ROS generation on primary pro-erythroblast differentiation was evaluated by flow cytometry using dichlorodihydrofluorescein. * p<0.05, N=4.

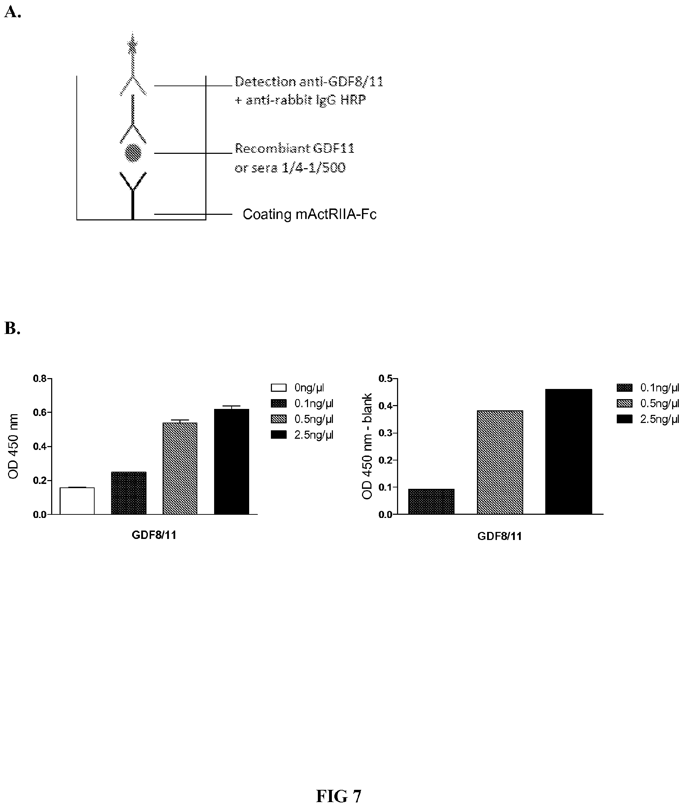

FIG. 7 illustrates a sandwich ELISA assay to detect GDF11 in serum. (A) Schematic of assay. (B) Plates were coated with 5 µg/mL of mActRIIA-Fc and increasing doses of recombinant GDF11 (0 ng/µl, 0.1 ng/µl, 0.5 ng/µl, 2.5 ng/µl) or control sera (1/4 to 1/500 dilution) were added to plates coated with mActRIIA-Fc, the plates washed and bound protein detected with anti-GDF8/11 antibodies, followed by detection using a horseradish peroxidase-coupled anti-rabbit IgG. GDF11 protein bound the plates in a dose-dependent manner.

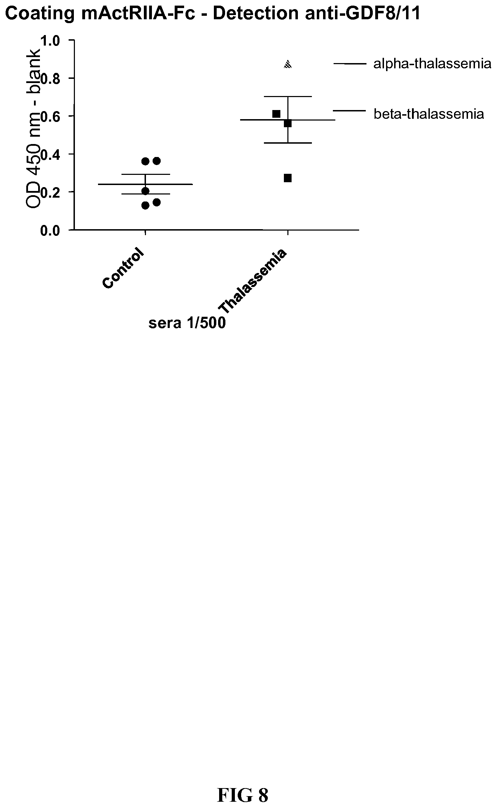

FIG. 8 illustrates the detection of increased levels of GDF11 in the serum of β-thalassemic patients. Sera were obtained from patients presenting thalassemia and healthy controls.

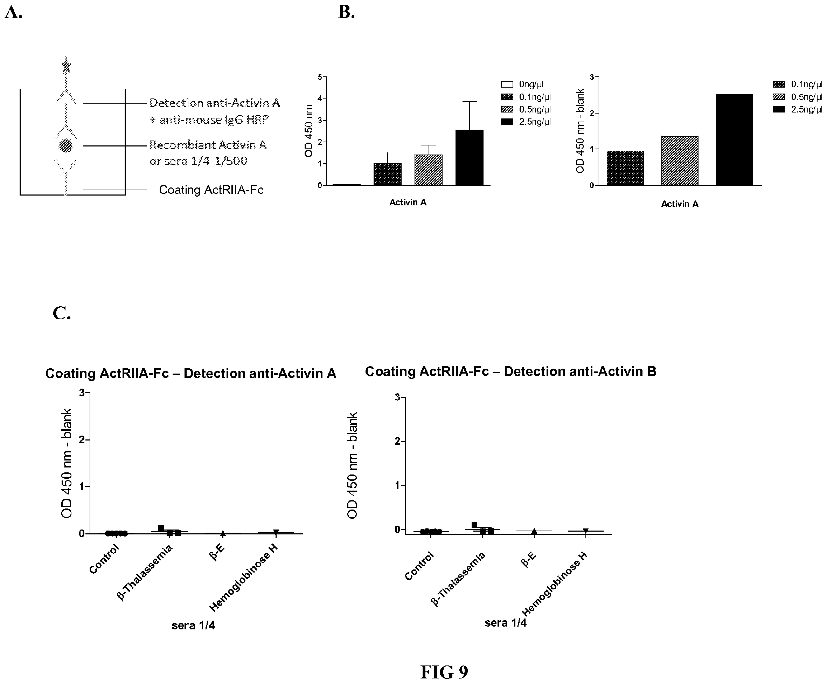

FIG. 9 illustrates a sandwich ELISA assay to detect activin A in serum. (A) Schematic of assay. (B) Plates were coated with 5 µg/mL of ActRIIA-Fc (SEQ ID NO.7) and increasing doses of recombinant activin A (0 ng/µl, 0.1 ng/µl, 0.5 ng/µl, 2.5 ng/µl) or control sera (1/4 to 1/500 dilution) were added to ActRIIA-Fc plates, the plates washed and bound protein detected with anti-activin A antibodies, followed by detection using a horseradish peroxidase-coupled anti-rabbit IgG. Activin A protein bound the plates in a dose-dependent manner. (C) Detection of levels of activin A in the serum of β-thalassemic patients. Sera were obtained from patients presenting thalassemia and healthy controls. There was no change in the serum levels of activin A in thalassemic patients.

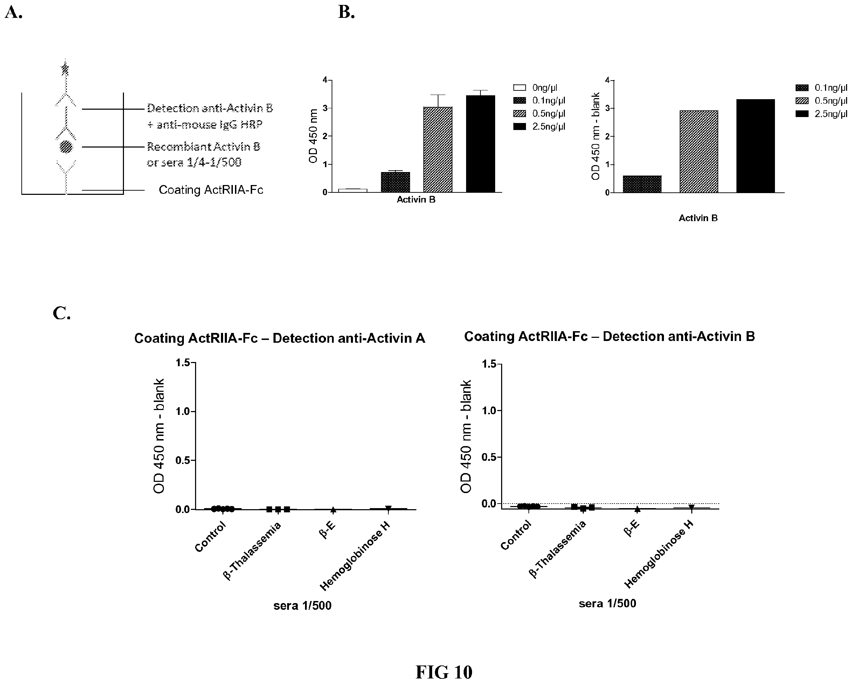

FIG 10 illustrates a sandwich ELISA assay to detect activin B in serum. (A) Schematic of assay. (B) Plates were coated with 5 µg/mL of ActRIIA-Fc and increasing doses of recombinant activin B (0 ng/µl, 0.1 ng/µl, 0.5 ng/µl, 2.5 ng/µl) or control sera (1/4 to 1/500 dilution) were added to ActRIIA-Fc plates, the plates washed and bound protein detected with anti-activin B antibodies, followed by detection using a horseradish peroxidase-coupled anti-rabbit IgG. Activin B protein bound the plates in a dose-dependent manner. (C) Detection of levels of activin B in the serum of β-thalassemic patients. Sera were obtained from patients presenting thalassemia and healthy controls. There was no change in the serum levels of activin B in thalassemic patients.

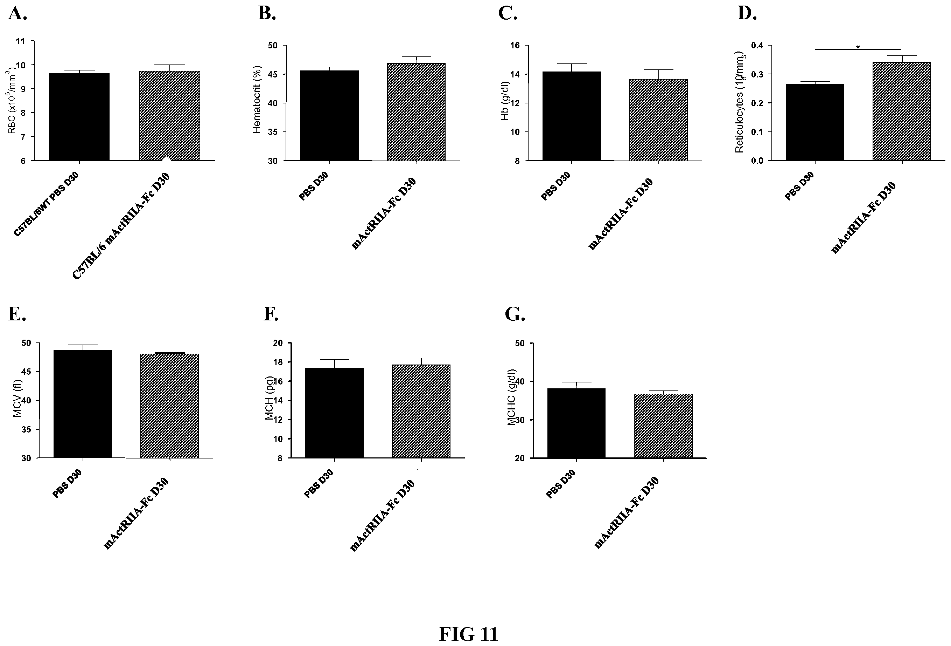

FIG. 11 illustrates that administration of mActRIIA-Fc to C57BL/6 wildtype mice does not change their hematological parameters. Evaluation of (A) red blood cell counts, (B) hematocrit, (C) hemoglobin showed no association with mActRIIA-Fc. There was a slight decrease (D) reticulocytosis. mActRIIA-Fc did not change red blood cells (RBC) parameters such as (E) mean corpuscular volume (MCV), (F) mean corpuscular hemoglobin (MCH) or (G) MCH concentration (MCHC). * p<0.05, N=3-5 for each independent experiment.

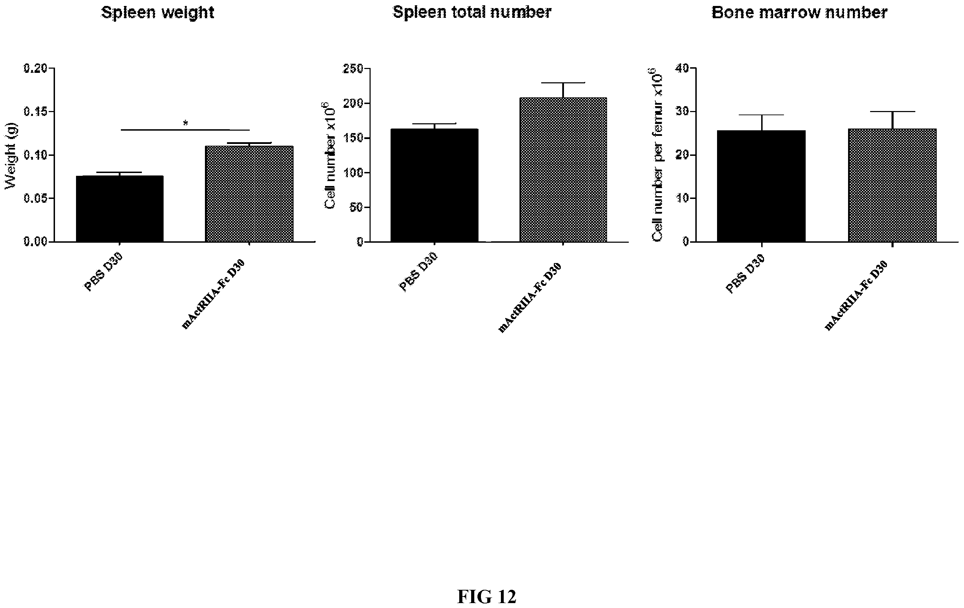

FIG. 12 illustrates that administration of mActRIIA-Fc to C57BL/6 wildtype mice had no effect on the number of spleen and bone marrow cells of the mice.

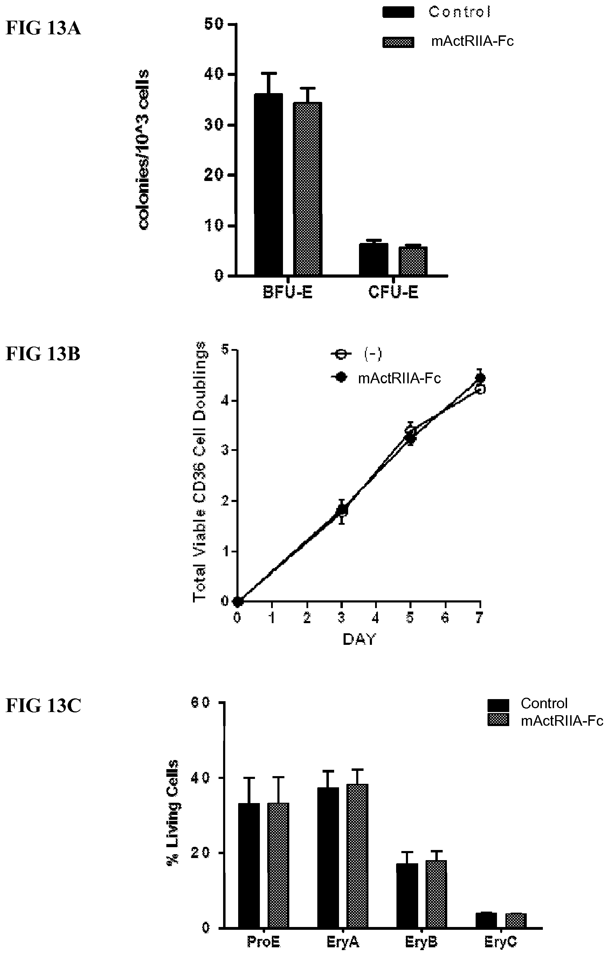

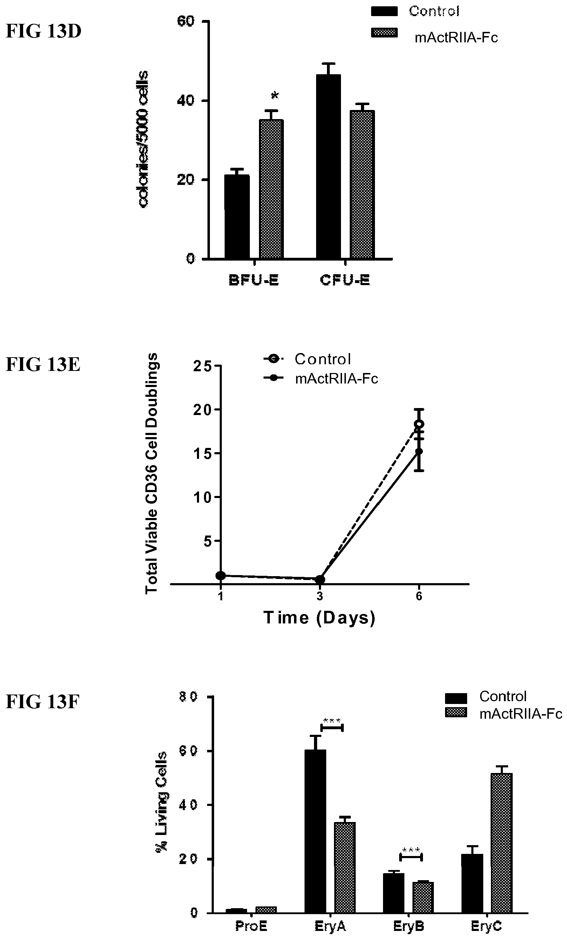

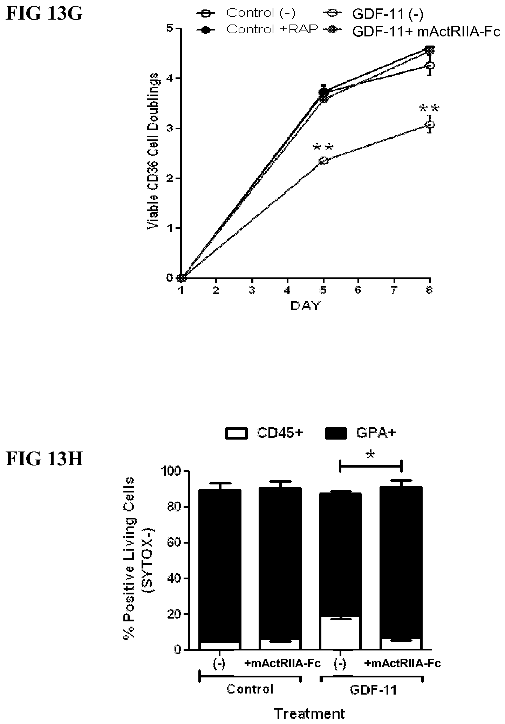

FIG. 13 illustrates that mActRIIA-Fc stimulates erythroid differentiation through inhibition of GDF11. (A-C) Erythroid differentiation of CD34+/CD36+ cells was performed by culturing in media containing EPO, +/-50µg/mL of mActRIIA-Fc; (A) erythroid progenitors, (B) cell proliferation and (C) erythoid precursors were analyzed. (D-F) Erythroid differentiation of CD34+/CD36+ cells when co-cultured with bone marrow (BM) cells in media containing EPO, +/-50µg/mL of mActRIIA-Fc; (D) erythroid progenitors, (E) cell proliferation and (F) erythoid precursors were analyzed. (G-H) Erythroid differentiation of CD36+ cells was performed by culturing in media containing EPO, +/- 200ng/mL of GDF11, +/- 100ug/mL of mActRIIA-Fc; (G) cell proliferation and (H) percentage of erythroid precursors GPA+ were analyzed

### 5. DETAILED DESCRIPTION

### 5.1 OVERVIEW

The invention provides an activin receptor type II inhibitor for use in a method for treatment of anemia, beta thalassemia, or for increasing red blood cell levels or orthochromatic erythroblasts (Ery-C) in a subject in need thereof, wherein the method comprises: (a) assessing the level and/or activity of GDF11 in a tissue sample of the subject, and (b) if the level and/or activity of GDF11 is elevated over the normal level and/or activity of GDF11, administering an activin receptor type II inhibitor to the subject.

The invention also provides an activin receptor type II inhibitor for use in a method for treatment of anemia, beta thalassemia, or for increasing red blood cell levels or orthochromatic erythroblasts (Ery-C) in a subject in need thereof, wherein the method comprises: (a) administering an activin receptor type II inhibitor, and (b) monitoring the level and/or activity of GDF11 in a tissue sample of the subject.

Within the boundary of the invention, the activin receptor type II inhibitor is a polypeptide comprising an amino acid sequence selected from the group consisting of:

| | |
|---|---|
| a | 90% identical to SEQ ID NO:2; |
| b | 95% identical to SEQ ID NO:2; |
| c | 98% identical to SEQ ID NO:2; |
| d | SEQ ID NO:2; |
| e | 90% identical to SEQ ID NO:3; |
| f | 95% identical to SEQ ID NO:3; |
| g | 98% identical to SEQ ID NO:3; |
| h | SEQ ID NO:3; |
| i | 90% identical to SEQ ID NO:6; |
| j | 95% identical to SEQ ID NO:6; |
| k | 98% identical to SEQ ID NO:6; |
| l | SEQ ID NO:6; |
| m | 90% identical to SEQ ID NO:7; |
| n | 95% identical to SEQ ID NO:7; |
| o | 98% identical to SEQ ID NO:7; |
| p | SEQ ID NO:7; |
| q | 90% identical to SEQ ID NO:12; |
| r | 95% identical to SEQ ID NO:12; |
| s | 98% identical to SEQ ID NO:12; |
| t | SEQ ID NO:12; |
| u | 90% identical to SEQ ID NO:17; |
| v | 95% identical to SEQ ID NO:17; |
| w | 98% identical to SEQ ID NO:17; |
| x | SEQ ID NO:17; |
| y | 90% identical to SEQ ID NO:20; |
| z | 95% identical to SEQ ID NO:20; |
| aa | 98% identical to SEQ ID NO:20; |
| bb | SEQ ID NO:20; |
| cc | 90% identical to SEQ ID NO:21; |
| dd | 95% identical to SEQ ID NO:21; |
| ee | 98% identical to SEQ ID NO:21; |
| ff | SEQ ID NO:21 |
| gg | 90% identical to SEQ ID NO:22; |
| hh | 95% identical to SEQ ID NO:22; |
| ii | 98% identical to SEQ ID NO:22; |
| jj | SEQ ID NO:22; |
| kk | 90% identical to SEQ ID NO:23; |
| ll | 95% identical to SEQ ID NO:23; |
| mm | 98% identical to SEQ ID NO:23; |
| nn | SEQ ID NO:23; |
| oo | 90% identical to SEQ ID NO:24; |
| pp | 95% identical to SEQ ID NO:24; |
| qq | 98% identical to SEQ ID NO:24; |
| rr | SEQ ID NO:24; |
| ss | 90% identical to SEQ ID NO:25; |
| tt | 95% identical to SEQ ID NO:25; |
| uu | 98% identical to SEQ ID NO:25; and |
| vv | SEQ ID NO:25; |

or
wherein the activin receptor type II inhibitor is a polypeptide comprising the extracellular domain of activin-receptor type IIA linked to the Fc portion of an antibody;
or
wherein the activin receptor type II inhibitor is a polypeptide comprising the extracellular domain of activin-receptor type IIB linked to the Fc portion of an antibody.

Provided herein is an activin receptor type II inhibitor for use in methods of treating anemia, or diseases or conditions associated with anemia or ineffective erythropoiesis, by using the level and/or activity of GDF11 as an indicator of responsiveness of a patient to the treatment with an activin type II receptor inhibitor, efficacy of the treatment with an activin type II receptor inhibitor, or appropriate dosage for the treatment with an activin type II receptor inhibitor. The diseases associated with anemia or ineffective erythropoiesis that can be treated in accordance with the methods described herein include, without limitation, thalassemia (e.g., beta thalassemia), myelodysplastic syndrome, chronic pernicious anemia, sickle cell anemia, and Diamond Blackfan anemia. The conditions associated with anemia or ineffective erythropoiesis that can be treated in accordance with the methods described herein include, without limitation, decreased red blood cell levels, decreased hemoglobin levels, decreased hematocrit levels, and decreased orthochromatic erythroblasts (Ery-C). In one embodiment, the methods are for treating anemia. In one embodiment, the methods are for treating thalassemia (e.g., beta thalassemia). In one embodiment, the methods are for treating decreased red blood cell levels or increasing red blood cell levels. An activin type II receptor inhibitor used in the methods described herein can be an inhibitor of ActRIIA and / or ActRIIB. In a preferred embodiment, an activin type II receptor inhibitor is a humanized fusion-protein consisting of the extracellular domain of ActRIIA and the human IgG1 Fc domain ("ActRIIA-Fc," e.g., SEQ ID NO:7).

The methods provided herein are based, in part, on the discovery that the levels of GDF11 are increased in the blood of human patients with beta-thalassemia and in a mouse model of beta-thalassemia, and that ActRIIA-mFc (ActRIIA fused to a murine IgG) reduces increased levels of GDF11 in the mouse model of beta-thalassemia (see Examples). Further, without being limited by theory, ligand trapping by ActRIIA-mFc corrects ineffective erythropoiesis and improves anemia in an experimental mouse model of thalassemia intermedia (Hbb^{th1/th1} mice). As shown in the Examples presented herein, treating of thalassemic mice (Hbb^{th1/th1}) with ActRIIA-mFc increased red blood cell counts, hematocrit, hemoglobin, mean cell volume (MCV) and mean cell hemoglobin, increased orthochromatic erythroblasts (Ery-C), reduced bone marrow and spleen cellularity, reduced late basophilic/polychromatic erythroblasts (Ery-B), in the spleen and bone marrow, reduced the levels of bilirubin levels (indicative of a decrease in red blood cell destruction), and reduced apoptotic cells. Furthermore, the methods provided herein are based, in part, on the discovery that in an *in vitro* culture system GDF-11 inhibits growth of human bone marrow-derived erythroid precursors and ActRIIA-mFc rescues this inhibitory effect (see Examples). Taken together, the data presented herein indicate that GDF-11 levels, e.g., blood and/or serum levels of GDF11, can identify which patients may respond to ActRIIA-Fc and can be used to monitor clinical response to the drug. The data presented herein also indicate that ActRIIA-Fc (e.g., ActRIIA-mFc, or ActRIIA-hFc such as SEQ ID NO:7) is useful in treating anemia associated with ineffective erythropoiesis.

The findings described herein, illustrated in the Examples, indicate that detection of the levels and/or activity of GDF11 can be used (i) as a marker (e.g., serum biomarker) of the extent of ineffective erythropoiesis in a patient, (ii) as a marker to measure the patient's response to an activin type II receptor inhibitor (such as ActRIIA-Fc), or (iii) to evaluate the pharmacodynamic effects of an activin type II receptor inhibitor (such as ActRIIA-Fc) in a patient following treatment, wherein the patient is a patient with anemia or a disease associated with anemia or ineffective erythropoiesis (such as thalassemia, e.g., beta-thalassemia). Thus, in certain embodiments, GDF11 can be used in the methods described herein as an indicator of efficacy of ActRIIA-Fc (e.g., ActRIIA-hFc such as SEQ ID NO:7) treatment and/or as an indicator of absence of response to the treatment with ActRIIA-Fc (e.g., ActRIIA-hFc such as SEQ ID NO:7). In addition, as described herein, GDF11 can be used as a reliable molecular marker to evaluate time-course treatment efficacy of ActRIIA-Fc (e.g., ActRIIA-hFc such as SEQ ID NO:7). Further, in a specific embodiment, the method comprises detection of the level and/or activity of GDF11 in the blood (e.g., detecting abnormal expression in a disease associated with ineffective erythropoiesis), and administration of an activin type II receptor inhibitor, such as ActRIIA-Fc, in a dose dependent on the level and/or activity of GDF11.

### 5.2 DIAGNOSTIC/ PROGNOSTIC/ THERAPEUTIC METHODS

In one aspect, the methods comprise determining the level and/or activity of GDF11 in a tissue sample of a subject (e.g., serum or blood), selecting subjects having elevated level and/or activity of GDF11 over the normal level and/or activity of GDF11, and treating subjects having elevated level and/or activity of GDF11 with an activin type II receptor inhibitor. Elevated levels and/or activity of GDF11 in a tissue sample of a subject (e.g., serum or blood) can indicate that the patient may be responsive to the treatment with an activin type II receptor inhibitor. In one embodiment, the activin type II receptor inhibitor is ActRIIA-Fc such as ActRIIA-hFc (e.g., SEQ ID NO:7).

GDF11 level and/or activity may be further used to evaluate appropriate dosing for a subject who is a candidate to be treated with an activin type II receptor inhibitor, to evaluate whether to adjust the dosage of the activin type II receptor inhibitor during treatment, and/or to evaluate an appropriate maintenance dose of the activin type II receptor inhibitor. If the GDF11 level and/or activity is outside the normal level and/or activity, dosing with an activin type II receptor inhibitor may be initiated, increased, reduced, delayed or terminated depending on the level and/or activity of GDF11. For example, if the GDF11 level and/or activity is elevated over the normal level and/or activity, dosing with an activin type II receptor inhibitor may be initiated or increased, and if the GDF11 level and/or activity is decreased over the normal level and/or activity, dosing with an activin type II receptor inhibitor may be reduced, delayed or terminated.

In one aspect, the methods comprise administering to a subject an activin type II receptor inhibitor, and monitoring the level and/or activity of GDF11 in the subject. A decrease in the level and/or activity of GDF11 after administration of an activin type II receptor inhibitor (relative to the level and/or activity before administration of the activin type II inhibitor) can indicate that the subject is responsive to the treatment with the activin type II receptor inhibitor and/or that the treatment with the activin type II receptor inhibitor is efficacious. No change or increase in the level and/or activity of GDF11 after administration of an activin type II receptor inhibitor (relative to the level and/or activity before administration of the activin type II inhibitor) can indicate that the subject is not responsive to the treatment with an activin type II receptor inhibitor or that the subject requires a higher dose of the activin type II receptor inhibitor for efficacy.

In other aspects, the degree of change in the level and/or activity of GDF11 after administration of an activin type II receptor inhibitor (relative to the level and/or activity before administration of the activin type II inhibitor or relative to the normal levels and/or activity of GDF11) can indicate the appropriate dosage or dosing regimen of activin type II receptor inhibitor. For example, no decrease or a small decrease in the level and/or activity of GDF11 (e.g., less than 5%, less than 10%, less than 15%, less than 20%, or less than 25%, or less than 30% decrease) after administration of a dose of an activin type II receptor inhibitor (relative to the level and/or activity before administration of the activin type II inhibitor) can indicate that a higher dose of the activin type II receptor inhibitor is required or desirable for efficacy. Accordingly, in some embodiments, when assessment of the level and/or activity of GDF11 shows no decrease or an undesirably small decrease in the level and/or activity of GDF11 (e.g., less than 5%, less than 10%, less than 15%, less than 20%, or less than 25%, or less than 30% decrease) after administration of a dose of an activin type II receptor inhibitor to the patient, the patient is administered a higher dose of an activin type II receptor inhibitor (e.g., 20%, 25%, 30%, 50%, 75%,100%, 200%, 300%, 400%, 500%, 600%, 700%, 800%, 900%, or 1000% higher dose).

In other embodiments, a decrease in the level and/or activity of GDF11 (for example, slight or average decrease, such as less than 10%, less than 15%, less than 20%, or less than 25%, less than 30%, less than 50%, or less than 75% decrease, or from 10% to 75% decrease, or from 25% to 50% decrease) after administration of a dose of an activin type II receptor inhibitor (relative to the level and/or activity before administration of the activin type II inhibitor) can indicate that the treatment with the activin type II receptor inhibitor is efficacious at the given dose and/or dosing regimen. Accordingly, in some embodiments, when assessment of the level and/or activity of GDF11 shows a decrease in the level and/or activity of GDF11 (for example, slight or average decrease, such as less than 10%, less than 15%, less than 20%, or less than 25%, less than 30%, less than 50%, or less than 75% decrease, or from 10% to 75% decrease, or from 25% to 50% decrease) after administration of a dose of an activin type II receptor inhibitor to the patient, the patient is administered the same dose of an activin type II receptor inhibitor. In a particular embodiment, when assessment of the level and/or activity of GDF11 shows an average or desirable decrease in the level and/or activity of GDF11 (for example, between 20% and 75%, between 25% and 75%, or between 30% and 60% decrease) after administration of a dose of an activin type II receptor inhibitor to the patient, the patient is administered the same dose of an activin type II receptor inhibitor. A large (or undesirably large) decrease in the level and/or activity of GDF11 (e.g., more than 50%, more than 60%, more than 70%, more than 75%, more than 80%, more than 90%, more than 95% decrease) after administration of a dose of an activin type II receptor inhibitor (relative to the level and/or activity before administration of the activin type II inhibitor) can indicate that a lower dose of the activin type II receptor inhibitor is desirable (e.g., to avoid the side effects of the treatment, such as hypertension). Accordingly, in some embodiments, when assessment of the level and/or activity of GDF11 shows a large (or undesirably large) decrease in the level and/or activity of GDF11 (e.g., more than 50%, more than 60%, more than 70%, more than 75%, more than 80%, more than 90%, more than 95% decrease) after administration of a dose of an activin type II receptor inhibitor to the patient, the patient is administered a lower dose of an activin type II receptor inhibitor (e.g., 20%, 25%, 30%, 40%, 50%, 60%, 75%, 80%, 90%, or 95% lower dose).

The monitoring of the level and/or activity of GDF11 can be performed by assessing the level and/or activity of GDF11 1 day, 1 week, 2 weeks, 3 weeks, 4 weeks, 5 weeks, 6 weeks, 7 weeks, 8 weeks, 3 months, 4 months, 5 months, 6 months, or 1 year after administration of an activin type II receptor inhibitor to the patient. The level and/or activity of GDF11 after administration of an activin type II receptor inhibitor to a patient, can also be used as an indicator of the appropriate or desirable frequency of administration of an activin type II receptor inhibitor to the patient. For example, if a decrease in the level and/or activity of GDF11 after administration of an activin receptor type II inhibitor is maintained over a time period (e.g., 1 day, 1 week, 2 weeks, 3 weeks, 4 weeks, 5 weeks, 6 weeks, 7 weeks, 8 weeks, 10 weeks, 3 months, 4 months, 5 months, 6 months, or 1 year), then the activin type II receptor inhibitor can be administered once during the time period (e.g. once a day, once a week, once in 2 weeks, once in 3 weeks, once in 4 weeks, once in 5 weeks, once in 6 weeks, once in 7 weeks, once in 8 weeks, once in 3 months, once in 4 months, once in 5 months, once in 6 months, or once a year). In a specific embodiment, if a decrease in the level and/or activity of GDF11 after administration of an activin receptor type II inhibitor is maintained over, e.g., 1 month, 2 months or 3 months, then the activin type II receptor inhibitor can be administered once in 1 month, 2 months, or 3 months, respectively.

In a specific embodiment, the methods comprise (i) assessing the level of GDF11 in a tissue (e.g., blood, serum, plasma, liver, bone marrow, and/or spleen) of a subject (e.g., a subject having anemia); (ii) administering an activin type II receptor inhibitor to the subject; and (iii) assessing the level of GDF11 in a tissue (e.g., blood, serum, plasma, liver, bone marrow, and/or spleen) of a subject (e.g., a subject having anemia) following the administration of the activin type II receptor inhibitor. The administration of step (ii) can comprise a single administration of an activin type II receptor inhibitor (i.e., a single dose is administered one time to the subject) or multiple administrations of an activin type II receptor inhibitor (e.g., the administration may comprise a complete administration regimen). The assessment of step (iii) can be performed at any point following the administration of step (ii). For example, the assessment of step (iii) can be performed 1 day, 2 days, 3 days, 4 days, 5 days, 6 days, 1 week, 2 weeks, 3 weeks, 4 weeks, 5 weeks, 6 weeks, 7 weeks, 8 weeks, 10 weeks, 3 months, 4 months, 5 months, 6 months, or 1 year following the administration of step (ii). As another example, the the assessment of step (iii) can be performed 1-3 days, 2-4 days, 3-5 days, 5-7 days, 1-2 weeks, 2-3 weeks, 3-4 weeks, 1-2 months, 2-3 months, 3-4 months, 4-5 months, 5-6 months, or 6-12 months following the administration of step (ii). The therapeutic regimen can be adjusted based on the outcome of the assessments in steps (i) and (iii). For example, if the levels of GDF11 in the tissue of the subject determined in the assessment of step (i) are decreased relative to the levels of GDF11 in the tissue of the subject determined in the assessment of step (iii), then the dose of the activin type II receptor inhibitor administered to the subject can be maintained or decreased. Conversely, if the levels of GDF11 in the tissue of the subject determined in the assessment of step (i) are increased relative to the levels of GDF11 in the tissue of the subject determined in the assessment of step (iii), then the dose of the activin type II receptor inhibitor administered to the subject can be increased. The steps of the foregoing method can be repeated/altered as necessary to determine the appropriate dose/therapeutic regimen that is appropriate for the subject undergoing treatment.

In another aspect, the methods comprise (a) administering a first dose of an activin type II receptor inhibitor to a patient, (b) determining the level and/or activity of GDF11 in a tissue sample of the patient (e.g., serum), and (c) administering a second dose of the activin type II receptor inhibitor, wherein if the level and/or activity of GDF11 is elevated over normal, the second dose of the activin receptor type II inhibitor is higher than the first dose (e.g., 20%, 30%, 40%, 50%, 60%, 70%, 80%, 90%, 100%, 200%, 300%, 400%, 500%, 600%, 700%, 800%, 900%, or 1000% higher), and wherein, if the level and/or activity of GDF11 is decreased over normal, the second dose of the activin receptor type II inhibitor is lower than the first dose (e.g., 20%, 30%, 40%, 50%, 60%, 70%, 80%, or 90% lower). In these embodiments, the level and/or activity of GDF11 in a tissue sample is compared to the normal level and/or activity of GDF11 (e.g., the average level and/or activity of GDF11 in a sample from the same tissue of healthy subjects, such as subjects in the same age group who do not have anemia). In certain embodiments, the level and/or activity of GDF11 is deemed to be elevated over the normal level and/or activity of GDF11 when it is elevated at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 200%, at least 300%, or at least 500% over the normal level and/or activity of GDF11. In certain embodiments, the level and/or activity of GDF11 is deemed to be elevated over the normal level and/or activity of GDF11 when it is elevated at least 25%, at least 50%, at least 75%, at least 100%, or at least 200% over the normal level and/or activity of GDF11. In some embodiments, the level and/or activity of GDF11 is deemed to be decreased over the normal level and/or activity of GDF11 when it is decreased at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% over the normal level and/or activity of GDF11. In some embodiments, the level and/or activity of GDF11 is deemed to be decreased over the normal level and/or activity of GDF11 when it is decreased at least 25%, at least 50%, at least 75%, or at least 90% over the normal level and/or activity of GDF11.

The methods described herein are based, in part, on a finding that GDF11 inhibits growth of precursors of red blood cell, and thus the level of GDF11 can correlate with the level of red blood cells. It is desirable to maintain an optimal level of red blood cells in a patient because, while decreases in the levels of red blood cells, hemoglobin or hematocrit are associated with ineffective erythropoiesis and anemia, excessive increases in the levels of red blood cells, hemoglobin or hematocrit are associated with increase in blood pressure and other undesirable side effects (which may be caused by treatment with higher than optimal doses of an activin type II receptor inhibitor). Accordingly, it can be desirable to administer an activin type II receptor inhibitor in a dosing regimen that maintains the level and/or activity of GDF11 in a patient at or around (e.g., within 5%, 10%, 15%, 20%, 25%, 30%, 40%, 50%, 60% or 75% of) the normal level and/or activity of GDF11.

In certain embodiments, if the level and/or activity of GDF11 is determined to be reduced below the normal level and/or activity of GDF11, then the administration of an activin type II receptor inhibitor is adjusted accordingly, e.g., is delayed, until the level and/or activity of GDF11 is returned to normal or within 5%, 10%, 15%, 20%, 25%, 30%, 40%, 50%, 60% or 75% of the normal level and/or activity of GDF11, or terminated either temporarily or permanently. In other embodiments, wherein the level and/or activity of GDF11 is determined to be reduced below the normal level and/or activity of GDF11, the administration of an activin type II receptor inhibitor is not delayed, but the dosage amount or frequency of dosing of the inhibitor is set at an amount that would reduce the risk of an unacceptable increase in red blood cells, hemoglobin or hematocrit, or alternatively, a therapeutic regimen is developed for the patient that combines the use of the inhibitor with an agent that addresses the unacceptable increase in red blood cells, hemoglobin or hematocrit (e.g., a blood pressure lowering agent).

In certain embodiments, provided herein is an activin receptor type II inhibitor for use in a method of treating anemia in an individual in need thereof by determining the level and/or activity of GDF11 in the individual, and if the level and/or activity of GDF11 is elevated, administering to the individual a therapeutically effective amount of an activin type II receptor inhibitor, particularly an ActRII polypeptide (e.g., ActRIIa-hFc), and optionally, further monitoring (or determining) the level and/or activity of GDF11, and adjusting the dose of the activin type II receptor inhibitor (wherein, for example, if GDF11 is elevated over the normal level and/or activity the dose of the activin type II receptor inhibitor is increased, and if the level and/or activity of GDF11 is lower than the normal level and/or activity the dose of the activin type II receptor inhibitor is decreased).

In certain embodiments, provided herein is an activin receptor type II inhibitor for use in a method of treating beta-thalassemia in an individual in need thereof by determining the level and/or activity of GDF11 in the individual, and if the level and/or activity of GDF11 is elevated, administering to the individual a therapeutically effective amount of an activin type II receptor inhibitor, particularly an ActRII polypeptide (e.g., ActRIIa-hFc), and optionally, further monitoring (or determining) the level and/or activity of GDF11 and adjusting the dose of the activin type II receptor inhibitor (wherein, for example, if the level and/or activity of GDF11 is elevated over normal the dose of the activin type II receptor inhibitor is increased, and if the level and/or activity of GDF11 is lower than normal the dose of the activin type II receptor inhibitor is decreased).

In certain embodiments, provided herein is an activin receptor type II inhibitor for use in a method of increasing red blood cell, hemoglobin, hematocrit or Ery-C levels in an individual in need thereof by determining the level and/or activity of GDF11 in the individual, and if the level and/or activity of GDF11 is elevated, administering to the individual a therapeutically effective amount of an activin type II receptor inhibitor, particularly an ActRII polypeptide (e.g., ActRIIa-hFc), and optionally, further monitoring (or determining) the level and/or activity of GDF11, and adjusting the dose of the activin type II receptor inhibitor (wherein, for example, if the level and/or activity of GDF11 is elevated over normal the dose of the activin receptor antagonist is increased, and if the level and/or activity of GDF11 is lower than normal the dose of the activin type II receptor inhibitor is decreased).

In certain embodiments, the methods are utilized in connection with a method for treating or ameliorating anemia, ineffective erythropoiesis, decreased red blood cell levels or any other blood disorder described herein. In certain embodiments, the methods are utilized in connection with a method for increasing red blood cell levels, hemoglobin levels, hematocrit levels, or levels of colony forming units in a patient with anemia, ineffective erythropoiesis, decreased red blood cell levels or any other blood disorder described herein. In certain embodiments, the red blood cell levels are increased by at least 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 250%, 300%, 350%, 400%, 450%, or by at least 500%. In certain embodiments, the hemoglobin levels are increased by at least 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 250%, 300%, 350%, 400%, 450%, or by at least 500%. In certain embodiments, the hematocrit levels are increased by at least 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 250%, 300%, 350%, 400%, 450%, or by at least 500%. In certain embodiments, the colony forming units levels are increased by at least 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 250%, 300%, 350%, 400%, 450%, or by at least 500%. In certain embodiments, the methods are utilized in connection with a method for decreasing the levels of apoptosis of erythroid progenitors and precursors.

### 5.3 PATIENT POPULATIONS

The subjects treated in accordance with the methods described herein can be any mammals such as rodents and primates, and in a preferred embodiment, humans. In certain embodiments, the methods described herein can be used to treat anemia or ineffective erythropoiesis, or to monitor and/or increase red blood cell, hemoglobin, hematocrit or Ery-C levels, in any mammals such as rodents and primates, and in a preferred embodiment, in human patients.

In one aspect, the methods are utilized in connection with a method of treatment. In some embodiments, the methods is for treating anemia (e.g., anemia caused by ineffective erythropoiesis). In certain embodiments, the method is for treating a disease associated with ineffective erythropoiesis (e.g., thalassemia, myelodysplastic syndromes, chronic pernicious anemia, or sickle cell anemia). In certain embodiments, the method is for treating an inherited bone marrow failure syndrome (such as, but not limited to, Amegakaryocytic Thrombocytopenia, Diamond-Blackfan Anemia, Dyskeratosis Congenita, Fanconi Anemia, Pearson Syndrome, Severe Congenital Neutropenia, Shwachman-Diamond Syndrome, Thrombocytopenia Absent Radii). In specific embodiments, the method is for treating an inherited bone marrow failure syndrome that specifically affects red blood cells. In certain embodiments, the method is for treating anemia and/or bone disorders associated with end stage renal disease. In some embodiments, the method is for treating thalassemia (e.g., β-thalassemia), myelodysplastic syndromes, chronic pernicious anemia, sickle cell anemia, or Diamond-Blackfan Anemia. In one embodiment, the method is for treating β-thalassemia. In one embodiment, the method is for treating Diamond-Blackfan Anemia.

In certain embodiments, the methods are for treating a patient having anemia (e.g., diagnosed with anemia). In some embodiments, the methods are for treating a patient having (e.g., diagnosed with) a disease associated with ineffective erythropoiesis, such as thalassemia, myelodysplastic syndromes, chronic pernicious anemia, or sickle cell anemia. In certain embodiments, the methods are for treating a patient having (e.g., diagnosed with) an inherited bone marrow failure syndrome, such as Amegakaryocytic Thrombocytopenia, Diamond-Blackfan Anemia, Dyskeratosis Congenita, Fanconi Anemia, Pearson Syndrome, Severe Congenital Neutropenia, Shwachman-Diamond Syndrome, Thrombocytopenia Absent Radii, or a bone marrow failure syndrome that specifically affects red blood cells. In specific embodiments, the methods are for treating a patient having (e.g., diagnosed with) anemia and/or bone disorders associated with end stage renal disease. In specific embodiments, the methods are for treating a patient having (e.g., diagnosed with) thalassemia (e.g., β-thalassemia), myelodysplastic syndromes, chronic pernicious anemia, sickle cell anemia, or Diamond-Blackfan Anemia. In one embodiment, the methods are for treating a patient having (e.g., diagnosed with) β-thalassemia.

Anemia is associated with a variety of disorders and conditions that include, without limitation: chronic renal failure, myelodysplastic syndrome, rheumatoid arthritis, bone marrow transplantation, solid tumors (e.g. breast cancer, lung cancer, colon cancer), tumors of the lymphatic system (e.g. chronic lymphocyte leukemia, non-Hodgkins and Hodgkins lymphomas), tumors of the hematopoietic system (e.g. leukemia, myelodysplastic syndrome, multiple myeloma), radiation therapy, chemotherapy (e.g. platinum containing regimens), inflammatory and autoimmune diseases (including, but not limited to, rheumatoid arthritis, other inflammatory arthritides, systemic lupus erythematosus (SLE), acute or chronic skin diseases (e.g. psoriasis), inflammatory bowel disease (e.g. Crohn's disease and ulcerative colitis)), acute or chronic renal disease or failure including idiopathic or congenital conditions, acute or chronic liver disease, acute or chronic bleeding, situations where transfusion of red blood cells is not possible due to patient allo- or auto-antibodies and/or for religious reasons, infections (e.g. malaria, osteomyelitis), hemoglobinopathies (including, for example, sickle cell disease, thalassemias), drug use or abuse (e.g. alcohol misuse; pediatric patients with anemia from any cause to avoid transfusion), and situations where elderly patients or patients with underlying cardiopulmonary disease with anemia cannot receive transfusions due to concerns about circulatory overload. In certain embodiments, the methods described herein are used to treat anemia, or monitor and/or increase red blood cell, hemoglobin, hematocrit or Ery-C levels, in any patient having one or more of the above-listed disorders or conditions.

In certain embodiments, the method is for treating an anemia wherein the subject is unresponsive to administration of erythropoietin. In certain embodiments, the method is for treating an anemia wherein the subject is unresponsive to administration of iron, vitamin B-12, and / or folate. In certain embodiments, the method is for treating an anemia resulting from erythroid progenitors and precursors being highly sensitive to death by apoptosis.

In certain embodiments, the subjects treated (e.g., selected for treatment) in accordance with the methods described herein have elevated levels and/or activity of GDF11 relative to the normal levels of GDF11. For example, the subjects treated (e.g., selected for treatment) in accordance with the methods described herein have elevated levels and/or activity of GDF11 is a tissue relative to the average level and/or activity of GDF11 in the same tissue from the healthy subjects in the same age category. The level and/or activity of GDF11 can be assessed by any method known in the art or described herein. In some embodiments, subjects treated in accordance with the methods described herein have the level and/or activity of GDF11 which is at least 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 250%, 300%, 350%, 400%, 450%, 500%, or 1000% over the normal level and/or activity of GDF11 (e.g., the average level in a healthy subject or subjects). In specific embodiments, subjects treated in accordance with the methods described herein have the level and/or activity of GDF11 which is at least 50%, at least 75%, at least 100%, at least 200%, or at least 500% % over the normal level and/or activity of GDF11. In some embodiments, the methods described herein are used to monitor, modulate or increase the level of red blood cells, hemoglobin, hematocrit or Ery-C levels in subjects that have been determined to have elevated levels of GDF11.

In certain embodiments, the subjects treated (e.g., selected for treatment) in accordance with the methods described herein have (i) elevated levels and/or activity of GDF11 relative to the normal levels of GDF11 (e.g., in serum, bone marrow, liver, and/or spleen), and (ii) anemia or a disease or condition associated with anemia or ineffective erythropoiesis (e.g., subjects diagnosed with anemia). In certain embodiments, a human patient treated (e.g., selected for treatment) in accordance with the methods described herein has elevated serum levels and/or activity of GDF11 relative to the normal levels of GDF11, and has anemia (e.g., has been diagnosed with anemia).

In some embodiments, the subjects treated in accordance with the methods described herein have an undesirably low red blood cell count, an undesirably low level of hemoglobin, an undesirably low level of hematocrit, and/or an undesirably low level of Ery-C. The methods described herein can be used to monitor, modulate and/or increase red blood cell, hemoglobin, hematocrit or Ery-C levels in selected patient populations. In other embodiments, the method is for treating a patient at risk for developing undesirably low red blood cell or hemoglobin levels, such as those patients that are about to undergo major surgery or other procedures that may result in substantial blood loss. In some embodiments, subjects treated in accordance with the methods described herein are about to undergo major surgery or another procedure that may result in substantial blood loss.

When determining whether the level of hemoglobin is undesirably low, a level of less than normal for the appropriate age and gender category may be indicative of anemia, although individual variations are taken into account. For example, a hemoglobin level of 12 g/dl is generally considered the lower limit of normal in adult humans. Potential causes of low levels of hemoglobin include blood-loss, nutritional deficits, medication reaction, various problems with the bone marrow and many diseases. Patients may be treated with a dosing regimen intended to restore the patient to a target hemoglobin level, usually between about 10 g/dl and about 12.5 g/dl, and typically about 11.0 g/dl (see also Jacobs et al. (2000) Nephrol Dial Transplant 15, 15-19), although lower target levels may cause fewer cardiovascular side effects. Optimally, the target hemoglobin level can be individualized for each patient. In certain embodiments, a patient to be treated with the methods described herein has hemoglobin levels of less than 13 g/dl, less than 12.5 g/dl, less than 12 g/dl, less than 11.5 g/dl, less than 11 g/dl, less than 10.5 g/dl, less than 10 g/dl, less than 9.5 g/dl, or less than 9 g/dl.

When determining whether the level of red blood cells is undesirably low, hematocrit levels (percentage of the volume of a blood sample occupied by the cells) can be used to assess the condition of red blood cells. Hematocrit levels for healthy individuals range from 41 to 51% for adult males and from 35 to 45% for adult females. Target hematocrit levels are usually around 30-33%, although hematocrit levels vary from person to person. Optimally, the target hematocrit level can be individualized for each patient.

In some embodiments, the methods described herein are used to treat anemia, or a disease or condition associated with anemia or ineffective erythropoiesis, in patients that are susceptible to adverse effects when administered higher than optimal dose of an activin type II receptor inhibitor, particularly an ActRII polypeptide (e.g., ActRIIa-hFc). Adverse effects that may be associated with administration of higher than optimal dose of an activin type II receptor inhibitor include, without limitation, an excessive increase in red blood cell levels, hematocrit levels or hemoglobin levels, an excessive increase in iron stores, and bone marrow or spleen hypercellularity. In turn, excessive increases in red blood cell levels, hemoglobin levels, or hematocrit levels may cause an increase in blood pressure and/or other undesirable side effects. In specific embodiments, patients treated in accordance with the methods described herein are susceptible to hypertension (e.g., increase in systolic blood pressure, diastolic blood pressure and/or mean arterial blood pressure) or another condition that may be related to excessive increase in red blood cell levels (e.g., headaches, influenza-like syndrome, or vascular thrombosis). In a specific embodiment, patients treated in accordance with the methods described herein are susceptible to hypertension or another condition that may be related to excessive increase in red blood cell levels when treated with an activin type II receptor inhibitor (e.g., ActRIIA-hFc such as SEQ ID NO:7).

Subjects of any age can be treated in accordance with the methods described herein. In some embodiments, subjects treated in accordance with the methods described herein are over 55 years of age. In some embodiments, subjects treated in accordance with the methods described herein are under 3 or 10 years of age. In other embodiments, subjects treated in accordance with the methods described herein are under 18 years of age. In yet other embodiments, subjects treated in accordance with the methods described herein are 18 to 55 years of age.

### 5.4 ASSESSING GDF11 LEVEL AND/OR ACTIVITY

The level or the activity of GDF11 can be determined by any method known in the art or described herein. For example, the level of GDF11 in a tissue sample can be determined by assessing (e.g., quantifying) transcribed RNA of GDF11 in the sample using, e.g., Northern blotting, PCR analysis, real time PCR analysis, or any other technique known in the art or described herein. In one embodiment, the level of GDF11 in a tissue sample can be determined by assessing (e.g., quantifying) mRNA of GDF11 in the sample.

The level of GDF11 in a tissue sample can also be determined by assessing (e.g., quantifying) the level of protein expression of GDF11 in the sample using, e.g., immunohistochemical analysis, Western blotting, ELISA, immunoprecipitation, flow cytometry analysis, or any other technique known in the art or described herein. In particular embodiments, the level of GDF11 is determined by a method capable of quantifying the amount of GDF11 present in a tissue sample of a patient (e.g., in human serum), and/or capable of detecting the correction of the level of GDF11 following treatment with an activin type II receptor inhibitor. In one embodiment, the level of GDF11 in a tissue sample is determined by assessing (e.g., quantifying) protein expression of GDF11 in the sample using ELISA. For example, GDF11 can be identified and quantified in the human serum using sandwich ELISA method described in the Examples. The sandwich ELISA method for use in determining the level of GDF11 in a tissue sample can comprise ActRIIA-Fc coating (using, e.g., ActRIIA-mFc, or ActRIIA-hFc such as SEQ ID NO:7) of ELISA plates, contacting the plates with the tissue sample (e.g., human serum), and detecting ActRIIA ligand (such as GDF11) in the tissue sample (e.g., human serum) that bind to ActRIIA-Fc by specific antibodies. In some embodiments, the method for use in determining the level and/or activity of GDF11 as described herein (e.g., sandwich ELISA) is capable of detecting from 100 pg/ml of recombinant GDF11, Activin A and/or Activin B bound to ActRIIA.

Antibodies for use in assays that measure the levels of GDF11 in a sample (e.g., in a tissue sample, e.g., a sample of blood, serum, plasma, liver, spleen, and/or bone marrow) are known in the art or could be readily developed using approaches known to those of skill in the art. Examples of monoclonal antibodies that can be used in assays that measure the levels of GDF11 in a sample include antibodies from LifeSpan Biosciences Inc., Seattle, WA, with catalog numbers LS-C121127, LS-C138772, LS-C105098; antibodies available from Santa Cruz Biotechnology, Inc., Santa Cruz, CA, with catalog number (X-19): sc-81952; and antibodies available from Sigma-Aldrich Co. LLC, with product number: WH0010220M3.

The activity of GDF11 can be measured by any assay known in the art including, without limitation, colony formation assay, a reporter gene assay (e.g., containing GDF11-responsive reporter gene construct), alkaline phosphatase assay, or any other bioactivity assay. Exemplary assays that can be used to measure activity of GDF11 are described in Souza et al., 2008, Mol. Endocrinology 22(12):2689-2702; and Bessa et al., 2009, Protein Expression and Purification 63:89-94.

The level and/or activity of GDF11 can be assessed in any tissue sample obtained from a patient treated in accordance with the methods described herein. In certain embodiments, GDF11 level and/or activity is assessed in a sample obtained from serum, liver, spleen or bone marrow of a patient treated in accordance with the methods described herein. In one embodiment, GDF11 level and/or activity is assessed in a sample obtained from serum of a patient treated in accordance with the methods described herein. In another embodiment, GDF11 level and/or activity is assessed in a sample obtained from spleen of a patient treated in accordance with the methods described herein. In yet another embodiment, GDF11 level and/or activity is assessed in a sample obtained from bone marrow of a patient treated in accordance with the methods described herein.

In some embodiments, the level and/or activity of GDF11 in a tissue sample of a patient is compared to the average level and/or activity of GDF11 in tissue samples (e.g., in samples from the same tissue) of healthy subjects (e.g., subjects that do not have anemia), such as subjects in the same age group and, optionally, of the same gender. In some embodiments, the level and/or activity of GDF11 in a tissue sample of a patient is compared to the level and/or activity of GDF11 in a tissue sample (e.g., in a sample from the same tissue) of the subject at an earlier time point (e.g., before the onset of disease, before the onset of treatment, or during treatment). In some embodiments, the level and/or activity of GDF11 in a tissue sample (e.g., serum, spleen, liver, or blood marrow) of a patient is compared to the level and/or activity of GDF11 in another tissue sample of the patient. In some embodiments, the level and/or activity of GDF11 in a tissue sample of a patient is compared to the level and/or activity of another gene product in the tissue sample of the patient (e.g., b-actin, Activin A, Activin B).

In some embodiments, the level and/or activity of GDF11 in a tissue sample of a patient is compared to the normal level and/or activity of GDF11 in the tissue sample. The normal level and/or activity of GDF11 in a tissue sample can be an average level and/or activity of GDF11 in tissue samples (e.g., in samples from the same tissue) of one or more healthy subjects (e.g., subjects that do not have anemia), such as subjects in the same age group and, optionally, of the same gender. In some embodiments, detection of the elevated level and/or activity of GDF11 in comparison to the normal level and/or activity of GDF11 is followed by administration of an activin receptor inhibitor (such as one or more activin receptor inhibitors described herein). In some embodiments, administration of an activin receptor inhibitor (such as one or more activin receptor inhibitors described herein) is followed by monitoring of the level and/or activity of GDF11 and, optionally, comparing the level and/or activity of GDF11 to the normal level and/or activity of GDF11. In some embodiments, administration of a first dose of activin type II receptor inhibitor (e.g., ActRIIA-hFc such as SEQ ID NO:7) is followed by determining the level and/or activity of GDF11, and if the level and/or activity of GDF11 is elevated over the normal level and/or activity administering a second dose of the activin type II receptor inhibitor which is higher (e.g., 1.25, 1.5, 1.75, 2, 2.5, 3, 4, 5, 6, 7, 8, 9, or 10 times higher) than the first dose, and if the level and/or activity of GDF11 is decreased over the normal level and/or activity administering a second dose of the activin type II receptor inhibitor which is lower (e.g., 1.25, 1.5, 1.75, 2, 2.5, 3, 4, 5, 6, 7, 8, 9, or 10 times lower) than the first dose.

As used herein, in certain embodiments, the normal level and/or activity of GDF11 is an average level and/or activity of GDF11 in a tissue sample of one or more healthy subjects (e.g., healthy subjects in the same age category and/or of the same gender). In some embodiments, the level and/or activity of GDF11 in a tissue sample of the treated subject is compared to the level and/or activity of GDF11 in a sample from the same tissue of one or more healthy subjects. In some embodiments, the tissue in which the level and/or activity of GDF11 is assessed is blood serum, bone marrow, liver, or spleen. In one embodiment, the tissue in which the level and/or activity of GDF11 is assessed is serum.

### 5.5 THERAPEUTIC USES

In certain embodiments, Activin type II receptor inhibitors are used for treatment of patients having anemia, or a disease or condition associated with anemia or ineffective erythropoiesis, in the methods described herein. In some embodiments, activin type II receptor inhibitors are administered to a patient having anemia, or a disease or condition associated with anemia or ineffective erythropoiesis. In some embodiments, activin type II receptor inhibitors are administered to a patient having anemia, or a disease or condition associated with anemia or ineffective erythropoiesis, and having elevated levels and/or activity of GDF11 (e.g., in serum, bone marrow, liver, and/or spleen of the patient). The activin type II receptor inhibitors described in this section and known in the art can be used in the methods provided herein. In certain embodiments, the activin type II receptor inhibitors described in this section can be used in the methods provided herein.

Inhibitors of ActRII receptors encompassed herein include ActRIIA inhibitors and ActRIIB inhibitors (see below). In certain embodiments, an ActRII receptor inhibitor is specific to ActRIIA. In other embodiments, an ActRII receptor inhibitor is specific to ActRIIB. In certain embodiments, an ActRII receptor inhibitor preferentially inhibits ActRIIA. In other embodiments, an ActRII receptor inhibitor preferentially inhibits ActRIIB. In certain embodiments, an ActRII receptor inhibitor inhibits both ActRIIA and ActRIIB.

In certain embodiments, inhibitors of ActRII receptors can be polypeptides comprising activin-binding domains of ActRII. Without being bound by theory, such activin-binding domain comprising polypeptides sequester activin and thereby prevent activin signaling. These activin-binding domain comprising polypeptides may comprise all or a portion of the extracellular domain of an ActRII receptor (i.e., all or a portion of the extracellular domain of ActRIIA or all or a portion of the extracellular domain of ActRIIB). In specific embodiments, the extracellular domain of an ActRII receptor is soluble.

In certain embodiments, the activin-binding domain comprising polypeptides are linked to an Fc portion of an antibody (i.e., a conjugate comprising an activin-binding domain comprising polypeptide of an ActRII receptor and an Fc portion of an antibody is generated). Without being bound by theory, the antibody portion confers increased stability on the conjugate. In certain embodiments, the activin-binding domain is linked to an Fc portion of an antibody via a linker, *e.g.,* a peptide linker.

The inhibitors of ActRII receptors used in the compositions and methods described herein comprise molecules that inhibit ActRIIA and/or ActRIIB, directly or indirectly, either extracellularly or intracellularly. In some embodiments, the inhibitors of ActRIIA and/or ActRIIB used in the compositions and methods described herein inhibit ActRIIA and/or ActRIIB via interactions with the receptor(s) itself. In other embodiments, the inhibitors of ActRIIA and/or ActRIIB used in the compositions and methods described herein inhibit ActRIIA and/or ActRIIB via interactions with an ActRIIA and/or ActRIIB ligand, e.g., Activin.

### 5.5.1 INHIBITORS OF ACTRIIA

As used herein, the term "ActRIIA" refers to a family of activin receptor type IIa (ActRIIA) proteins from any species and variants derived from such ActRIIA proteins by mutagenesis or other modification. Reference to ActRIIA herein is understood to be a reference to any one of the currently identified forms. Members of the ActRIIA family are generally transmembrane proteins, composed of a ligand-binding extracellular domain with a cysteine-rich region, a transmembrane domain, and a cytoplasmic domain with predicted serine/threonine kinase activity.

ActRIIA inhibitors to be used in the compositions and methods described herein include, without limitation, activin-binding soluble ActRIIA polypeptides; antibodies that bind to activin (particularly the activin A or B subunits, also referred to as β_{A} or β_{B}) and disrupt ActRIIA binding; antibodies that bind to ActRIIA and disrupt activin binding; non-antibody proteins selected for activin or ActRIIA binding (see e.g., WO/2002/088171, WO/2006/055689, WO/2002/032925, WO/2005/037989, US 2003/0133939, and US 2005/0238646, for examples of such proteins and methods for design and selection of same); and randomized peptides selected for activin or ActRIIA binding, which can be conjugated to an Fc domain.

In certain aspects, two or more different proteins (or other moieties) with activin or ActRIIA binding activity, especially activin binders that block the type I (e.g., a soluble type I activin receptor) and type II (e.g., a soluble type II activin receptor) binding sites, respectively, may be linked together to create a bifunctional or multifunctional binding molecule that inhibits ActRIIA and thus can be used in the compositions and methods described herein. In certain aspects, Activin-ActRIIA signaling axis antagonists that inhibit ActRIIA include nucleic acid aptamers, small molecules and other agents are used in the compositions and methods described herein include.

### (a) ActRIIA Inhibitors Comprising ActRIIA Polypeptides

The term "ActRIIA polypeptide" includes polypeptides comprising any naturally occurring polypeptide of an ActRIIA family member as well as any variants thereof (including mutants, fragments, fusions, and peptidomimetic forms) that retain a useful activity. For example, ActRIIA polypeptides include polypeptides derived from the sequence of any known ActRIIA having a sequence at least about 80% identical to the sequence of an ActRIIA polypeptide, and optionally at least 85%, 90%, 95%, 97%, 98%, 99% or greater identity. For example, an ActRIIA polypeptide may bind to and inhibit the function of an ActRIIA protein and/or activin. An ActRIIB polypeptide may be selected for its ability to promote bone growth and bone mineralization. Examples of ActRIIA polypeptides include human ActRIIA precursor polypeptide (SEQ ID NO: 1) and soluble human ActRIIA polypeptides (e.g., SEQ ID NOs: 2, 3, 7 and 12). With respect to the ActRIIA precursor polypeptide whose amino acid sequence is depicted at SEQ ID NO:1, the signal peptide of the human ActRIIA precursor polypeptide located at amino acid positions 1 to 20; the extracellular domain is located at amino acid positions 21 to 135 and the N-linked glycosylation sites of the human ActRIIA precursor polypeptide (SEQ ID NO: 1) are located at amino acid positions 43 and 56 of SEQ ID NO:1. The nucleic acid sequence encoding the human ActRIIB precursor polypeptide of SEQ ID NO:1 is disclosed as SEQ ID NO:4 (nucleotides 164-1705 of Genbank entry NM_001616). The nucleic acid sequence encoding the soluble human ActRIIA polypeptide of SEQ ID NO:2 is disclosed as SEQ ID NO:5. See Table 1 for a description of the sequences.

In specific embodiments, the ActRIIA polypeptides used in the compositions and methods described herein are soluble ActRIIA polypeptides. An extracellular domain of an ActRIIA protein can bind to activin and is generally soluble, and thus can be termed a soluble, activin-binding ActRIIA polypeptide. Thus, as used herein, the term "soluble ActRIIA polypeptide" generally refers to polypeptides comprising an extracellular domain of an ActRIIA protein, including any naturally occurring extracellular domain of an ActRIIA protein as well as any variants thereof (including mutants, fragments and peptidomimetic forms). Soluble ActRIIA polypeptides can bind to activin; however, the wild type ActRIIA protein does not exhibit significant selectivity in binding to activin versus GDF8/11. Native or altered ActRIIA proteins may be given added specificity for activin by coupling them with a second, activin-selective binding agent. Examples of soluble, activin-binding ActRIIA polypeptides include the soluble polypeptides illustrated in SEQ ID NOs: 2, 3, 7, 12 and 13. Other examples of soluble, activin-binding ActRIIA polypeptides comprise a signal sequence in addition to the extracellular domain of an ActRIIA protein, for example, the honey bee mellitin leader sequence (SEQ ID NO: 8), the tissue plasminogen activator (TPA) leader (SEQ ID NO: 9) or the native ActRIIA leader (SEQ ID NO: 10). The ActRIIA-hFc polypeptide illustrated in SEQ ID NO:13 uses a TPA leader.

In certain embodiments, the inhibitors of ActRIIA used in the compositions and methods described herein comprise a conjugate/fusion protein comprising an activin-binding domain of ActRIIA linked to an Fc portion of an antibody. In certain embodiments, the activin-binding domain is linked to an Fc portion of an antibody via a linker, *e.g.,* a peptide linker. Optionally, the Fc domain has one or more mutations at residues such as Asp-265, lysine 322, and Asn-434. In certain cases, the mutant Fc domain having one or more of these mutations (e.g., an Asp-265 mutation) has a reduced ability to bind to the Fcγ receptor relative to a wild-type Fc domain. In other cases, the mutant Fc domain having one or more of these mutations (e.g., an Asn-434 mutation) has an increased ability to bind to the MHC class I- related Fc-receptor (FcRN) relative to a wild-type Fc domain. Exemplary fusion proteins comprising a soluble extracellular domain of ActRIIA fused to an Fc domain are set forth in SEQ ID NOs: 6, 7, 12, and 13.

In a specific embodiment, the ActRIIA inhibitors used in the compositions and methods described herein comprise the extracellular domain of ActRIIA, or a portion thereof, linked to an Fc portion of an antibody, wherein said ActRIIA inhibitor comprises an amino acid sequence that is at least 75% identical to an amino acid sequence selected from SEQ ID NOs: 6, 7, 12, and 13. In another specific embodiment, the ActRIIA inhibitors used in the compositions and methods described herein comprise the extracellular domain of ActRIIA, or a portion thereof, linked to an Fc portion of an antibody, wherein said ActRIIA inhibitor comprises an amino acid sequence that is at least 80%, 85%, 90%, 95%, 96%, 97%, 98%, or 99% identical to an amino acid sequence selected from SEQ ID NOs: 6, 7, 12, and 13.

In certain embodiments, the inhibitors of ActRIIA used in the compositions and methods described herein comprise a truncated form of an extracellular domain of ActRIIA. The truncation can be at the carboxy terminus and/or the amino terminus of the ActRIIA polypeptide. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 amino acids long relative to the mature ActRIIB polypeptide extracellular domain. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 N-terminal amino acids of the mature ActRIIA polypeptide extracellular domain. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 C-terminal amino acids of the mature ActRIIA polypeptide extracellular domain. For example, truncated forms of ActRIIA include polypeptides with amino acids 20-119; 20-128; 20-129; 20-130; 20-131; 20-132; 20-133; 20-134; 20-131; 21-131; 22-131; 23-131; 24-131; and 25-131, wherein the amino acid positions refer to the amino acid positions in SEQ ID NO:1.

In certain embodiments, the inhibitors of ActRIIA used in the compositions and methods described herein comprise an extracellular domain of ActRIIA with one or more amino acid substitutions. In certain embodiments, the inhibitors of ActRIIA used in the compositions and methods described herein comprise a truncated form of an ActRIIA extracellular domain that also carries an amino acid substitution.

In a specific embodiment, the ActRIIA inhibitor to be used in the compositions and methods described herein is a fusion protein between the extracellular domain of the human ActRIIA receptor and the Fc portion of IgG1. In another specific embodiment, the ActRIIA inhibitor to be used in the compositions and methods described herein is a fusion protein between a truncated extracellular domain of the human ActRIIA receptor and the Fc portion of IgG1. In another specific embodiment, the ActRIIA inhibitor to be used in the compositions and methods described herein is a fusion protein between a truncated extracellular domain of the human ActRIIA receptor and the Fc portion of IgG1, wherein the truncated extracellular domain of the human ActRIIA receptor possesses one or more amino acid substitutions.

Functionally active fragments of ActRIIA polypeptides can be obtained, for example, by screening polypeptides recombinantly produced from the corresponding fragment of the nucleic acid encoding an ActRIIA polypeptide. In addition, fragments can be chemically synthesized using techniques known in the art such as conventional Merrifield solid phase f-Moc or t-Boc chemistry. The fragments can be produced (recombinantly or by chemical synthesis) and tested to identify those peptidyl fragments that can function as antagonists (inhibitors) of ActRIIA protein or signaling mediated by activin.

In addition, functionally active variants of ActRIIA polypeptides can be obtained, for example, by screening libraries of modified polypeptides recombinantly produced from the corresponding mutagenized nucleic acids encoding an ActRIIA polypeptide. The variants can be produced and tested to identify those that can function as antagonists (inhibitors) of ActRIIA protein or signaling mediated by activin. In certain aspects, a functional variant of the ActRIIA polypeptides comprises an amino acid sequence that is at least 75% identical to an amino acid sequence selected from SEQ ID NOs: 2 or 3. In certain cases, the functional variant has an amino acid sequence at least 80%, 85%, 90%, 95%, 97%, 98%, 99% or 100% identical to an amino acid sequence selected from SEQ ID NOs: 2 or 3.

Functional variants may be generated, for example, by modifying the structure of an ActRIIA polypeptide for such purposes as enhancing therapeutic efficacy, or stability (e.g., ex vivo shelf life and resistance to proteolytic degradation in vivo). Such modified ActRIIA polypeptides when selected to retain activin binding, can be considered functional equivalents of the naturally-occurring ActRIIA polypeptides. Modified ActRIIA polypeptides can also be produced, for instance, by amino acid substitution, deletion, or addition. For instance, it is reasonable to expect that an isolated replacement of a leucine with an isoleucine or valine, an aspartate with a glutamate, a threonine with a serine, or a similar replacement of an amino acid with a structurally related amino acid (e.g., conservative mutations) will not have a major effect on the biological activity of the resulting molecule. Conservative replacements are those that take place within a family of amino acids that are related in their side chains. Whether a change in the amino acid sequence of an ActRIIA polypeptide results in a functional homolog can be readily determined by assessing the ability of the variant ActRIIA polypeptide to produce a response in cells in a fashion similar to the wild-type ActRIIA polypeptide.

In certain embodiments, the ActRIIA inhibitor to be used in the compositions and methods described herein provided herein may comprise an ActRIIA polypeptide having one or more specific mutations that can alter the glycosylation of the polypeptide. Such mutations may introduce or eliminate one or more glycosylation sites, such as O-linked or N-linked glycosylation sites. Asparagine-linked glycosylation recognition sites generally comprise a tripeptide sequence, asparagine-X-threonine (or asparagines-X-serine) (where "X" is any amino acid) which is specifically recognized by appropriate cellular glycosylation enzymes. The alteration may also be made by the addition of, or substitution by, one or more serine or threonine residues to the sequence of the wild-type ActRIIA polypeptide (for O-linked glycosylation sites). A variety of amino acid substitutions or deletions at one or both of the first or third amino acid positions of a glycosylation recognition site (and/or amino acid deletion at the second position) results in non-glycosylation at the modified tripeptide sequence. Another means of increasing the number of carbohydrate moieties on an ActRIIA polypeptide is by chemical or enzymatic coupling of glycosides to the ActRIIA polypeptide. Depending on the coupling mode used, the sugar(s) may be attached to (a) arginine and histidine; (b) free carboxyl groups; (c) free sulfhydryl groups such as those of cysteine; (d) free hydroxyl groups such as those of serine, threonine, or hydroxyproline; (e) aromatic residues such as those of phenylalanine, tyrosine, or tryptophan; or (f) the amide group of glutamine. These methods are described in WO 87/05330 published Sep. 11, 1987, and in Aplin and Wriston (1981) CRC Crit. Rev. Biochem., pp. 259-306. Removal of one or more carbohydrate moieties present on an ActRIIA polypeptide may be accomplished chemically and/or enzymatically. Chemical deglycosylation may involve, for example, exposure of the ActRIIA polypeptide to the compound trifluoromethanesulfonic acid, or an equivalent compound. This treatment results in the cleavage of most or all sugars except the linking sugar (N-acetylglucosamine or N-acetylgalactosamine), while leaving the amino acid sequence intact. Chemical deglycosylation is further described by Hakimuddin et al. (1987) Arch. Biochem. Biophys. 259:52 and by Edge et al. (1981) Anal. Biochem. 118:131. Enzymatic cleavage of carbohydrate moieties on ActRIIA polypeptides can be achieved by the use of a variety of endo- and exo-glycosidases as described by Thotakura et al. (1987) Meth. Enzymol. 138:350. The sequence of an ActRIIA polypeptide may be adjusted, as appropriate, depending on the type of expression system used, as mammalian, yeast, insect and plant cells may all introduce differing glycosylation patterns that can be affected by the amino acid sequence of the peptide. In general, ActRIIA proteins for use in humans can be expressed in a mammalian cell line that provides proper glycosylation, such as HEK293 or CHO cell lines, although other expression systems, such as other mammalian expression cell lines, yeast cell lines with engineered glycosylation enzymes and insect cells, are expected to be useful as well.

Further provided herein are methods of generating mutants, particularly sets of combinatorial mutants of an ActRIIA polypeptide, as well as truncation mutants; pools of combinatorial mutants are especially useful for identifying functional variant sequences. The purpose of screening such combinatorial libraries may be to generate, for example, ActRIIA polypeptide variants which can act as either agonists or antagonist, or alternatively, which possess novel activities all together. A variety of screening assays are provided below, and such assays may be used to evaluate variants. For example, an ActRIIA polypeptide variant may be screened for ability to bind to an ActRIIA ligand, to prevent binding of an ActRIIA ligand to an ActRIIA polypeptide or to interfere with signaling caused by an ActRIIA ligand.

Combinatorially-derived variants can be generated which have a selective or generally increased potency relative to a naturally occurring ActRIIA polypeptide. Likewise, mutagenesis can give rise to variants which have intracellular half-lives dramatically different than the corresponding a wild-type ActRIIA polypeptide. For example, the altered protein can be rendered either more stable or less stable to proteolytic degradation or other cellular processes which result in destruction of, or otherwise inactivation of a native ActRIIA polypeptide. Such variants, and the genes which encode them, can be utilized to alter ActRIIA polypeptide levels by modulating the half-life of the ActRIIA polypeptides. For instance, a short half-life can give rise to more transient biological effects and can allow tighter control of recombinant ActRIIA polypeptide levels within the patient. In an Fc fusion protein, mutations may be made in the linker (if any) and/or the Fc portion to alter the half-life of the protein.

A combinatorial library may be produced by way of a degenerate library of genes encoding a library of polypeptides which each include at least a portion of potential ActRIIA polypeptide sequences. For instance, a mixture of synthetic oligonucleotides can be enzymatically ligated into gene sequences such that the degenerate set of potential ActRIIA polypeptide nucleotide sequences are expressible as individual polypeptides, or alternatively, as a set of larger fusion proteins (e.g., for phage display).

There are many ways by which the library of potential homologs can be generated from a degenerate oligonucleotide sequence. Chemical synthesis of a degenerate gene sequence can be carried out in an automatic DNA synthesizer, and the synthetic genes then be ligated into an appropriate vector for expression. The synthesis of degenerate oligonucleotides is well known in the art (see for example, Narang, S A (1983) Tetrahedron 39:3; Itakura et al., (1981) Recombinant DNA, Proc. 3rd Cleveland Sympos. Macromolecules, ed. AG Walton, Amsterdam: Elsevier pp 273-289; Itakura et al., (1984) Annu. Rev. Biochem. 53:323; Itakura et al., (1984) Science 198:1056; Ike et al., (1983) Nucleic Acid Res. 11:477). Such techniques have been employed in the directed evolution of other proteins (see, for example, Scott et al., (1990) Science 249:386-390; Roberts et al., (1992) PNAS USA 89:2429-2433; Devlin et al., (1990) Science 249: 404-406; Cwirla et al., (1990) PNAS USA 87: 6378-6382; as well as U.S. Pat. Nos. 5,223,409, 5,198,346, and 5,096,815).

Alternatively, other forms of mutagenesis can be utilized to generate a combinatorial library. For example, ActRIIA polypeptide variants can be generated and isolated from a library by screening using, for example, alanine scanning mutagenesis and the like (Ruf et al., (1994) Biochemistry 33:1565-1572; Wang et al., (1994) J. Biol. Chem. 269:3095-3099; Balint et al., (1993) Gene 137:109-118; Grodberg et al., (1993) Eur. J. Biochem. 218:597-601; Nagashima et al., (1993) J. Biol. Chem. 268:2888-2892; Lowman et al., (1991) Biochemistry 30:10832-10838; and Cunningham et al., (1989) Science 244:1081-1085), by linker scanning mutagenesis (Gustin et al., (1993) Virology 193:653-660; Brown et al., (1992) Mol. Cell Biol. 12:2644-2652; McKnight et al., (1982) Science 232:316); by saturation mutagenesis (Meyers et al., (1986) Science 232:613); by PCR mutagenesis (Leung et al., (1989) Method Cell Mol Biol 1:11-19); or by random mutagenesis, including chemical mutagenesis, etc. (Miller et al., (1992) A Short Course in Bacterial Genetics, CSHL Press, Cold Spring Harbor, N.Y.; and Greener et al., (1994) Strategies in Mol Biol 7:32-34). Linker scanning mutagenesis, particularly in a combinatorial setting, is an attractive method for identifying truncated (bioactive) forms of ActRIIA polypeptides.

A wide range of techniques are known in the art for screening gene products of combinatorial libraries made by point mutations and truncations, and, for that matter, for screening cDNA libraries for gene products having a certain property. Such techniques will be generally adaptable for rapid screening of the gene libraries generated by the combinatorial mutagenesis of ActRIIA polypeptides. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Preferred assays include activin binding assays and activin-mediated cell signaling assays.

In certain embodiments, ActRIIA polypeptides used in the inhibitors of the methods and compositions described herein may further comprise post-translational modifications in addition to any that are naturally present in the ActRIIA polypeptides. Such modifications may include, but are not limited to, acetylation, carboxylation, glycosylation, phosphorylation, lipidation, and acylation. As a result, the modified ActRIIA polypeptides may contain non-amino acid elements, such as polyethylene glycols, lipids, poly- or mono-saccharide, and phosphates. Effects of such non-amino acid elements on the functionality of a ActRIIA polypeptide may be tested by any method known to the skilled artisan. When an ActRIIA polypeptide is produced in cells by cleaving a nascent form of the ActRIIA polypeptide, post-translational processing may also be important for correct folding and/or function of the protein. Different cells (such as CHO, HeLa, MDCK, 293, W138, NIH-3T3 or HEK293) have specific cellular machinery and characteristic mechanisms for such post-translational activities and may be chosen to ensure the correct modification and processing of the ActRIIA polypeptides.

In certain aspects, functional variants or modified forms of the ActRIIA polypeptides used in the inhibitors of the methods and compositions described herein include fusion proteins having at least a portion of the ActRIIA polypeptides and one or more fusion domains. Well known examples of such fusion domains include, but are not limited to, polyhistidine, Glu-Glu, glutathione S transferase (GST), thioredoxin, protein A, protein G, an immunoglobulin heavy chain constant region (Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt-conjugated resins are used. Many of such matrices are available in "kit" form, such as the Pharmacia GST purification system and the QIAexpress.TM. system (Qiagen) useful with (HIS₆) fusion partners. As another example, a fusion domain may be selected so as to facilitate detection of the ActRIIA polypeptides. Examples of such detection domains include the various fluorescent proteins (e.g., GFP) as well as "epitope tags," which are usually short peptide sequences for which a specific antibody is available. Well known epitope tags for which specific monoclonal antibodies are readily available include FLAG, influenza virus hemagglutinin (HA), and c-myc tags. In some cases, the fusion domains have a protease cleavage site, such as for Factor Xa or Thrombin, which allows the relevant protease to partially digest the fusion proteins and thereby liberate the recombinant proteins therefrom. The liberated proteins can then be isolated from the fusion domain by subsequent chromatographic separation. In certain preferred embodiments, an ActRIIA polypeptide is fused with a domain that stabilizes the ActRIIA polypeptide in vivo (a "stabilizer" domain). By "stabilizing" is meant anything that increases serum half life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Likewise, fusions to human serum albumin can confer desirable properties. Other types of fusion domains that may be selected include multimerizing (e.g., dimerizing, tetramerizing) domains and functional domains (that confer an additional biological function, such as further stimulation of bone growth or muscle growth, as desired).

It is understood that different elements of the fusion proteins may be arranged in any manner that is consistent with the desired functionality. For example, an ActRIIA polypeptide may be placed C-terminal to a heterologous domain, or, alternatively, a heterologous domain may be placed C-terminal to an ActRIIA polypeptide. The ActRIIA polypeptide domain and the heterologous domain need not be adjacent in a fusion protein, and additional domains or amino acid sequences may be included C- or N-terminal to either domain or between the domains.

In certain embodiments, the ActRIIA polypeptides used in the inhibitors of the methods and compositions described herein may contain one or more modifications that are capable of stabilizing the ActRIIA polypeptides. For example, such modifications may enhance the in vitro half life of the ActRIIA polypeptides, enhance circulatory half life of the ActRIIA polypeptides or reduce proteolytic degradation of the ActRIIA polypeptides. Such stabilizing modifications may include, but are not limited to, fusion proteins (including, for example, fusion proteins comprising an ActRIIA polypeptide and a stabilizer domain), modifications of a glycosylation site (including, for example, addition of a glycosylation site to an ActRIIA polypeptide), and modifications of carbohydrate moiety (including, for example, removal of carbohydrate moieties from an ActRIIA polypeptide). In the case of fusion proteins, an ActRIIA polypeptide is fused to a stabilizer domain such as an IgG molecule (e.g., an Fc domain). As used herein, the term "stabilizer domain" not only refers to a fusion domain (e.g., Fc) as in the case of fusion proteins, but also includes nonproteinaceous modifications such as a carbohydrate moiety, or nonproteinaceous polymer, such as polyethylene glycol.

In certain embodiments, isolated and/or purified forms of ActRIIA polypeptides, which are isolated from, or otherwise substantially free of, other proteins can be used with the methods and compositions described herein. ActRIIA polypeptides can generally be produced by expression from recombinant nucleic acids.

In certain aspects, the ActRIIA polypeptides used in the compositions and methods described herein are generated using isolated and/or recombinant nucleic acids encoding any of the ActRIIA polypeptides (e.g., soluble ActRIIA polypeptides), including fragments, functional variants and fusion proteins disclosed herein. For example, SEQ ID NO: 4 encodes the naturally occurring human ActRIIA precursor polypeptide, while SEQ ID NO: 5 encodes the processed extracellular domain of ActRIIA. Such nucleic acids may be single-stranded or double stranded. Such nucleic acids may be DNA or RNA molecules. These nucleic acids may be used, for example, in methods for making ActRIIA polypeptides or as direct therapeutic agents (e.g., in a gene therapy approach).

In certain aspects, nucleic acids encoding ActRIIA polypeptides may include nucleic acids that are variants of SEQ ID NO: 4 or 5. Variant nucleotide sequences include sequences that differ by one or more nucleotide substitutions, additions or deletions, such as allelic variants.

In certain aspects, isolated or recombinant nucleic acid sequences encoding ActRIIA polypeptides may be least 80%, 85%, 90%, 95%, 97%, 98%, 99% or 100% identical to SEQ ID NO: 4 or 5. One of ordinary skill in the art will appreciate that nucleic acid sequences complementary to SEQ ID NO: 4 or 5, and variants of SEQ ID NO: 4 or 5 may be used in the production of ActRIIA polypeptides suitable for use in the methods and compositions described herein. In further embodiments, such nucleic acid sequences can be isolated, recombinant, and/or fused to a heterologous nucleotide sequence, or be from a DNA library.

In other embodiments, nucleic acids used in the production of ActRIIA polypeptides suitable for use in the methods and compositions described herein may include nucleotide sequences that hybridize under highly stringent conditions to the nucleotide sequence designated in SEQ ID NO: 4 or 5, complement sequence of SEQ ID NO: 4 or 5, or fragments thereof. One of ordinary skill in the art will understand that appropriate stringency conditions which promote DNA hybridization can be varied. For example, one can perform the hybridization at 6.0 times sodium chloride/sodium citrate (SSC) at about 45 degree Celsius, followed by a wash of 2.0 times SSC at 50 degree Celsius. For example, the salt concentration in the wash step can be selected from a low stringency of about 2.0 times SSC at 50 degree Celsius to a high stringency of about 0.2 times SSC at 50 degree Celsius. In addition, the temperature in the wash step can be increased from low stringency conditions at room temperature, about 22 degree Celsius, to high stringency conditions at about 65 degree Celsius. Both temperature and salt may be varied, or temperature or salt concentration may be held constant while the other variable is changed. In one embodiment, nucleic acids which hybridize under low stringency conditions of 6 times SSC at room temperature followed by a wash at 2 times SSC at room temperature can be used with the methods and compositions described herein.

Isolated nucleic acids which differ from the nucleic acids as set forth in SEQ ID NOs: 4 or 5 due to degeneracy in the genetic code also can be used in the production of ActRIIA polypeptides suitable for use in the methods and compositions described herein. For example, a number of amino acids are designated by more than one triplet. Codons that specify the same amino acid, or synonyms (for example, CAU and CAC are synonyms for histidine) may result in "silent" mutations which do not affect the amino acid sequence of the protein. However, it is expected that DNA sequence polymorphisms that do lead to changes in the amino acid sequences of the subject proteins will exist among mammalian cells. One skilled in the art will appreciate that these variations in one or more nucleotides (up to about 3-5% of the nucleotides) of the nucleic acids encoding a particular protein may exist among individuals of a given species due to natural allelic variation.

In certain embodiments, the recombinant nucleic acids may be operably linked to one or more regulatory nucleotide sequences in an expression construct. Regulatory nucleotide sequences will generally be appropriate to the host cell used for expression. Numerous types of appropriate expression vectors and suitable regulatory sequences are known in the art for a variety of host cells. Typically, said one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art are contemplated herein. The promoters may be either naturally occurring promoters, or hybrid promoters that combine elements of more than one promoter. An expression construct may be present in a cell on an episome, such as a plasmid, or the expression construct may be inserted in a chromosome. In a preferred embodiment, the expression vector contains a selectable marker gene to allow the selection of transformed host cells. Selectable marker genes are well known in the art and will vary with the host cell used.

In certain aspects, the a nucleic acid used in the production of ActRIIA polypeptides suitable for use in the methods and compositions described herein can be provided in an expression vector comprising a nucleotide sequence encoding an ActRIIA polypeptide and operably linked to at least one regulatory sequence. Regulatory sequences are art-recognized and are selected to direct expression of the ActRIIA polypeptide. Accordingly, the term regulatory sequence includes promoters, enhancers, and other expression control elements. Exemplary regulatory sequences are described in Goeddel; Gene Expression Technology: Methods in Enzymology, Academic Press, San Diego, Calif. (1990). For instance, any of a wide variety of expression control sequences that control the expression of a DNA sequence when operatively linked to it may be used in these vectors to express DNA sequences encoding an ActRIIA polypeptide. Such useful expression control sequences, include, for example, the early and late promoters of SV40, tet promoter, adenovirus or cytomegalovirus immediate early promoter, RSV promoters, the lac system, the trp system, the TAC or TRC system, T7 promoter whose expression is directed by T7 RNA polymerase, the major operator and promoter regions of phage lambda, the control regions for fd coat protein, the promoter for 3-phosphoglycerate kinase or other glycolytic enzymes, the promoters of acid phosphatase, e.g., Pho5, the promoters of the yeast .alpha.-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other protein encoded by the vector, such as antibiotic markers, should also be considered.

A recombinant nucleic acid used in the production of ActRIIA polypeptides suitable for use in the methods and compositions described herein can be produced by ligating the cloned gene, or a portion thereof, into a vector suitable for expression in either prokaryotic cells, eukaryotic cells (yeast, avian, insect or mammalian), or both. Expression vehicles for production of a recombinant ActRIIA polypeptide include plasmids and other vectors. For instance, suitable vectors include plasmids of the types: pBR322-derived plasmids, pEMBL-derived plasmids, pEX-derived plasmids, pBTac-derived plasmids and pUC-derived plasmids for expression in prokaryotic cells, such as E. coli.

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures, see Molecular Cloning A Laboratory Manual, 3rd Ed., ed. by Sambrook, Fritsch and Maniatis (Cold Spring Harbor Laboratory Press, 2001). In some instances, it may be desirable to express the recombinant polypeptides by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the .beta.-gal containing pBlueBac III).

Vectors can be designed for production of the subject ActRIIA polypeptides in CHO cells, such as a Pcmv-Script vector (Stratagene, La Jolla, Calif.), pcDNA4 vectors (Invitrogen, Carlsbad, Calif.) and pCI-neo vectors (Promega, Madison, Wis.). As will be apparent, the subject gene constructs can be used to cause expression of the subject ActRIIA polypeptides in cells propagated in culture, e.g., to produce proteins, including fusion proteins or variant proteins, for purification.

Host cells transfected with a recombinant gene including a coding sequence (e.g., SEQ ID NO: 4 or 5) for one or more of the subject ActRIIA polypeptides can be used in the production of ActRIIA polypeptides suitable for use in the methods and compositions described herein. The host cell may be any prokaryotic or eukaryotic cell. For example, an ActRIIA polypeptide provided herein may be expressed in bacterial cells such as E. coli, insect cells (e.g., using a baculovirus expression system), yeast, or mammalian cells. Other suitable host cells are known to those skilled in the art.

Accordingly, provided herein are methods of producing the ActRIIA polypeptides. For example, a host cell transfected with an expression vector encoding an ActRIIA polypeptide can be cultured under appropriate conditions to allow expression of the ActRIIA polypeptide to occur. The ActRIIA polypeptide may be secreted and isolated from a mixture of cells and medium containing the ActRIIA polypeptide. Alternatively, the ActRIIA polypeptide may be retained cytoplasmically or in a membrane fraction and the cells harvested, lysed and the protein isolated. A cell culture includes host cells, media and other byproducts. Suitable media for cell culture are well known in the art. The subject ActRIIA polypeptides can be isolated from cell culture medium, host cells, or both, using techniques known in the art for purifying proteins, including ion-exchange chromatography, gel filtration chromatography, ultrafiltration, electrophoresis, immunoaffinity purification with antibodies specific for particular epitopes of the ActRIIA polypeptides and affinity purification with an agent that binds to a domain fused to the ActRIIA polypeptide (e.g., a protein A column may be used to purify an ActRIIA-Fc fusion). In a preferred embodiment, the ActRIIA polypeptide is a fusion protein containing a domain which facilitates its purification. In one embodiment, purification is achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. As demonstrated herein, ActRIIA-hFc protein was purified to a purity of >98% as determined by size exclusion chromatography and >95% as determined by SDS PAGE. This level of purity was sufficient to achieve desirable effects on bone in mice and an acceptable safety profile in mice, rats and non-human primates.

In another embodiment, a fusion gene coding for a purification leader sequence, such as a poly-(His)/enterokinase cleavage site sequence at the N-terminus of the desired portion of a recombinant ActRIIA polypeptide, can allow purification of the expressed fusion protein by affinity chromatography using a Ni²⁺ metal resin. The purification leader sequence can then be subsequently removed by treatment with enterokinase to provide the purified ActRIIA polypeptide (e.g., see Hochuli et al., (1987) J. Chromatography 411:177; and Janknecht et al., PNAS USA 88:8972).

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence (see, for example, Current Protocols in Molecular Biology, eds. Ausubel et al., John Wiley & Sons: 1992).

ActRIIA-Fc fusion protein can be expressed in stably transfected CHO-DUKX B1 1 cells from a pAID4 vector (SV40 ori/enhancer, CMV promoter), using a tissue plasminogen leader sequence of SEQ ID NO:9. The Fc portion is a human IgGl Fc sequence, as shown in SEQ ID NO:7. In certain embodiments, upon expression, the protein contained has, on average, between about 1.5 and 2.5 moles of sialic acid per molecule of ActRIIA-Fc fusion protein.

In certain embodiments, the long serum half-life of an ActRIIA-Fc fusion can be 25-32 days in human patients. Additionally, the CHO cell expressed material can have a higher affinity for activin B ligand than that reported for an ActRIIA-hFc fusion protein expressed in human 293 cells (del Re et al., J Biol Chem. 2004 Dec 17;279(51):53126-35). Additionally, without being bound by theory, the use of the TPA leader sequence provided greater production than other leader sequences and, unlike ActRIIA-Fc expressed with a native leader, may provide a highly pure N-terminal sequence. Use of the native leader sequence may result in two major species of ActRIIA-Fc, each having a different N-terminal sequence.

### 5.5.2 INHIBITORS OF ACTRIIB

As used herein, the term "ActRIIB" refers to a family of activin receptor type IIB (ActRIIB) proteins from any species and variants derived from such ActRIIB proteins by mutagenesis or other modification. Reference to ActRIIB herein is understood to be a reference to any one of the currently identified forms of the receptor. Members of the ActRIIB family are generally transmembrane proteins, composed of a ligand-binding extracellular domain with a cysteine-rich region, a transmembrane domain, and a cytoplasmic domain with predicted serine/threonine kinase activity.

ActRIIB inhibitors to be used in the compositions and methods described herein include, without limitation, activin-binding soluble ActRIIB polypeptides; antibodies that bind to activin (particularly the activin A or B subunits, also referred to as β_{A} or β_{B}) and disrupt ActRIIB binding; antibodies that bind to ActRIIB and disrupt activin binding; non-antibody proteins selected for activin or ActRIIB binding; and randomized peptides selected for activin or ActRIIB binding, which can be conjugated to an Fc domain.

In certain aspects, two or more different proteins (or other moieties) with activin or ActRIIB binding activity, especially activin binders that block the type I (e.g., a soluble type I activin receptor) and type II (e.g., a soluble type II activin receptor) binding sites, respectively, may be linked together to create a bifunctional or multifunctional binding molecule that inhibits ActRIIB and thus can be used in the compositions and methods described herein include. In certain aspects, Activin-ActRIIB signaling axis antagonists that inhibit ActRIIB include nucleic acid aptamers, small molecules and other agents are used in the compositions and methods described herein include.

### (a) ActRIIB Inhibitors Comprising ActRIIB Polypeptides

As used herein, the term "ActRIIB polypeptide" refers to polypeptides comprising any naturally occurring polypeptide of an ActRIIB family member as well as any variants thereof (including mutants, fragments, fusions, and peptidomimetic forms) that retain a useful activity. For example, ActRIIB polypeptides include polypeptides derived from the sequence of any known ActRIIB receptor having a sequence at least about 80% identical to the sequence of an ActRIIB polypeptide, and optionally at least 85%, 90%, 95%, 96%, 97%, 98%, 99% or greater identity. For example, an ActRIIB polypeptide may bind to and inhibit the function of an ActRIIB protein and/or activin. An example of an ActRIIB polypeptide includes the human ActRIIB precursor polypeptide (SEQ ID NO:16 or SEQ ID NO:28). With respect to the ActRIIB precursor polypeptide whose amino acid sequence is depicted as SEQ ID NO:16 or SEQ ID NO:28 (i.e., the human ActRIIB precursor polypeptide), the signal peptide of the ActRIIB precursor polypeptide is located at amino acids 1 to 18; the extracellular domain is located at amino acids 19 to 134 and the potential N-linked glycosylation sites are located at amino acid positions 42 and 65. The nucleic acid sequence encoding the human ActRIIB precursor polypeptide of SEQ ID NO:16 is disclosed as SEQ ID NO:19 (SEQ ID NO:19 provides an alanine at the codon corresponding to amino acid position 64, but could be readily modified by one of skill in the art using methods known in the art to provide an arginine at the codon corresponding to amino acid position 64 instead). See Table 1 for a description of the sequences.

The numbering of amino acids for all of the ActRIIB-related polypeptides described herein is based on the amino acid numbering for SEQ ID NO:16 and SEQ ID NO:28 (which only differ in the amino acid expressed at position 64), unless specifically designated otherwise. For example, if an ActRIIB polypeptide is described as having a substitution/mutation at amino acid position 79, then it is to be understood that position 79 refers to the 79^{th} amino acid in SEQ ID NO:16 or SEQ ID NO:28, from which the ActRIIB polypeptide is derived. Likewise, if an ActRIIB polypeptide is described as having an alanine or an arginine at amino acid position 64, then it is to be understood that position 64 refers to the 64^{th} amino acid in SEQ ID NO:16 or SEQ ID NO:28, from which the ActRIIB polypeptide is derived.

In certain embodiments, the inhibitors of ActRIIB used in the compositions and methods described herein comprise polypeptides comprising an activin-binding domain of ActRIIB. In some embodiments, the activin-binding domains of ActRIIB comprise the extracellular domain of ActRIIB, or a portion thereof. In specific embodiments, the extracellular domain or portion thereof of ActRIIB is soluble. Illustrative modified forms of ActRIIB polypeptides are disclosed in U.S. Patent Application Publication Nos. 20090005308 and 20100068215.

In specific embodiments, the ActRIIB polypeptides used in the compositions and methods described herein are soluble ActRIIB polypeptides. The term "soluble ActRIIB polypeptide" generally refers to polypeptides comprising an extracellular domain of an ActRIIB protein, including any naturally occurring extracellular domain of an ActRIIB protein as well as any variants thereof (including mutants, fragments and peptidomimetic forms). Soluble ActRIIB polypeptides can bind to activin; however, the wild type ActRIIB protein does not exhibit significant selectivity in binding to activin versus GDF8/11. In certain embodiments, altered forms of ActRIIB with different binding properties can be used in the methods provided herein. Such altered forms are disclosed, *e.g.,* in international patent application publication Nos. WO 2006/012627 and WO 2010/019261. Native or altered ActRIIB proteins may be given added specificity for activin by coupling them with a second, activin-selective binding agent. Exemplary soluble ActRIIB polypeptides include the extracellular domain of a human ActRIIB polypeptide (e.g., SEQ ID NOs: 17, 18, 23, 26, 27, 29, 30, 31, 32, 33, 36, 37, 42, and 43).

An Fc fusion protein having the ActRIIB extracellular sequence disclosed by Hilden et al. (Blood, 1994, 83(8):2163-70), which has an alanine at the position corresponding to amino acid 64 of the ActRIIB precursor amino acid sequence, i.e., SEQ ID NO: 16 (herein referred to as "A64"), has been demonstrated to possess a relatively low affinity for activin and GDF-11. By contrast, an Fc fusion protein with an arginine at position 64 of the ActRIIB precursor amino acid sequence (herein referred to as "R64") has an affinity for activin and GDF-11 in the low nanomolar to high picomolar range (see, e.g., U.S. Patent Application Publication No. 20100068215). An ActRIIB precursor amino acid sequence with an arginine at position 64 is presented in SEQ ID NO:28. As such, in certain embodiments, the ActRIIB polypeptides used in accordance with the compositions and methods described herein may comprise either (i) an alanine at the position corresponding to amino acid 64 of the ActRIIB precursor amino acid sequence, i.e., SEQ ID NO: 16; or (ii) an arginine at position 64 of the ActRIIB precursor amino acid sequence, i.e., SEQ ID NO: 28. In other embodiments, the ActRIIB polypeptides used in accordance with the compositions and methods described herein may comprise an amino acid that is not alanine or arginine at the position corresponding to amino acid 64 of the ActRIIB precursor amino acid sequence, i.e., SEQ ID NO: 16 or SEQ ID NO:28.

It has been shown that a deletion of the proline knot at the C-terminus of the extracellular domain of ActRIIB reduces the affinity of the receptor for activin (see, e.g., Attisano et al., Cell, 1992, 68(1):97-108). An ActRIIB-Fc fusion protein containing amino acids 20-119 of SEQ ID NO: 28 (i.e., SEQ ID NO:32), "ActRIIB(20-119)-Fc" has reduced binding to GDF-11 and activin relative to an ActRIIB-Fc fusion protein containing amino acids 20-134 of SEQ ID NO: 28 (i.e., SEQ ID NO:31), "ActRIIB(20-134)-Fc", which includes the proline knot region and the complete juxtamembrane domain. However, an ActRIIB-Fc fusion protein containing amino acids 20-129 of SEQ ID NO: 28, "ActRIIB(20-129)-Fc" retains similar but somewhat reduced activity relative to the non-truncated extracellular domain of ActRIIB, even though the proline knot region is disrupted. Thus, ActRIIB polypeptides comprising extracellular domains that stop at amino acid 134, 133, 132, 131, 130 and 129 of SEQ ID NO: 28 (or SEQ ID NO:16) are all expected to be active, but constructs stopping at amino acid 134 or 133 may be most active. Similarly, mutations at any of residues 129-134 are not expected to alter ligand binding affinity by large margins, as indicated by the fact that mutations of P129 and P130 of SEQ ID NO: 28 do not substantially decrease ligand binding. Therefore, the ActRIIB polypeptides used in accordance with the methods and compositions described herein may end as early as amino acid 109 (i.e., the final cysteine) of SEQ ID NO:28 (or SEQ ID NO:16), however, forms ending at or between amino acid positions 109 and 119 of SEQ ID NO:28 (or SEQ ID NO:16) are expected to have reduced ligand binding ability.

Amino acid 29 of SEQ ID NO:16 and SEQ ID NO:28 represents the initial cysteine in the ActRIIB precursor sequence. It is expected that an ActRIIB polypeptide beginning at amino acid 29 of the N-terminus of SEQ ID NO:16 or SEQ ID NO:28, or before these amino acid positions, will retain ligand binding activity. An alanine to asparagine mutation at position 24 of SEQ ID NO:16 or SEQ ID NO:28 introduces an N-linked glycosylation sequence without substantially affecting ligand binding. This confirms that mutations in the region between the signal cleavage peptide and the cysteine cross-linked region, corresponding to amino acids 20-29 of SEQ ID NO:16 or SEQ ID NO:28, are well tolerated. In particular, ActRIIB polypeptides beginning at amino acid position 20, 21, 22, 23 and 24 of SEQ ID NO:16 or SEQ ID NO:28 will retain activity, and ActRIIB polypeptides beginning at amino acid positions 25, 26, 27, 28 and 29 of SEQ ID NO:16 or SEQ ID NO:28 are also expected to retain activity. An ActRIIB polypeptide beginning at amino acid position 22, 23, 24 or 25 of SEQ ID NO:16 or SEQ ID NO:28 will have the most activity.

Taken together, the active portions (i.e., ActRIIB polypeptides) of the ActRIIB precursor protein (i.e., SEQ ID NO:16 or SEQ ID NO:28) to be used in accordance with the methods and compositions described herein will generally comprise amino acids 29-109 of SEQ ID NO:16 or SEQ ID NO:28, and such ActRIIB polypeptides may, for example, begin at a residue corresponding to any one of amino acids 19-29 of SEQ ID NO:16 or SEQ ID NO:28 and end at a position corresponding to any one of amino acids 109-134 of SEQ ID NO:16 or SEQ ID NO:28. Specific examples of ActRIIB polypeptides encompassed herein include those that begin at an amino acid position from 19-29, 20-29 or 21-29 of SEQ ID NO:16 or SEQ ID NO:28 and end at an amino acid position from 119-134, 119-133 or 129-134, 129-133 of SEQ ID NO:16 or SEQ ID NO:28. Other specific examples of ActRIIB polypeptides encompassed herein include those that begin at an amino acid position from 20-24 (or 21-24, or 22-25) of SEQ ID NO:16 or SEQ ID NO:28 and end at an amino acid position from 109-134 (or 109-133), 119-134 (or 119-133) or 129-134 (or 129-133) of SEQ ID NO:16 or SEQ ID NO:28. Variant ActRIIB polypeptides falling within these ranges are also contemplated, particularly those having at least 80%, 85%, 90%, 91%, 92%, 93%, 94%, 95%, 96%, 97%, 98%, or 99% sequence identity or sequence homology to the corresponding portion of SEQ ID NO:16 or SEQ ID NO:28.

In certain embodiments, the inhibitors of ActRIIB used in the compositions and methods described herein comprise a truncated form of an extracellular domain of ActRIIB. The truncation can be at the carboxy terminus and/or the amino terminus of the ActRIIB polypeptide. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 amino acids long relative to the mature ActRIIB polypeptide extracellular domain. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 N-terminal amino acids of the mature ActRIIB polypeptide extracellular domain. In certain embodiments, the truncation can be 1, 2, 3, 4, 5, 6, 7, 8, 9, 10, 11, 12, 13, 14, 15, 16, 17, 18, 19, 20, 21, 22, 23, 24, or 25 C-terminal amino acids of the mature ActRIIB polypeptide extracellular domain. For example, truncated forms of ActRIIB include polypeptides with amino acids 20-119; 20-128; 20-129; 20-130; 20-131; 20-132; 20-133; 20-134; 20-131; 21-131; 22-131; 23-131; 24-131; and 25-131, wherein the amino acid positions refer to the amino acid positions in SEQ ID NO:16 or SEQ ID NO:28.

Additional exemplary truncated forms of ActRIIB include (i) polypeptides beginning at amino acids at any of amino acids 21-29 of SEQ ID NO:16 or SEQ ID NO:28 (optionally beginning at 22-25 of SEQ ID NO:16 or SEQ ID NO:28) and ending at any of amino acids 109-134 of SEQ ID NO:16 or SEQ ID NO:28; (ii) polypeptides beginning at any of amino acids 20-29 of SEQ ID NO:16 or SEQ ID NO:28 (optionally beginning at 22-25 of SEQ ID NO:16 or SEQ ID NO:28) and ending at any of amino acids 109-133 of SEQ ID NO:16 or SEQ ID NO:28; (iii) polypeptides beginning at any of amino acids 20-24 of SEQ ID NO:16 or SEQ ID NO:28 (optionally beginning at 22-25 of SEQ ID NO:16 or SEQ ID NO:28) and ending at any of amino acids 109-133 of SEQ ID NO:16 or SEQ ID NO:28; (iv) polypeptides beginning at any of amino acids 21-24 of SEQ ID NO:16 or SEQ ID NO:28 and ending at any of amino acids 109-134 of SEQ ID NO:16 or SEQ ID NO:28; (v) polypeptides beginning at any of amino acids 20-24 of SEQ ID NO:16 or SEQ ID NO:28 and ending at any of amino acids 118-133 of SEQ ID NO:16 or SEQ ID NO:28; (vi) polypeptides beginning at any of amino acids 21-24 of SEQ ID NO:16 or SEQ ID NO:28 and ending at any of amino acids 118-134 of SEQ ID NO:16 or SEQ ID NO:28; (vii) polypeptides beginning at any of amino acids 20-24 of SEQ ID NO:16 or SEQ ID NO:28 and ending at any of amino acids 128-133 of SEQ ID NO:16 or SEQ ID NO:28; (viii) polypeptides beginning at any of amino acids 20-24 of SEQ ID NO:16 or SEQ ID NO:28 and ending at any of amino acids 128-133 of SEQ ID NO:16 or SEQ ID NO:28; (ix) polypeptides beginning at any of amino acids 21-29 of SEQ ID NO:16 or SEQ ID NO:28 and ending at any of amino acids 118-134 of SEQ ID NO:16 or SEQ ID NO:28; (x) polypeptides beginning at any of amino acids 20-29 of SEQ ID NO:16 or SEQ ID NO:28 and ending at any of amino acids 118-133 of SEQ ID NO:16 or SEQ ID NO:28; (xi) polypeptides beginning at any of amino acids 21-29 of SEQ ID NO:16 or SEQ ID NO:28 and ending at any of amino acids 128-134 of SEQ ID NO:16 or SEQ ID NO:28; and (xii) polypeptides beginning at any of amino acids 20-29 of SEQ ID NO:16 or SEQ ID NO:28 and ending at any of amino acids 128-133 of SEQ ID NO:16 or SEQ ID NO:28. In a specific example, an ActRIIB polypeptides comprises, consists essentially of, or consists of, an amino acid sequence beginning at amino acid position 25 of SEQ ID NO:16 or SEQ ID NO:28 and ending at amino acid position 131 of SEQ ID NO:16 or SEQ ID NO:28. In another specific example, an ActRIIB polypeptide consists of, or consists essentially of, the amino acid sequence of SEQ ID NO:17, 18, 23, 26, 27, 29, 30, 31, 32, 33, 36, 37, 42, or 43.

Any of the ActRIIB polypeptides used in the compositions and methods described herein may be produced as a homodimer. Any of the ActRIIB polypeptides used in the compositions and methods described herein may be formulated as a fusion protein having a heterologous portion that comprises a constant region from an IgG heavy chain, such as an Fc domain. Any of the ActRIIB polypeptides used in the compositions and methods described herein may comprise an acidic amino acid at the position corresponding to position 79 of SEQ ID NO:16 or SEQ ID NO:28, optionally in combination with one or more additional amino acid substitutions, deletions or insertions relative to SEQ ID NO:16 or SEQ ID NO:28.

In specific embodiments, the inhibitors of ActRIIB used in the compositions and methods described herein comprise an extracellular domain of ActRIIB with one or more amino acid substitutions/mutations. Such an amino acid substitution/mutation can be, for example, an exchange from the leucine at amino acid position 79 of SEQ ID NO:16 or SEQ ID NO:28 to an acidic amino acid, such as aspartic acid or glutamic acid. For example, position L79 of SEQ ID NO:16 or SEQ ID NO:28 may be altered in ActRIIB extracellular domain polypeptides to confer altered activin-myostatin (GDF-11) binding properties. L79A and L79P mutations reduce GDF-11 binding to a greater extent than activin binding. L79E and L79D mutations retain GDF-11 binding, while demonstrating greatly reduced activin binding.

In certain embodiments, the inhibitors of ActRIIB used in the compositions and methods described herein comprise a truncated form of an ActRIIB extracellular domain that also carries an amino acid substitution, e.g., an exchange from the leucine at amino acid position 79 of SEQ ID NO:16 or SEQ ID NO:28 to an acidic amino acid, such as aspartic acid or glutamic acid. In a specific embodiment, the truncated form of an extracellular domain of ActRIIB polypeptide that also carries an amino acid substitution used in the compositions and methods described herein is SEQ ID NO:23. Forms of ActRIIB that are truncated and/or carry one or more amino acid substitutions can be linked to an Fc domain of an antibody as discussed above.

Functionally active fragments of ActRIIB polypeptides can be obtained, for example, by screening polypeptides recombinantly produced from the corresponding fragment of the nucleic acid encoding an ActRIIB polypeptide. In addition, fragments can be chemically synthesized using techniques known in the art such as conventional Merrifield solid phase f-Moc or t-Boc chemistry. The fragments can be produced (recombinantly or by chemical synthesis) and tested to identify those peptidyl fragments that can function as antagonists (inhibitors) of ActRIIB protein or signaling mediated by activin.

In addition, functionally active variants of ActRIIB polypeptides can be obtained, for example, by screening libraries of modified polypeptides recombinantly produced from the corresponding mutagenized nucleic acids encoding an ActRIIB polypeptide. The variants can be produced and tested to identify those that can function as antagonists (inhibitors) of ActRIIB protein or signaling mediated by activin. In certain aspects, a functional variant of the ActRIIB polypeptides comprises an amino acid sequence that is at least 75% identical to an amino acid sequence selected from SEQ ID NO:17, 18, 23, 26, 27, 29, 30, 31, 32, 33, 36, 37, 42, and 43. In certain aspects, the functional variant has an amino acid sequence at least 80%, 85%, 90%, 95%, 96%, 97%, 98%, or 99% identical to an amino acid sequence selected from SEQ ID NO:17, 18, 23, 26, 27, 29, 30, 31, 32, 33, 36, 37, 42, and 43.

Functional variants may be generated, for example, by modifying the structure of an ActRIIB polypeptide for such purposes as enhancing therapeutic efficacy, or stability (e.g., ex vivo shelf life and resistance to proteolytic degradation in vivo). Such modified ActRIIB polypeptides when selected to retain activin binding, are considered functional equivalents of the naturally-occurring ActRIIB polypeptides. Modified ActRIIB polypeptides can also be produced, for instance, by amino acid substitution, deletion, or addition. For instance, it is reasonable to expect that an isolated replacement of a leucine with an isoleucine or valine, an aspartate with a glutamate, a threonine with a serine, or a similar replacement of an amino acid with a structurally related amino acid (e.g., conservative mutations) will not have a major effect on the biological activity of the resulting molecule. Conservative replacements are those that take place within a family of amino acids that are related in their side chains. Whether a change in the amino acid sequence of an ActRIIB polypeptide results in a functional homolog can be readily determined by assessing the ability of the variant ActRIIB polypeptide to produce a response in cells in a fashion similar to the wild-type ActRIIB polypeptide.

ActRIIB polypeptide mutants, particularly sets of combinatorial mutants of an ActRIIB polypeptide, as well as truncation mutants; pools of combinatorial mutants are especially useful for identifying functional variant sequences can be used in the methods and compositions described herein. The purpose of screening such combinatorial libraries may be to generate, for example, ActRIIB polypeptide variants which can act as either agonists or antagonist, or alternatively, which possess novel activities all together.

It has been demonstrated that the ligand binding pocket of ActRIIB is defined by residues Y31, N33, N35, L38 through T41, E47, E50, Q53 through K55, L57, H58, Y60, S62, K74, W78 through N83, Y85, R87, A92, and E94 through F101 of SEQ ID NO:16 or SEQ ID NO:28. At these positions, it is expected that conservative mutations will be tolerated, although a K74A mutation is well-tolerated, as are R40A, K55A, F82A and mutations at position L79. R40 is a K in Xenopus, indicating that basic amino acids at this position will be tolerated. Q53 is R in bovine ActRIIB and K in Xenopus ActRIIB, and therefore amino acids including R, K, Q, N and H will be tolerated at this position. Thus, a general formula for an ActRIIB polypeptide for use in the methods and compositions described herein is one that comprises amino acids 29-109 of SEQ ID NO:16 or SEQ ID NO:28, but optionally beginning at an amino acid position ranging from 20-24 or 22-25 of SEQ ID NO:16 or SEQ ID NO:28 and ending at an amino acid position ranging from 129-134 of SEQ ID NO:16 or SEQ ID NO:28, and comprising no more than 1, 2, 5, or 15 conservative amino acid changes in the ligand binding pocket, and zero, one or more non-conservative alterations at amino acid positions 40, 53, 55, 74, 79 and/or 82 of SEQ ID NO:16 or SEQ ID NO:28 in the ligand binding pocket. Such an ActRIIB polypeptide may retain greater than 80%, 90%, 95% or 99% sequence identity or sequence homology to the sequence of amino acids 29-109 of SEQ ID NO:16 or SEQ ID NO:28. Sites outside the binding pocket, at which variability may be particularly well tolerated, include the amino and carboxy termini of the extracellular domain of ActRIIB, and positions 42-46 and 65-73. An asparagine to alanine alteration at position 65 of SEQ ID NO:16 or SEQ ID NO:28 (N65A) actually improves ligand binding in the A64 background, and is thus expected to have no detrimental effect on ligand binding in the R64 background. This change probably eliminates glycosylation at N65 in the A64 background, thus demonstrating that a significant change in this region is likely to be tolerated. While an R64A change is poorly tolerated, R64K is well-tolerated, and thus another basic residue, such as H may be tolerated at position 64.

As a specific example of an ActRIIB polypeptide with a mutation in the ligand binding domain, the positively-charged amino acid residue Asp (D80) of the ligand-binding domain of ActRIIB can be mutated to a different amino acid residue such that the variant ActRIIB polypeptide preferentially binds to GDF8, but not activin. In a specific embodiment, the D80 residue is changed to an amino acid residue selected from the group consisting of: an uncharged amino acid residue, a negative amino acid residue, and a hydrophobic amino acid residue. As a further specific example, the hydrophobic residue L79 can be altered to the acidic amino acids aspartic acid or glutamic acid to greatly reduce activin binding while retaining GDF11 binding. As will be recognized by one of skill in the art, most of the described mutations, variants or modifications may be made at the nucleic acid level or, in some cases, by post translational modification or chemical synthesis. Such techniques are well known in the art.

In specific embodiments, the inhibitors of ActRIIB used in the compositions and methods described herein comprise a conjugate/fusion protein comprising an extracellular domain (e.g., an activin-binding domain) of an ActRIIB receptor linked to an Fc portion of an antibody. Such conjugate/fusion proteins may comprise any of the ActRIIB polypeptides disclosed herein (e.g., any of SEQ ID NOs:17, 18, 23, 26, 27, 29, 30, 31, 32, 33, 36, 37, 42, or 43), any ActRIIB polypeptides known in the art, or any ActRIIB polypeptides generated using methods known in the art and/or provided herein.

In certain embodiments, the extracellular domain is linked to an Fc portion of an antibody via a linker, *e.g.,* a peptide linker. Exemplary linkers include short polypeptide sequences such as 2-10, 2-5, 2-4, 2-3 amino acid residues (e.g., glycine residues), such as, for example, a Gly-Gly-Gly linker. In a specific embodiment, the linker comprises the amino acid sequence Gly-Gly-Gly (GGG). In another specific embodiment, the linker comprises the amino acid sequence Thr-Gly-Gly-Gly (TGGG). Optionally, the Fc domain has one or more mutations at residues such as Asp-265, lysine 322, and Asn-434. In certain cases, the mutant Fc domain having one or more of these mutations (e.g., an Asp-265 mutation) has a reduced ability to bind to the Fcγ receptor relative to a wild-type Fc domain. In other cases, the mutant Fc domain having one or more of these mutations (e.g., an Asn-434 mutation) has an increased ability to bind to the MHC class I- related Fc-receptor (FcRN) relative to a wild-type Fc domain. Exemplary fusion proteins comprising a soluble extracellular domain of ActRIIB fused to an Fc domain are set forth in SEQ ID NOs:20, 21, 24, 25, 34, 35, 38, 39, 40, 41, 44, 46, and 47.

In a specific embodiment, the ActRIIB inhibitors used in the compositions and methods described herein comprise the extracellular domain of ActRIIB, or a portion thereof, linked to an Fc portion of an antibody, wherein said ActRIIB inhibitor comprises an amino acid sequence that is at least 75% identical to an amino acid sequence selected from SEQ ID NOs:20, 21, 24, 25, 34, 35, 38, 39, 40, 41, 44, 46, and 47. In another specific embodiment, the ActRIIB inhibitors used in the compositions and methods described herein comprise the extracellular domain of ActRIIB, or a portion thereof, linked to an Fc portion of an antibody, wherein said ActRIIB inhibitor comprises an amino acid sequence that is at least 80%, 85%, 90%, 95%, 96%, 97%, 98%, or 99% identical to an amino acid sequence selected from SEQ ID NOs:20, 21, 24, 25, 34, 35, 38, 39, 40, 41, 44, 46, and 47.

In a specific embodiment, the ActRIIB inhibitor to be used in the compositions and methods described herein is a fusion protein between the extracellular domain of the human ActRIIB receptor and the Fc portion of IgG1. In another specific embodiment, the ActRIIB inhibitor to be used in the compositions and methods described herein is a fusion protein between a truncated extracellular domain of the human ActRIIB receptor and the Fc portion of IgG1. In another specific embodiment, the ActRIIB inhibitor to be used in the compositions and methods described herein is a fusion protein between a truncated extracellular domain of the human ActRIIB receptor and the Fc portion of IgG1, wherein the truncated extracellular domain of the human ActRIIB receptor possesses an amino acid substitution at the amino acid position corresponding to amino acid 79 of SEQ ID NO:16 or SEQ ID NO:28. In one embodiment, the amino acid substitution at the amino acid position corresponding to amino acid 79 of SEQ ID NO:16 or SEQ ID NO:28 is substitution of Leucine for Aspartic Acid (i.e., an L79D mutation).

In a specific embodiment, the ActRIIB inhibitor to be used in the compositions and methods described herein is SEQ ID NO:24 or 25, which represents a fusion protein between the extracellular domain of the human ActRIIB receptor and the Fc portion of IgG1, wherein said ActRIIB extracellular domain comprises amino acids 25-131 of SEQ ID NO:28 with an L79D mutation. The nucleic acid sequence encoding the ActRIIB-Fc fusion protein of SEQ ID NO:24 is presented in SEQ ID NO:45.

In another specific embodiment, the ActRIIB inhibitor to be used in the compositions and methods described herein is SEQ ID NO:34 or 35, which represents a fusion protein between the extracellular domain of the human ActRIIB receptor and the Fc portion of IgG1, wherein said ActRIIB extracellular domain comprises amino acids 25-131 of SEQ ID NO:16 with an L79D mutation.

Asparagine-linked glycosylation recognition sites generally comprise a tripeptide sequence, asparagine-X-threonine (or asparagine-X-serine) (where "X" is any amino acid) which is specifically recognized by appropriate cellular glycosylation enzymes. The alteration may also be made by the addition of, or substitution by, one or more serine or threonine residues to the sequence of the wild-type ActRIIB polypeptide (for O-linked glycosylation sites). A variety of amino acid substitutions or deletions at one or both of the first or third amino acid positions of a glycosylation recognition site (and/or amino acid deletion at the second position) results in non-glycosylation at the modified tripeptide sequence. Another means of increasing the number of carbohydrate moieties on an ActRIIB polypeptide is by chemical or enzymatic coupling of glycosides to the ActRIIB polypeptide. Depending on the coupling mode used, the sugar(s) may be attached to (a) arginine and histidine; (b) free carboxyl groups; (c) free sulfhydryl groups such as those of cysteine; (d) free hydroxyl groups such as those of serine, threonine, or hydroxyproline; (e) aromatic residues such as those of phenylalanine, tyrosine, or tryptophan; or (f) the amide group of glutamine. These methods are described in International Patent Application No. WO 87/05330 published Sep. 11, 1987, and in Aplin and Wriston (1981) CRC Crit. Rev. Biochem., pp. 259-306. Removal of one or more carbohydrate moieties present on an ActRIIB polypeptide may be accomplished chemically and/or enzymatically. Chemical deglycosylation may involve, for example, exposure of the ActRIIB polypeptide to the compound trifluoromethanesulfonic acid, or an equivalent compound. This treatment results in the cleavage of most or all sugars except the linking sugar (N-acetylglucosamine or N-acetylgalactosamine), while leaving the amino acid sequence intact. Chemical deglycosylation is further described by Hakimuddin et al. (1987) Arch. Biochem. Biophys. 259:52 and by Edge et al. (1981) Anal. Biochem. 118:131. Enzymatic cleavage of carbohydrate moieties on ActRIIB polypeptides can be achieved by the use of a variety of endo- and exo-glycosidases as described by Thotakura et al. (1987) Meth. Enzymol. 138:350. The sequence of an ActRIIB polypeptide may be adjusted, as appropriate, depending on the type of expression system used, as mammalian, yeast, insect and plant cells may all introduce differing glycosylation patterns that can be affected by the amino acid sequence of the peptide. In general, ActRIIB proteins for use in humans will be expressed in a mammalian cell line that provides proper glycosylation, such as HEK293 or CHO cell lines, although other expression systems, such as other mammalian expression cell lines, yeast cell lines with engineered glycosylation enzymes and insect cells, are expected to be useful as well.

In specific embodiments, mutated ActRIIB polypeptides comprising the addition of a further N-linked glycosylation site (N-X-S/T) that increases the serum half-life of an ActRIIB-Fc fusion protein, relative to the ActRIIB(R64)-Fc form can be used in the methods and compositions described herein. In a specific embodiment, introduction of an asparagine at position 24 of SEQ ID NO:16 or SEQ ID NO:28 (A24N) results in the creation of an NXT sequence that confers a longer half-life. Other NX(T/S) sequences can be found at 42-44 (NQS) and 65-67 (NSS), although the latter may not be efficiently glycosylated with the R at position 64 (i.e., in R64 polypeptides). N-X-S/T sequences may be generally introduced at positions outside the ligand binding pocket of ActRIIB, which is detailed above. Particularly suitable sites for the introduction of non-endogenous N-X-S/T sequences include amino acids 20-29, 20-24, 22-25, 109-134, 120-134 or 129-134 of SEQ ID NO:16 or SEQ ID NO:28. N-X-S/T sequences may also be introduced into the linker between the ActRIIB sequence and the Fc or other fusion component. Such a site may be introduced with minimal effort by introducing an N in the correct position with respect to a pre-existing S or T, or by introducing an S or T at a position corresponding to a pre-existing N. Thus, desirable alterations that would create an N-linked glycosylation site are: A24N, R64N, S67N (possibly combined with an N65A alteration), E106N, R112N, G120N, E123N, P129N, A132N, R112S and R112T (with all amino acid positions corresponding to the positions they can be found in SEQ ID NO:16 or SEQ ID NO:28). Any S that is predicted to be glycosylated may be altered to a T without creating an immunogenic site, because of the protection afforded by the glycosylation. Likewise, any T that is predicted to be glycosylated may be altered to an S. Thus the alterations S67T and S44T are encompassed herein. Likewise, in an A24N variant, an S26T alteration may be used. Accordingly, an ActRIIB polypeptide may include one or more additional, non-endogenous N-linked glycosylation consensus sequences.

A variety of screening assays may be used to evaluate ActRIIB polypeptide variants. For example, an ActRIIB polypeptide variant may be screened for ability to bind to an ActRIIB ligand, to prevent binding of an ActRIIB ligand to an ActRIIB polypeptide or to interfere with signaling caused by an ActRIIB ligand. The activity of an ActRIIB polypeptide or its variants may also be tested in a cell-based or *in vivo* assay.

Combinatorially-derived variants can be generated which have a selective or generally increased potency relative to a naturally occurring ActRIIB polypeptide. Likewise, mutagenesis can give rise to variants which have intracellular half-lives dramatically different than the corresponding wild-type ActRIIB polypeptide. For example, the altered protein can be rendered either more stable or less stable to proteolytic degradation or other cellular processes which result in destruction of, or otherwise inactivation of a native ActRIIB polypeptide. Such variants, and the genes which encode them, can be utilized to alter ActRIIB polypeptide levels by modulating the half-life of the ActRIIB polypeptides. For instance, a short half-life can give rise to more transient biological effects and can allow tighter control of recombinant ActRIIB polypeptide levels within the patient. In an Fc fusion protein, mutations may be made in the linker (if any) and/or the Fc portion to alter the half-life of the protein.

A combinatorial library may be produced by way of a degenerate library of genes encoding a library of polypeptides which each include at least a portion of potential ActRIIB polypeptide sequences. For instance, a mixture of synthetic oligonucleotides can be enzymatically ligated into gene sequences such that the degenerate set of potential ActRIIB polypeptide nucleotide sequences are expressible as individual polypeptides, or alternatively, as a set of larger fusion proteins (e.g., for phage display).

There are many ways by which the library of potential homologs can be generated from a degenerate oligonucleotide sequence. Chemical synthesis of a degenerate gene sequence can be carried out in an automatic DNA synthesizer, and the synthetic genes then be ligated into an appropriate vector for expression. The synthesis of degenerate oligonucleotides is well known in the art (see for example, Narang, S A (1983) Tetrahedron 39:3; Itakura et al., (1981) Recombinant DNA, Proc. 3rd Cleveland Sympos. Macromolecules, ed. AG Walton, Amsterdam: Elsevier pp 273-289; Itakura et al., (1984) Annu. Rev. Biochem. 53:323; Itakura et al., (1984) Science 198:1056; Ike et al., (1983) Nucleic Acid Res. 11:477). Such techniques have been employed in the directed evolution of other proteins (see, for example, Scott et al., (1990) Science 249:386-390; Roberts et al., (1992) PNAS USA 89:2429-2433; Devlin et al., (1990) Science 249: 404-406; Cwirla et al., (1990) PNAS USA 87: 6378-6382; as well as U.S. Pat. Nos. 5,223,409, 5,198,346, and 5,096,815).

Alternatively, other forms of mutagenesis can be utilized to generate a combinatorial library. For example, ActRIIB polypeptide variants can be generated and isolated from a library by screening using, for example, alanine scanning mutagenesis and the like (Ruf et al., (1994) Biochemistry 33:1565-1572; Wang et al., (1994) J. Biol. Chem. 269:3095-3099; Balint et al., (1993) Gene 137:109-118; Grodberg et al., (1993) Eur. J. Biochem. 218:597-601; Nagashima et al., (1993) J. Biol. Chem. 268:2888-2892; Lowman et al., (1991) Biochemistry 30:10832-10838; and Cunningham et al., (1989) Science 244:1081-1085), by linker scanning mutagenesis (Gustin et al., (1993) Virology 193:653-660; Brown et al., (1992) Mol. Cell Biol. 12:2644-2652; McKnight et al., (1982) Science 232:316); by saturation mutagenesis (Meyers et al., (1986) Science 232:613); by PCR mutagenesis (Leung et al., (1989) Method Cell Mol Biol 1:11-19); or by random mutagenesis, including chemical mutagenesis, etc. (Miller et al., (1992) A Short Course in Bacterial Genetics, CSHL Press, Cold Spring Harbor, N.Y.; and Greener et al., (1994) Strategies in Mol Biol 7:32-34). Linker scanning mutagenesis, particularly in a combinatorial setting, is an attractive method for identifying truncated (bioactive) forms of ActRIIB polypeptides.

A wide range of techniques are known in the art for screening gene products of combinatorial libraries made by point mutations and truncations, and, for that matter, for screening cDNA libraries for gene products having a certain property. Such techniques will be generally adaptable for rapid screening of the gene libraries generated by the combinatorial mutagenesis of ActRIIB polypeptides. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Preferred assays include activin binding assays and activin-mediated cell signaling assays.

In certain embodiments, ActRIIB polypeptides used in the methods and compositions described herein may further comprise post-translational modifications in addition to any that are naturally present in the ActRIIB polypeptides. Such modifications include, but are not limited to, acetylation, carboxylation, glycosylation, phosphorylation, lipidation, and acylation. As a result, the modified ActRIIB polypeptides may contain non-amino acid elements, such as polyethylene glycols, lipids, poly- or mono-saccharide, and phosphates. Effects of such non-amino acid elements on the functionality of a ActRIIB polypeptide may be tested by any method known to the skilled artisan. When an ActRIIB polypeptide is produced in cells by cleaving a nascent form of the ActRIIB polypeptide, post-translational processing may also be important for correct folding and/or function of the protein. Different cells (such as CHO, HeLa, MDCK, 293, W138, NIH-3T3 or HEK293) have specific cellular machinery and characteristic mechanisms for such post-translational activities and may be chosen to ensure the correct modification and processing of the ActRIIB polypeptides.

In certain aspects, functional variants or modified forms of the ActRIIB polypeptides include fusion proteins having at least a portion of the ActRIIB polypeptides and one or more fusion domains. Well known examples of such fusion domains include, but are not limited to, polyhistidine, Glu-Glu, glutathione S transferase (GST), thioredoxin, protein A, protein G, an immunoglobulin heavy chain constant region (Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt-conjugated resins are used. Many of such matrices are available in "kit" form, such as the Pharmacia GST purification system and the QIAexpress™ system (Qiagen) useful with (HIS₆) fusion partners. As another example, a fusion domain may be selected so as to facilitate detection of the ActRIIB polypeptides. Examples of such detection domains include the various fluorescent proteins (e.g., GFP) as well as "epitope tags," which are usually short peptide sequences for which a specific antibody is available. Well known epitope tags for which specific monoclonal antibodies are readily available include FLAG, influenza virus hemagglutinin (HA), and c-myc tags. In some cases, the fusion domains have a protease cleavage site, such as for Factor Xa or Thrombin, which allows the relevant protease to partially digest the fusion proteins and thereby liberate the recombinant proteins therefrom. The liberated proteins can then be isolated from the fusion domain by subsequent chromatographic separation. In certain preferred embodiments, an ActRIIB polypeptide is fused with a domain that stabilizes the ActRIIB polypeptide in vivo (a "stabilizer" domain). By "stabilizing" is meant anything that increases serum half life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Likewise, fusions to human serum albumin can confer desirable properties. Other types of fusion domains that may be selected include multimerizing (e.g., dimerizing, tetramerizing) domains and functional domains (that confer an additional biological function, such as further stimulation of bone growth or muscle growth, as desired).

It is understood that different elements of the fusion proteins may be arranged in any manner that is consistent with the desired functionality. For example, an ActRIIB polypeptide may be placed C-terminal to a heterologous domain, or, alternatively, a heterologous domain may be placed C-terminal to an ActRIIB polypeptide. The ActRIIB polypeptide domain and the heterologous domain need not be adjacent in a fusion protein, and additional domains or amino acid sequences may be included C- or N-terminal to either domain or between the domains.

In certain embodiments, the ActRIIB polypeptides used in the methods and compositions described herein contain one or more modifications that are capable of stabilizing the ActRIIB polypeptides. For example, such modifications enhance the in vitro half life of the ActRIIB polypeptides, enhance circulatory half life of the ActRIIB polypeptides or reduce proteolytic degradation of the ActRIIB polypeptides. Such stabilizing modifications include, but are not limited to, fusion proteins (including, for example, fusion proteins comprising an ActRIIB polypeptide and a stabilizer domain), modifications of a glycosylation site (including, for example, addition of a glycosylation site to an ActRIIB polypeptide), and modifications of carbohydrate moiety (including, for example, removal of carbohydrate moieties from an ActRIIB polypeptide). In the case of fusion proteins, an ActRIIB polypeptide is fused to a stabilizer domain such as an IgG molecule (e.g., an Fc domain). As used herein, the term "stabilizer domain" not only refers to a fusion domain (e.g., Fc) as in the case of fusion proteins, but also includes nonproteinaceous modifications such as a carbohydrate moiety, or nonproteinaceous polymer, such as polyethylene glycol.

In certain embodiments, the methods and compositions described herein use isolated or purified ActRIIB polypeptides, i.e., ActRIIB polypeptides which are isolated from, or otherwise substantially free of, other proteins can be used with the methods and compositions described herein. ActRIIB polypeptides will generally be produced by expression from recombinant nucleic acids.

In certain aspects, the ActRIIB polypeptides used in the methods and compositions described herein are encoded by isolated and/or recombinant nucleic acids, including fragments, functional variants and fusion proteins disclosed herein. For example, SEQ ID NO: 19 encodes the naturally occurring human ActRIIB precursor polypeptide. The subject nucleic acids may be single-stranded or double stranded. Such nucleic acids may be DNA or RNA molecules. These nucleic acids may be used, for example, in methods for making ActRIIB polypeptides or as direct therapeutic agents (e.g., in a gene therapy approach).

In certain aspects, the nucleic acids that can be used to produce ActRIIB polypeptides suitable for use in the methods and compositions described herein are further understood to include nucleic acids that are variants of SEQ ID NO: 19 as well as variants of those nucleic acid sequences that encode soluble ActRIIB polypeptides (e.g., nucleic acids that encode SEQ ID NOs: 17, 18, 23, 26, 27, 29, 30, 31, 32, 33, 36, 37, 42, and 43). Variant nucleotide sequences include sequences that differ by one or more nucleotide substitutions, additions or deletions, such as allelic variants.

In certain aspects, the isolated or recombinant nucleic acid sequences that can be used to produce ActRIIB polypeptides suitable for use in the methods and compositions described herein are at least 80%, 85%, 90%, 95%, 97%, 98%, 99% or 100% identical to SEQ ID NO:19 or those nucleic acid sequences that encode soluble ActRIIB polypeptides (e.g., nucleic acids that encode SEQ ID NOs: 17, 18, 23, 26, 27, 29, 30, 31, 32, 33, 36, 37, 42, and 43). One of ordinary skill in the art will appreciate that nucleic acid sequences complementary to SEQ ID NO:19 or those nucleic acid sequences that encode soluble ActRIIB polypeptides (e.g., nucleic acids that encode SEQ ID NOs: 17, 18, 23, 26, 27, 29, 30, 31, 32, 33, 36, 37, 42, and 43), and variants of SEQ ID NO:19 or those nucleic acid sequences that encode soluble ActRIIB polypeptides (e.g., nucleic acids that encode SEQ ID NOs: 17, 18, 23, 26, 27, 29, 30, 31, 32, 33, 36, 37, 42, and 43) can be used with the methods and compositions described herein. In further embodiments, the nucleic acid sequences can be isolated, recombinant, and/or fused with a heterologous nucleotide sequence, or in a DNA library.

In other embodiments, nucleic acids that can be used to produce ActRIIB polypeptides suitable for use in the methods and compositions described herein include nucleotide sequences that hybridize under highly stringent conditions to the nucleotide sequence designated in SEQ ID NO:19 or those nucleic acid sequences that encode soluble ActRIIB polypeptides (e.g., nucleic acids that encode SEQ ID NOs: 17, 18, 23, 26, 27, 29, 30, 31, 32, 33, 36, 37, 42, and 43), complement sequence of SEQ ID NO:19 or those nucleic acid sequences that encode soluble ActRIIB polypeptides (e.g., nucleic acids that encode SEQ ID NOs: 17, 18, 23, 26, 27, 29, 30, 31, 32, 33, 36, 37, 42, and 43), or fragments thereof. One of ordinary skill in the art will understand that appropriate stringency conditions which promote DNA hybridization can be varied. For example, one can perform the hybridization at 6.0 times sodium chloride/sodium citrate (SSC) at about 45 degree Celsius, followed by a wash of 2.0 times SSC at 50 degree Celsius. For example, the salt concentration in the wash step can be selected from a low stringency of about 2.0 times SSC at 50 degree Celsius to a high stringency of about 0.2 times SSC at 50 degree Celsius. In addition, the temperature in the wash step can be increased from low stringency conditions at room temperature, about 22 degree Celsius, to high stringency conditions at about 65 degree Celsius. Both temperature and salt may be varied, or temperature or salt concentration may be held constant while the other variable is changed. In one embodiment, nucleic acids which hybridize under low stringency conditions of 6 times SSC at room temperature followed by a wash at 2 times SSC at room temperature can be used with the methods and compositions described herein.

Isolated nucleic acids which differ from the nucleic acids as set forth in SEQ ID NO:19 or those nucleic acid sequences that encode soluble ActRIIB polypeptides (e.g., nucleic acids that encode SEQ ID NOs: 17, 18, 23, 26, 27, 29, 30, 31, 32, 33, 36, 37, 42, and 43) due to degeneracy in the genetic code can also be used to produce ActRIIB polypeptides suitable for use in the methods and compositions described herein. For example, a number of amino acids are designated by more than one triplet. Codons that specify the same amino acid, or synonyms (for example, CAU and CAC are synonyms for histidine) may result in "silent" mutations which do not affect the amino acid sequence of the protein. However, it is expected that DNA sequence polymorphisms that do lead to changes in the amino acid sequences of the subject proteins will exist among mammalian cells. One skilled in the art will appreciate that these variations in one or more nucleotides (up to about 3-5% of the nucleotides) of the nucleic acids encoding a particular protein may exist among individuals of a given species due to natural allelic variation. Any and all such nucleotide variations and resulting amino acid polymorphisms can be used with the methods and compositions described herein.

In certain embodiments, the recombinant nucleic acids that can be used to produce ActRIIB polypeptides suitable for use in the methods and compositions described herein may be operably linked to one or more regulatory nucleotide sequences in an expression construct. Regulatory nucleotide sequences will generally be appropriate to the host cell used for expression. Numerous types of appropriate expression vectors and suitable regulatory sequences are known in the art for a variety of host cells. Typically, said one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art can be used with the methods and compositions described herein. The promoters may be either naturally occurring promoters, or hybrid promoters that combine elements of more than one promoter. An expression construct may be present in a cell on an episome, such as a plasmid, or the expression construct may be inserted in a chromosome. In a preferred embodiment, the expression vector contains a selectable marker gene to allow the selection of transformed host cells. Selectable marker genes are well known in the art and will vary with the host cell used.

In certain aspects, the nucleic acids that can be used to produce ActRIIB polypeptides suitable for use in the methods and compositions described herein are provided in an expression vector comprising a nucleotide sequence encoding an ActRIIB polypeptide and operably linked to at least one regulatory sequence. Regulatory sequences are art-recognized and are selected to direct expression of the ActRIIB polypeptide. Accordingly, the term regulatory sequence includes promoters, enhancers, and other expression control elements. Exemplary regulatory sequences are described in Goeddel; Gene Expression Technology: Methods in Enzymology, Academic Press, San Diego, Calif. (1990). For instance, any of a wide variety of expression control sequences that control the expression of a DNA sequence when operatively linked to it may be used in these vectors to express DNA sequences encoding an ActRIIB polypeptide. Such useful expression control sequences, include, for example, the early and late promoters of SV40, tet promoter, adenovirus or cytomegalovirus immediate early promoter, RSV promoters, the lac system, the trp system, the TAC or TRC system, T7 promoter whose expression is directed by T7 RNA polymerase, the major operator and promoter regions of phage lambda, the control regions for fd coat protein, the promoter for 3-phosphoglycerate kinase or other glycolytic enzymes, the promoters of acid phosphatase, e.g., Pho5, the promoters of the yeast .alpha.-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other protein encoded by the vector, such as antibiotic markers, should also be considered.

A recombinant nucleic acid can be produced by ligating the cloned gene, or a portion thereof, into a vector suitable for expression in either prokaryotic cells, eukaryotic cells (yeast, avian, insect or mammalian), or both. Expression vehicles for production of a recombinant ActRIIB polypeptide include plasmids and other vectors. For instance, suitable vectors include plasmids of the types: pBR322-derived plasmids, pEMBL-derived plasmids, pEX-derived plasmids, pBTac-derived plasmids and pUC-derived plasmids for expression in prokaryotic cells, such as E. coli.

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures, see Molecular Cloning A Laboratory Manual, 3rd Ed., ed. by Sambrook, Fritsch and Maniatis (Cold Spring Harbor Laboratory Press, 2001). In some instances, it may be desirable to express the recombinant polypeptides by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the .beta.-gal containing pBlueBac III).

In one embodiment, a vector can be designed for production of the ActRIIB polypeptides used in the methods and compositions described herein in CHO cells, such as a Pcmv-Script vector (Stratagene, La Jolla, Calif.), pcDNA4 vectors (Invitrogen, Carlsbad, Calif.) and pCI-neo vectors (Promega, Madison, Wis.). As will be apparent, the subject gene constructs can be used to cause expression of the subject ActRIIB polypeptides in cells propagated in culture, e.g., to produce proteins, including fusion proteins or variant proteins, for purification.

Host cells transfected with a recombinant gene including a coding sequence (e.g., SEQ ID NO:19 or those nucleic acid sequences that encode soluble ActRIIB polypeptides (e.g., nucleic acids that encode SEQ ID NOs: 17, 18, 23, 26, 27, 29, 30, 31, 32, 33, 36, 37, 42, and 43)) for one or more of the subject ActRIIB polypeptides can be used to produce ActRIIB polypeptides suitable for use in the methods and compositions described herein. The host cell may be any prokaryotic or eukaryotic cell. For example, an ActRIIB polypeptide may be expressed in bacterial cells such as E. coli, insect cells (e.g., using a baculovirus expression system), yeast, or mammalian cells. Other suitable host cells are known to those skilled in the art.

Accordingly, provided herein are methods of producing the ActRIIB polypeptides used in the methods and compositions described herein. For example, a host cell transfected with an expression vector encoding an ActRIIB polypeptide can be cultured under appropriate conditions to allow expression of the ActRIIB polypeptide to occur. The ActRIIB polypeptide may be secreted and isolated from a mixture of cells and medium containing the ActRIIB polypeptide. Alternatively, the ActRIIB polypeptide may be retained cytoplasmically or in a membrane fraction and the cells harvested, lysed and the protein isolated. A cell culture includes host cells, media and other byproducts. Suitable media for cell culture are well known in the art. The subject ActRIIB polypeptides can be isolated from cell culture medium, host cells, or both, using techniques known in the art for purifying proteins, including ion-exchange chromatography, gel filtration chromatography, ultrafiltration, electrophoresis, immunoaffinity purification with antibodies specific for particular epitopes of the ActRIIB polypeptides and affinity purification with an agent that binds to a domain fused to the ActRIIB polypeptide (e.g., a protein A column may be used to purify an ActRIIB-Fc fusion). In a preferred embodiment, the ActRIIB polypeptide is a fusion protein containing a domain which facilitates its purification. In a preferred embodiment, purification is achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. As demonstrated herein, ActRIIB -hFc protein was purified to a purity of >98% as determined by size exclusion chromatography and >95% as determined by SDS PAGE. This level of purity was sufficient to achieve desirable effects on bone in mice and an acceptable safety profile in mice, rats and non-human primates.

In another embodiment, a fusion gene coding for a purification leader sequence, such as a poly-(His)/enterokinase cleavage site sequence at the N-terminus of the desired portion of the recombinant ActRIIB polypeptide, can allow purification of the expressed fusion protein by affinity chromatography using a Ni²⁺ metal resin. The purification leader sequence can then be subsequently removed by treatment with enterokinase to provide the purified ActRIIB polypeptide (e.g., see Hochuli et al., (1987) J. Chromatography 411:177; and Janknecht et al., PNAS USA 88:8972).

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence (see, for example, Current Protocols in Molecular Biology, eds. Ausubel et al., John Wiley & Sons: 1992).

ActRIIB -Fc fusion protein can be expressed in stably transfected CHO-DUKX Bl 1 cells from a pAID4 vector (SV40 ori/enhancer, CMV promoter), using a tissue plasminogen leader sequence of SEQ ID NO:8. The Fc portion can comprise a human IgGl Fc sequence, as shown in SEQ ID NO:7. In certain embodiments, upon expression, the protein contained has, on average, between about 1.5 and 2.5 moles of sialic acid per molecule of ActRIIB-Fc fusion protein.

In certain embodiments, the long serum half-life of an ActRIIB-Fc fusion can be 25-32 days in human patients. Additionally, the CHO cell expressed material can have a higher affinity for activin B ligand than that reported for an ActRIIB-hFc fusion protein expressed in human 293 cells (del Re et al., J Biol Chem. 2004 Dec 17;279(51):53126-35). Additionally, without being bound by theory, the use of the TPA leader sequence provided greater production than other leader sequences and, unlike ActRIIB-Fc expressed with a native leader, may provide a highly pure N-terminal sequence. Use of the native leader sequence may result in two major species of ActRIIB-Fc, each having a different N-terminal sequence.

### 5.5.3 Other ActRII Receptor Inhibitors

In certain aspects, the inhibitors of ActRII receptors used in the compositions and methods described herein are nucleic acid compounds.

Examples of categories of nucleic acid compounds that inhibit ActRII receptors include antisense nucleic acids, siRNA or RNAi constructs and catalytic nucleic acid constructs. A nucleic acid compound may be single- or double-stranded. A double-stranded compound may also include regions of overhang or non-complementarity, where one or the other of the strands is single-stranded. A single-stranded compound may include regions of self-complementarity, meaning that the compound may form a so-called "hairpin" or "stem-loop" structure, with a region of double helical structure.

The nucleic acid compounds that inhibit ActRII receptors may comprise a nucleotide sequence that is complementary to a region consisting of no more than 1000, no more than 500, no more than 250, no more than 100 or no more than 50, 35, 30, 25, 22, 20 or 18 nucleotides of the full-length ActRII receptor nucleic acid sequence or activin nucleic acid sequence (e.g., the nucleic acid sequence of an activin A or activin B subunit, also referred to as β_{A} or β_{B}). In specific aspects, the region of complementarity will be at least 8 nucleotides, and optionally at least 10 or at least 15 nucleotides, and optionally between 15 and 25 nucleotides. A region of complementarity may fall within an intron, a coding sequence or a noncoding sequence of the target transcript, such as the coding sequence portion. Generally, a nucleic acid compound that inhibits an ActRII receptor will have a length of about 8 to about 500 nucleotides or base pairs in length, and optionally the length will be about 14 to about 50 nucleotides. A nucleic acid compound that inhibits an ActRII receptor may be a DNA (particularly for use as an antisense), an RNA, or an RNA:DNA hybrid. Any one strand may include a mixture of DNA and RNA, as well as modified forms that cannot readily be classified as either DNA or RNA. Likewise, a double stranded nucleic acid compound may be DNA:DNA, DNA:RNA, or RNA:RNA, and any one strand may also include a mixture of DNA and RNA, as well as modified forms that cannot readily be classified as either DNA or RNA.

The nucleic acid compounds that inhibit an ActRII receptor may include any of a variety of modifications, including one or modifications to the backbone (the sugar-phosphate portion in a natural nucleic acid, including internucleotide linkages) or the base portion (the purine or pyrimidine portion of a natural nucleic acid). In certain aspects, an antisense nucleic acid compound will have a length of about 15 to about 30 nucleotides and will often contain one or more modifications to improve certain characteristics, such as stability in the serum, stability in a cell, or stability in a place where the compound is likely to be delivered, such as, e.g., the stomach in the case of orally delivered compounds and the lung for inhaled compounds. In the case of an RNAi construct, the strand complementary to the target transcript will generally be RNA or modifications thereof. The other strand may be RNA, DNA, or any other variation. The duplex portion of double stranded or single stranded "hairpin" RNAi construct may, in certain aspects, have a length of 18 to 40 nucleotides in length and optionally about 21 to 23 nucleotides in length, so long as it serves as a Dicer substrate. Catalytic or enzymatic nucleic acids may be ribozymes or DNA enzymes and may also contain modified forms. In certain aspects, nucleic acid compounds that inhibit ActRII receptors may inhibit expression of their target by about 50%, 60%, 70%, 75%, 80%, 85%, 90%, 95%, 99%, or more under physiological conditions and at a concentration where a nonsense or sense control has little or no effect. Concentrations for testing the effect of nucleic acid compounds include 1,5, 10 micromolar, or more.

In other aspects, the inhibitors of ActRII receptors used in the compositions and methods described herein are antibodies. Such antibodies include antibodies that bind to activin (particularly the activin A or B subunits, also referred to as β_{A} or β_{B}) and disrupt ActRII receptor binding; and antibodies that bind to ActRII receptor polypeptides (e.g., a soluble ActRIIA or soluble ActRIIB polypeptide) and disrupt activin binding.

By using immunogens derived from an ActRII receptor polypeptide or an activin polypeptide, anti-protein/anti-peptide antisera or monoclonal antibodies can be made by standard protocols (see, for example, Antibodies: A Laboratory Manual ed. by Harlow and Lane (Cold Spring Harbor Press: 1988)). A mammal, such as a mouse, a hamster or rabbit can be immunized with an immunogenic form of the ActRII receptor polypeptide, an antigenic fragment which is capable of eliciting an antibody response, or a fusion protein. Techniques for conferring immunogenicity on a protein or peptide include conjugation to carriers or other techniques well known in the art. An immunogenic portion of an ActRII receptor or activin polypeptide can be administered in the presence of adjuvant. The progress of immunization can be monitored by detection of antibody titers in plasma or serum. Standard ELISA or other immunoassays can be used with the immunogen as antigen to assess the levels of antibodies.

Following immunization of an animal with an antigenic preparation of an ActRII receptor polypeptide, antisera can be obtained and, if desired, polyclonal antibodies can be isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique (Kozbar et al., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with an ActRII receptor polypeptide and monoclonal antibodies isolated from a culture comprising such hybridoma cells.

The term "antibody" as used herein is intended to include fragments thereof which are also specifically reactive with a subject polypeptide. Antibodies can be fragmented using conventional techniques and the fragments screened for utility in the same manner as described above for whole antibodies. For example, F(ab)₂ fragments can be generated by treating antibody with pepsin. The resulting F(ab)₂ fragment can be treated to reduce disulfide bridges to produce Fab fragments. An antibody is further intended to include bispecific, single-chain, chimeric, humanized and fully human molecules having affinity for an ActRII receptor or activin polypeptide conferred by at least one CDR region of the antibody. An antibody may further comprise a label attached thereto and able to be detected (e.g., the label can be a radioisotope, fluorescent compound, enzyme or enzyme co-factor).

In certain aspects, the antibody is a recombinant antibody, which term encompasses any antibody generated in part by techniques of molecular biology, including CDR-grafted or chimeric antibodies, human or other antibodies assembled from library-selected antibody domains, single chain antibodies and single domain antibodies (e.g., human V_{H} proteins or camelid V_{HH} proteins). In certain aspects, an antibody can be a monoclonal antibody. For example, a method for generating a monoclonal antibody that binds specifically to an ActRII receptor polypeptide or activin polypeptide may comprise administering to a mouse an amount of an immunogenic composition comprising the antigen polypeptide effective to stimulate a detectable immune response, obtaining antibody-producing cells (e.g., cells from the spleen) from the mouse and fusing the antibody-producing cells with myeloma cells to obtain antibody-producing hybridomas, and testing the antibody-producing hybridomas to identify a hybridoma that produces a monoclonal antibody that binds specifically to the antigen. Once obtained, a hybridoma can be propagated in a cell culture, optionally in culture conditions where the hybridoma-derived cells produce the monoclonal antibody that binds specifically to the antigen. The monoclonal antibody may be purified from the cell culture.

The adjective "specifically reactive with" as used in reference to an antibody is intended to mean, as is generally understood in the art, that the antibody is sufficiently selective between the antigen of interest (e.g., an ActRII receptor polypeptide) and other antigens that are not of interest that the antibody is useful for, at minimum, detecting the presence of the antigen of interest in a particular type of biological sample. In certain methods employing the antibody, such as therapeutic applications, a higher degree of specificity in binding may be desirable. Monoclonal antibodies generally have a greater tendency (as compared to polyclonal antibodies) to discriminate effectively between the desired antigens and cross-reacting polypeptides. One characteristic that influences the specificity of an antibody:antigen interaction is the affinity of the antibody for the antigen. Although the desired specificity may be reached with a range of different affinities, generally preferred antibodies will have an affinity (a dissociation constant) of about 10⁻⁶, 10⁻⁷, 10⁻⁸, 10⁻⁹ or less. Given the extraordinarily tight binding between activin and an ActRII receptor, it is expected that a neutralizing anti-activin or anti-ActRII receptor antibody would generally have a dissociation constant of 10⁻¹⁰ or less.

In addition, the techniques used to screen antibodies in order to identify a desirable antibody may influence the properties of the antibody obtained. For example, if an antibody is to be used for binding an antigen in solution, it may be desirable to test solution binding. A variety of different techniques are available for testing interaction between antibodies and antigens to identify particularly desirable antibodies. Such techniques include ELISAs, surface plasmon resonance binding assays (e.g., the Biacore.TM. binding assay, Biacore AB, Uppsala, Sweden), sandwich assays (e.g., the paramagnetic bead system of IGEN International, Inc., Gaithersburg, Md.), Western blots, immunoprecipitation assays, and immunohistochemistry.

In certain aspects, ActRII receptor inhibitors to be used in the compositions and methods described herein include alternative forms of activin, particularly those with alterations in the type I receptor binding domain can bind to type II receptors and fail to form an active ternary complex. In certain aspects, nucleic acids, such as antisense molecules, siRNAs or ribozymes that inhibit activin A, B, C or E, or, particularly, ActRII receptor expression, can be used in the compositions and methods described herein. In certain embodiments, the ActRII receptor inhibitors to be used in the compositions and methods described herein exhibit selectivity for inhibiting GDF11-mediated signaling versus other members of the TGF-beta family, particularly with respect to GDF8 and activin.

In other aspects, the inhibitors of ActRII receptors used in the compositions and methods described herein are non-antibody proteins with ActRII receptor antagonist activity, including inhibin (i.e., inhibin alpha subunit), follistatin (e.g., follistatin-288 and follistatin-315), Cerberus, follistatin related protein ("FSRP"), endoglin, activin C, alpha(2)-macroglobulin, and an M108A (methionine to alanine change at position 108) mutant activin A.

In a specific aspects, the ActRII receptor inhibitor to be used in the compositions and methods described herein is a follistatin polypeptide that antagonizes activin bioactivity and/or binds to activin. The term "follistatin polypeptide" includes polypeptides comprising any naturally occurring polypeptide of follistatin as well as any variants thereof (including mutants, fragments, fusions, and peptidomimetic forms) that retain a useful activity, and further includes any functional monomer or multimer of follistatin. Variants of follistatin polypeptides that retain activin binding properties can be identified based on previous studies involving follistatin and activin interactions. For example, WO2008/030367, which is included by reference herein in its entirety, discloses specific follistatin domains ("FSDs") that are shown to be important for activin binding. Follistatin polypeptides include polypeptides derived from the sequence of any known follistatin having a sequence at least about 80% identical to the sequence of a follistatin polypeptide, and optionally at least 85%, 90%, 95%, 96%, 97%, 98%, 99% or greater identity. Examples of follistatin polypeptides include the mature follistatin polypeptide or shorter isoforms or other variants of the human follistatin precursor polypeptide as described, for example, in WO2005/025601, which is included by reference herein in its entirety.

In a specific aspect, the ActRII receptor inhibitor to be used in the compositions and methods described herein is a follistatin-like related gene (FLRG) that antagonizes activin bioactivity and/or binds to activin. The term "FLRG polypeptide" includes polypeptides comprising any naturally occurring polypeptide of FLRG as well as any variants thereof (including mutants, fragments, fusions, and peptidomimetic forms) that retain a useful activity. Variants of FLRG polypeptides that retain activin binding properties can be identified using routine methods to assay FLRG and activin interactions. See, for example, U.S. Pat. No. 6,537,966, which is included by reference herein in its entirety. FLRG polypeptides include polypeptides derived from the sequence of any known FLRG having a sequence at least about 80% identical to the sequence of an FLRG polypeptide, and optionally at least 85%, 90%, 95%, 96%, 97%, 98%, 99% or greater identity.

In certain aspect, functional variants or modified forms of the follistatin polypeptides and FLRG polypeptides include fusion proteins having at least a portion of the follistatin polypeptides or FLRG polypeptides and one or more fusion domains, such as, for example, domains that facilitate isolation, detection, stabilization or multimerization of the polypeptide. Suitable fusion domains are discussed in detail above with reference to the ActRIIA and ActRIIB polypeptides. In one aspect, an ActRII receptor inhibitor is a fusion protein comprising an activin binding portion of a follistaton polypeptide fused to an Fc domain. In another aspect, an ActRII receptor inhibitor is a fusion protein comprising an activin binding portion of an FLRG polypeptide fused to an Fc domain.

### 5.6 ASSAYS

Various ActRII polypeptide variants, or soluble ActRII polypeptide variants, may be tested for their ability to inhibit ActRII. In addition, compounds can be tested for their ability to inhibit ActRII. Once inhibitors of ActRII activity are confirmed, these compounds can be used with the methods provided herein. ActRII can be ActRIIa or ActRIIb. The assays below are described for ActRIIa but can be performed analogously for ActRIIb.

### 5.6.1 RED BLOOD CELL LEVELS

RBC count is a count of the actual number of red blood cells per volume of blood and may be included as part of a standard complete blood count. Normally, males have a RBC count of between 4.7 to 6.1 million cells per microliter and females have a RBC count of between 4.2 to 5.4 million cells per microliter. However, thalassemia patients may have a RBC count lower than that normally seen. Thus, determination of the RBC count in an anemia (e.g., thalassemia) patient being treated in accordance with the methods provided herein allows for the determination of the efficacy of such treatment.

### 5.6.2 ERYTHROID COLONY-FORMING UNITS (CFU-E)

CFU-e can be assayed and identified, e.g., in a colony formation assay by the number and morphology of the cells and by the presence or absence of certain cell surface markers. Levels of erythroid colony-forming units can be measured using, e.g., antibody staining followed by flow cytometry analysis (FACs) to evaluate expression of markers, such as differentiation state markers, e.g., Epo receptor, c-Kit (Stem cell factor receptor), transferring receptor (CD71+), CD36 and Ter119 (glycophorin-A associated antigen) (CFU-e cells are Ter119 (glycophorin-A associated antigen)-negative (see, e.g., Terszowsky et al., 2005). Cells at the CFU-e stage express erythropoietin receptor (EpoR) and can be induced to terminally differentiate in vitro in 2-3 days in the presence of only erythropoietin in a culture media. CFU-e cells can be plated on methylcellulose and stained with diaminobenzidine reagent for hemoglobin, and then CFU-e colonies can be counted. By day 2 from the time of plating, each CFU-e colony can yield between 8 and 64 hemoglobinized cells most of which are in their end-stage of erythroid differentiation.

Colony-forming unit assays are known in the art (e.g., MesenCultTM medium, Stem Cell Technologies Inc., Vancouver British Columbia; see also Wu et al. (Wu H, Liu X, Jaenisch R, Lodish HF (1995). "Generation of committed erythroid BFU-E and CFU-E progenitors does not require erythropoietin or the erythropoietin receptor". Cell 83 (1): 59-67; Marley SB, Lewis JL, Goldman JM, Gordon MY (1996)).

### 5.6.3 ERYTHROID BURST-FORMING UNITS (BFU-E)

Similarly to CFU-e, BFU-e can be assayed and identified, e.g., in a colony formation assay by the number and morphology of the cells and by the presence or absence of certain cell surface markers. Specifically, BFU-e can be identified by the expression of several cell surface markers such as CD33, CD34 and HLA-DR, and lack of expression of glycophorin-A. For example, BFU-e assays described in Wu et al. can be utilized (Wu H, Liu X, Jaenisch R, Lodish HF (1995). "Generation of committed erythroid BFU-E and CFU-E progenitors does not require erythropoietin or the erythropoietin receptor". Cell 83 (1): 59-67).

### 5.6.4 HEMATOCRIT

A hematocrit measures the percentage of red blood cells in a given volume of whole blood and may be included as part of a standard complete blood count. The hematocrit is normally about 45% for men and about 40% for women. However, thalassemia patients typically have a hematocrit lower than that normally seen. Thus, determination of the hematocrit in a thalassemia patient being treated in accordance with the methods provided herein allows for the determination of the efficacy of such treatment.

### 5.6.5 URINARY N-TELOPEPTIDE (uNTX)

Urinary N-telopeptide of type 1 collagen (NTx) can be measured in using, *e.g.,* an automated immunoassay (Vitros ECi; Ortho Clinical., Rochester, NT, USA).

### 5.6.6 SERUM BONE SPECIFIC ALKALINE PHOSPHATASE (BSAP)

Serum bone specific alkaline phosphatase (BSAP) levels can be measured using, *e.g.,* an enzyme immunoassay.

### 5.6.7 APOPTOSIS OF ERYTHROID PROGENITORS

Apoptosis of Erythroid progenitors can be determined, *e.g.,* by using Terminal deoxynucleotidyltransferase-mediated dUTP nick end-labeling (TUNEL) staining. TUNEL staining can be performed using an *in situ* Apoptosis Detection Kit (Takara Bio, Otsu, Japan).

### 5.6.8 ERYTHROID CO-CULTURE SYSTEM

To test the effect of an agent on erythroid differentiation in an in vitro environment more analogous to the in vivo environment, a co-culture system of bone marrow cells and human CD36+ cells can be used. Human CD36+ cells, which are highly enriched for erythroid progenitor cells, are co-cultured with long-term bone marrow cultures in erythropoietin-(EPO) supplemented media (2U/mL). After 6 days, the cellular output (e.g., cell type) of the culture can be assessed by, for example, flow cytometry (e.g., FACS) analysis. The numbers of erythroid cells at the various levels of erythroid differentiation (e.g., proerythroblast, basophil, late basophilic/polychromatic, orthochromatic/reticulocytes, glycoprotein A+ cells) indicate the ability of the agent being tested to modulate erythroid differentiation.

### 5.6.9 TRANSCRIPTIONAL RESPONSE ASSAY

In certain embodiments, a transcription response assay can be used to test an activin type II receptor inhibitor or activity of GDF11. Upon ActRII and GDF11 signaling, transcription of certain genes is up- or downregulated. A cell culture system used and the transcriptional response can be measured (e.g., by RT-PCT). The effect of an agent on the transcriptional response is a measure of its effectiveness or activity. In certain embodiments, the promoter region that is known to be responsive to ActRII or GDF11 signaling can be cloned upstream of a reporter gene. In this way, the assay can be simplified such that only the activity of the reporter gene need to be assayed.

### 5.7 DOSE OF ACTIVIN RECEPTOR TYPE II INHIBITOR

An ActRII inhibitor in the context of the invention is specified at the beginning of Section 3 Summary.

In certain embodiments, a therapeutically effective amount of an ActRII inhibitor is sufficient to ameliorate one symptom of anemia. In certain embodiments, a therapeutically effective amount of an ActRII inhibitor is sufficient to prevent at least one symptom of anemia from worsening. In certain embodiments, a therapeutically effective amount of an ActRII inhibitor increases the red blood cell level, hemoglobin levels, hematocrit levels, and/or Ery-C in the patient.

In certain embodiments, the ActRII inhibitor is dosed at intervals and amounts sufficient to achieve serum concentrations of 0.2 microgram/kg or greater, and serum levels of 1 microgram/kg or 2 microgram/kg or greater are desirable for achieving significant effects on bone density and strength. Dosing regimens may be designed to reach serum concentrations of between 0.2 and 15 microgram/kg, and optionally between 1 and 5 microgram/kg. In humans, serum levels of 0.2 microgram/kg may be achieved with a single dose of 0.1 mg/kg or greater and serum levels of 1 microgram/kg may be achieved with a single dose of 0.3 mg/kg or greater. The observed serum half-life of the molecule is between about 20 and 30 days, substantially longer than most Fc fusion proteins, and thus a sustained effective serum level may be achieved, for example, by dosing with 0.2-0.4 mg/kg on a weekly or biweekly basis, or higher doses may be used with longer intervals between dosings. For example, doses of 1-3 mg/kg might be used on a monthly or bimonthly basis, and the effect on bone may be sufficiently durable that dosing is necessary only once every 3, 4, 5, 6, 9, 12 or more months. Serum levels of the ActRII inhibitor can be measured by any means known to the skilled artisan. For example, antibodies against the ActRII inhibitor can be used to determine the serum levels of the ActRII inhibitor using, e.g., an ELISA.

In certain embodiments, the dose of the ActRII inhibitor ranges from 0.01 to 3.0 mg/kg intravenously or from 0.03 to 0.1 mg/kg subcutaneously. In certain embodiments, the dose of ActRII inhibitor is about 0.01 mg/kg, about 0.1 mg/kg, about 0.2 mg/kg, about 0.3 mg/kg, about 0.4 mg/kg, about 0.5 mg/kg, about 1.0 mg/kg, about 1.5 mg/kg, about 2.0 mg/kg, about 2.5 mg/kg, about 3.0 mg/kg, about 3.5 mg/kg, about 4.0 mg/kg, about 4.5 mg/kg, or about 5.0 mg/kg. In certain embodiments, the dose of ActRII inhibitor is about 10.0 mg/kg, about 15.0 mg/kg, about 20.0 mg/kg, about 25.0 mg/kg, or about 30.0 mg/kg. In certain embodiments, the dose of ActRII inhibitor is between 0.01 mg/kg and 0.1 mg/kg, between 0.1 mg/kg and 0.3 mg/kg, between 0.3 mg/kg and 0.5 mg/kg, between 0.5 mg/kg and 1.0 mg/kg, between 1.0 mg/kg and 2.0 mg/kg, between 1.0 mg/kg and 3.0 mg/kg, between 2.0 mg/kg and 3.0 mg/kg, between 2.0 mg/kg and 4.0 mg/kg, between 3.0 mg/kg and 5.0 mg/kg, between 5.0 mg/kg and 10.0 mg/kg, between 10.0 mg/kg and 15.0 mg/kg, between 10.0 mg/kg and 20.0 mg/kg, between 15.0 mg/kg and 20.0 mg/kg, or between 20.0 mg/kg and 30.0 mg/kg. When used in conjunction with a dose provided herein (e.g., a dose of an ActRII inhibitor or a dose of a second active agent), the word "about" refers to any number within 1, 5 or 10% of the referenced number.

### 5.8 PHARMACEUTICAL COMPOSITIONS

In certain embodiments, activin-ActRII antagonists (e.g., ActRII polypeptides) are formulated with a pharmaceutically acceptable carrier for use with the methods described herein. For example, an ActRII polypeptide can be administered alone or as a component of a pharmaceutical formulation (therapeutic composition). The subject compounds may be formulated for administration in any convenient way for use in human or veterinary medicine. ActRII can be ActRIIa or ActRIIb.

In certain embodiments, the therapeutic methods provided herein include administering the composition (comprising an ActRII inhibitor) systemically, or locally as an implant or device. When administered, the therapeutic composition for uses provided herein is in a pyrogen-free, physiologically acceptable form. Therapeutically useful agents other than the ActRII antagonists which may also optionally be included in the composition as described above, may be administered simultaneously or sequentially with the subject compounds (*e.g.*, ActRII polypeptides, such as ActRIIa and / or ActRIIb polypeptides (see Section 5.2)).

Typically, ActRII antagonists will be administered parenterally. Pharmaceutical compositions suitable for parenteral administration may comprise one or more ActRII polypeptides in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions for use in the methods described herein include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

Further, the composition may be encapsulated or injected in a form for delivery to a target tissue site (e.g., bone). In certain embodiments, compositions for use in the methods described herein may include a matrix capable of delivering one or more therapeutic compounds (e.g., ActRIIa polypeptides) to a target tissue site (e.g., bone), providing a structure for the developing tissue and optimally capable of being resorbed into the body. For example, the matrix may provide slow release of the ActRIIa polypeptides. Such matrices may be formed of materials presently in use for other implanted medical applications.

The choice of matrix material is based on biocompatibility, biodegradability, mechanical properties, cosmetic appearance and interface properties. The particular application of the subject compositions will define the appropriate formulation. Potential matrices for the compositions may be biodegradable and chemically defined calcium sulfate, tricalciumphosphate, hydroxyapatite, polylactic acid and polyanhydrides. Other potential materials are biodegradable and biologically well defined, such as bone or dermal collagen. Further matrices are comprised of pure proteins or extracellular matrix components. Other potential matrices are non-biodegradable and chemically defined, such as sintered hydroxyapatite, bioglass, aluminates, or other ceramics. Matrices may be comprised of combinations of any of the above mentioned types of material, such as polylactic acid and hydroxyapatite or collagen and tricalciumphosphate. The bioceramics may be altered in composition, such as in calcium-aluminate-phosphate and processing to alter pore size, particle size, particle shape, and biodegradability.

In certain embodiments, the compositions for use in the methods described herein (comprising ActRII inhibitor) can be administered orally, e.g., in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of an agent as an active ingredient. An agent may also be administered as a bolus, electuary or paste.

In solid dosage forms for oral administration (capsules, tablets, pills, dragees, powders, granules, and the like), one or more therapeutic compounds described herein may be mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose, and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such as talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

Liquid dosage forms for oral administration include pharmaceutically acceptable emulsions, microemulsions, solutions, suspensions, syrups, and elixirs. In addition to the active ingredient, the liquid dosage forms may contain inert diluents commonly used in the art, such as water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor, and sesame oils), glycerol, tetrahydrofuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, coloring, perfuming, and preservative agents.

Suspensions, in addition to the active compounds, may contain suspending agents such as ethoxylated isostearyl alcohols, polyoxyethylene sorbitol, and sorbitan esters, microcrystalline cellulose, aluminum metahydroxide, bentonite, agar-agar and tragacanth, and mixtures thereof.

The compositions described herein may also contain adjuvants, such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption, such as aluminum monostearate and gelatin.

It is understood that the dosage regimen will be determined by the attending physician considering various factors which modify the action of the compounds described herein (e.g., ActRII polypeptides, such as ActRIIa and / or ActRIIb polypeptides (see Section 5.2)). The various factors include, but are not limited to, amount of bone weight desired to be formed, the degree of bone density loss, the site of bone damage, the condition of the damaged bone, the patient's age, sex, and diet, the severity of any disease that may be contributing to bone loss, time of administration, and other clinical factors. Optionally, the dosage may vary with the type of matrix used in the reconstitution and the types of compounds in the composition. The addition of other known growth factors to the final composition, may also affect the dosage. Progress can be monitored by periodic assessment of bone growth and/or repair, for example, X-rays (including DEXA), histomorphometric determinations, and tetracycline labeling.

In certain embodiments, provided herein is gene therapy for the in vivo production of ActRII polypeptides. Such therapy would achieve its therapeutic effect by introduction of the ActRII polynucleotide sequences into cells or tissues having the disorders as listed above. Delivery of ActRII polynucleotide sequences can be achieved using a recombinant expression vector such as a chimeric virus or a colloidal dispersion system. Preferred for therapeutic delivery of ActRII polynucleotide sequences is the use of targeted liposomes. The ActRII polypeptides can be ActRIIa and / or ActRIIb polypeptides (see Section 5.2)).

Various viral vectors which can be utilized for gene therapy as taught herein include adenovirus, herpes virus, vaccinia, or, preferably, an RNA virus such as a retrovirus. Preferably, the retroviral vector is a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous Sarcoma Virus (RSV). A number of additional retroviral vectors can incorporate multiple genes. All of these vectors can transfer or incorporate a gene for a selectable marker so that transduced cells can be identified and generated. Retroviral vectors can be made target-specific by attaching, for example, a sugar, a glycolipid, or a protein. Preferred targeting is accomplished by using an antibody. Those of skill in the art will recognize that specific polynucleotide sequences can be inserted into the retroviral genome or attached to a viral envelope to allow target specific delivery of the retroviral vector containing the ActRII polynucleotide. In a preferred embodiment, the vector is targeted to bone or cartilage.

Alternatively, tissue culture cells can be directly transfected with plasmids encoding the retroviral structural genes gag, pol and env, by conventional calcium phosphate transfection. These cells are then transfected with the vector plasmid containing the genes of interest. The resulting cells release the retroviral vector into the culture medium.

Another targeted delivery system for ActRII polynucleotides is a colloidal dispersion system. Colloidal dispersion systems include macromolecule complexes, nanocapsules, microspheres, beads, and lipid-based systems including oil-in-water emulsions, micelles, mixed micelles, and liposomes. The preferred colloidal system for use in the methods described herein is a liposome. Liposomes are artificial membrane vesicles which are useful as delivery vehicles in vitro and in vivo. RNA, DNA and intact virions can be encapsulated within the aqueous interior and be delivered to cells in a biologically active form (see e.g., Fraley, et al., Trends Biochem. Sci., 6:77, 1981). Methods for efficient gene transfer using a liposome vehicle, are known in the art, see e.g., Mannino, et al., Biotechniques, 6:682, 1988. The composition of the liposome is usually a combination of phospholipids, usually in combination with steroids, especially cholesterol. Other phospholipids or other lipids may also be used. The physical characteristics of liposomes depend on pH, ionic strength, and the presence of divalent cations.

Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Illustrative phospholipids include egg phosphatidylcholine, dipalmitoylphosphatidylcholine, and distearoylphosphatidylcholine. The targeting of liposomes is also possible based on, for example, organ-specificity, cell-specificity, and organelle-specificity and is known in the art.

In certain embodiments, the ActRII inhibitor is substantially pure in a pharmaceutical composition. Specifically, at most 20%, 10%, 5%, 2.5%, 1%, 0.1%, or at most 0.05% of the compounds in the pharmaceutical composition are compounds other than the ActRII inhibitor and the pharmaceutical acceptable carrier.

### 6. EXAMPLE

The example presented herein demonstrates that GDF11 protein levels are elevated in thalassemia and that inhibition of GDF11 can treat anemia in a mouse model of beta-thalassemia. Accordingly, the example provided herein demonstrates that GDF11 can be used as a biomarker for thalassemia, and that methods of treating anemia such as thalassemia can assess

### 6.1 AN ACTRIIA DECOY TREATS beta-THALASSEMIA

Beta-thalassemia is associated with ineffective erythropoiesis, accelerated erythoid differentiation and apoptosis, resulting in anemia and iron overload. The molecular mechanism underlying the effects of ineffective erythropoiesis is incompletely understood. Although members of the TGF-beta superfamily are implicated in both proliferation and differentiation of erythroid progenitor cells, the role of the numerous TGF-beta family members in the ineffective erythropoiesis seen in beta-thalassemia is unknown.

To assess the role of TGF-beta family members in the ineffective erythropoiesis of beta-thalassemia, a recombinant fusion protein that binds a number of TGF-beta superfamily ligands was used in a mouse model of human beta-thalassemia. The ActRIIA-Fc fusion protein (i.e., the murine counterpart of SEQ ID NO:7) consists of the extracellular domain of Activin Receptor IIA (ActRIIA) linked to a human immunoglobulin 1 (IgG1) Fc domain and the protein acts as a ligand trap for TGF-beta family members like activin A, activin B, growth differentiation factor-11 (GDF11) and bone morphogenetic protein-10 (BMP-10). Hbbth1/th1 is a mouse model of human beta-thalassemia, the mice having a naturally occurring deletion of the beta-major gene (Skow et al., 1983). Hbbth1/th1 mice have abnormally low hemoglobin (Hgb), hemocrit (Hct) and mean cell volume (MCV) as well as bone marrow (BM) hypercellularity and abnormally high levels of bilirubin, a by-product of hemoglobin breakdown that represents extensive red blood cell destruction.

### 6.2 MATERIALS AND METHODS

### 6.2.1 Mice

C57BL/6 were bred and housed in the pathogen-free facilities of INSERM U699. All protocols were approved by the Animal Care Committee of INSERM. The Hbbth1/th1 model was originated from a natural occurring deletion of the β-major gene (Skow LC et al; Cell 1983). Hbbth1/th1 mice constitute a model of β-thalassemia intermedia. These mice have several clinical parameters reproducing human β-thalassemia such as ineffective bone marrow erythropoiesis, precursor apoptosis, parenchymal iron distribution, decreased hepcidin expression and lower bone marrow iron levels while levels in the liver and spleen are increased.

### 6.2.2 Complete blood counts

Blood samples were collected in EDTA-coated tubes and complete blood counts were measured on a MS9-5 Blood Analyzer (Melet Schloesing Laboratories) according to the manufacturer's instructions. The selected parameters were red blood cells (RBC), hematocrit (Ht), mean corpuscular volume (MCV), hemoglobin (Hb). Reticulocyte numbers were determined with retic-count reagent (BD Biosciences Retic-Count™ Kit).

### 6.2.3 Quantitative real-time RT-PCR

RNA was extracted from erythroid progenitors using RNeasy Plus Mini Kit (Qiagen). One microgram of total RNA was used for reverse transcription using iScript reverse transcription Supermix (Bio-rad) during 30 minutes at 42°C. The enzyme was then inactivated at 85°C for 5 minutes. For qPCR, the cDNA samples were amplified in a CFX96 PCR System (Bio-rad). The PCR products were quantified using SsoFast EvaGreen Supermix (Bio-rad).

### 6.2.4 In vitro erythroblast cultures

Cells from each tissue were resuspended in serum-free "erythroid expansion media" consisting in either StemPro34 plus nutrient supplement (Life Technologies Gibco-BRL) supplemented with 2 U/mL human recombinant Epo (Roche), 100 ng/mL SCF (PeproTech), 10-6 M dexamethasone (D2915; Sigma), 40 and penicillin/streptomycin (Pen/Strep; Invitrogen). After 5 days of culture, the nonadherent cells were transferred to the differentiation medium (StemPro-34 supplemented with 1U/ml Epo and 1mg/ml ferro-transferrin (Sigma)) during two to three days supplemented or not with 10µg/ml of mActRIIA-Fc. Live and dead cell numbers were determined daily by Trypan Blue (Gibco/BRL) exclusion, and cell concentration was adjusted to 2 × 10⁶ total cells/mL daily through partial medium changes.

### 6.2.5 Methylcellulose assays

Single cell suspensions of adult mouse BM or spleen were mixed with methocult M3434 medium (Stem Cell Technologies), plated into 35 mm dishes and cultured at 37°C under a 5% CO₂ humidified atmosphere. The BFU-E colonies were scored at day 10 (in some experiments mouse BFU-E were scored from day 7 onward to day 10 and there was no difference between colony numbers at day 7 and at day 10).

### 6.2.6 Statistical analyses

Statistical analyses were performed with GraphPad Prism (version 5.0; GraphPad Software). The data are expressed as the mean ± SEM of N determinations unless noted otherwise. Student's t-test or the Mann-Whitney test were used to compare two groups, whereas multigroup comparisons were made using two-way ANOVA test followed by post-hoc analysis (Bonferroni test). Differences were considered significant at a P value less than 0.05 (*), less than 0.01 (**) or less than 0.001 (***).

### 6.2.7 Immunofluorescence analysis by flow cytometry

For bone marrow (BM) and splenocyte suspensions from mice, blocking of IgG receptors was performed with anti-FcgammaR mAb 2.4G2. Cells (1 x 106) were then stained with anti-TER-119 antibody and anti-mouse TfR1 antibody. Stained cells were further analyzed by flow cytometry (FACScalibur; Becton Dickinson) using FlowJo software (Tree Star).

### 6.2.8 Tissue collection and histology

Bone marrow and spleens were collected and fixed in 10% formalin, paraffin embedded, and sectioned at 3-6 µm for hematoxylin and eosin (H&E) staining.

### 6.2.9 Iron, ferritin, bilirubin and transferrin quantification.

Blood was drawn into heparinized tubes and centrifuged (5 min, 4 °C, 1,100g). Plasma was de novo centrifuged (5 min, 4 °C, 1,100g) to remove contaminating red blood cells. Biochemical parameters were quantified with an Olympus AU400 automat according to the manufacturer's instructions.

### 6.3 RESULTS

### 6.3.1 mActRIIA-Fc ameliorates hematological parameters in thalassemic mice.

beta-thalassemia is a disease characterized by a defect in hemoglobin synthesis leading to impaired erythrocyte maturation and production. The decrease in erythrocytes is primarily thought to be due to abnormally accelerated erythroid differentiation and apoptosis at the late basophilic/polychromatic erythroblast stage of red blood cell differentiation that results in an overall decrease in mature red blood cell production. The disease is characterized by a hypercellular bone marrow compartment in which abnormal erythroblasts accumulate and undergo apoptosis, resulting in systemic anemia.

To examine the role of TGF-BETA ligands in the disease mechanism of beta-thalassemia, Hbbth1/th1 mice were subcutaneously treated with mActRIIA-Fc (the murine counterpart of SEQ ID NO:7) or PBS for 0, 5, 10, 30 or 60 days (10mg/Kg body weight) twice a week (*p <0.05, N=3-5 for each independent experiment). Compared to PBS-treated animals, treatment with mActRIIA-Fc significantly increased red blood cell counts (FIG 1A) and hemocrit (FIG 1B) and hemoglobin (FIG 1C) levels, with a concomitant decrease in reticulocyte counts (FIG 1D) (from 10 days post-treatment until day 60). Analysis of circulating red blood cell (RBC) parameters also showed that mean corpuscular volume (MCV) (FIG IE), mean corpuscular hemoglobin (MCH) (FIG 1F) and MCH concentration (MCHC) (FIG 1G) all increased in all mice treated with mActRIIA-Fc, suggesting that mActRIIA-Fc improved the microcytic anemia of thalassemia and restored hemoglobin content per RBC. In addition, bone marrow and spleen cellularity and late basophilic/polychromatic erythroblasts were reduced significantly following treatment with mActRIIA-Fc. Morphological analysis of erythrocytes was evaluated by May-Grünwald (MGG) staining and showed a reduction in anisocytosis, poikilocytosis and target cells (FIG 1I) To determine the effect of mActRIIA-Fc on the anemia associated with beta-thalassemia, systemic iron levels (FIG 1J), transferrin synthesis (FIG 1K), transferrin saturation (FIG 1L) and ferritin levels (FIG 1M) were evaluated on thalassemic mice. Transferrin saturation was reduced with the induction of transferrin synthesis or decreased systemic iron levels (FIG 1L). Platelet, monocyte, lymphocyte and neutrophil levels were also assessed (FIG IN).

The effects of mActRIIA-Fc on splenomegaly in thalassemic mice was evaluated by spleen weight and measuring total spleen cell number. Spleen cell number and spleen weight were reduced in animals treated with mActRIIA-Fc compared to PBS-treated thalassemic mice (FIG 10). Similarly, bone marrow erythroblasts numbers and expansion (as determined by eosin/hematoxylin staining) (FIG IP) were also decreased in mActRIIA-Fc-treated mice. Bone marrow and spleen were harvested and erythroblasts were quantified by flow cytometry by TER119 staining. Treatment with mActRIIA-Fc significantly reduced the number of erythrocytes in thalassemic mice (FIG 1Q), indicating that it corrected the ineffective erythropoiesis in the mice.

### 6.3.2 mActRIIA-Fc reduces ineffective erythropoiesis in thalassemic mice.

To further explore the role of TGF-BETA superfamily ligands in ineffective erythropoiesis in beta-thalassemia, spleen (FIG 2A, FIG 2C) and bone marrow (FIG 2B, FIG 2C) were harvested and erythroblast differentiation evaluated by flow cytometry by CD71/TER119 staining and forward scatter/side scatter (FSC/SSC) distribution. A time course analysis of the percent of cells at progressive stages in erythropoietic differentiation, Proerythroblast (Pro-E), Basophil erythroblasts (Ery-A), late basophilic (Ery-B) and polychromatic erythroblasts and orthochromatic erythroblasts (Ery-C), showed that mice treated with mActRIIA-Fc presented significantly decreased immature TER119/CD71 cells (late basophilic and polychromatic erythroblasts, Ery-B) in spleen with a concomitant increase in the percentage of orthochromatic erythroblasts (Ery-C) (FIG 2A). These results were in accordance with mActRIIA-Fc reduction of ineffective erythropoiesis. Although there was a decrease in TER-199+ erythroblasts and in Ery-B numbers in bone marrow from mice treated with mActRIIA-Fc (FIG 2B), there was no increase in the amount of mature erythroblasts suggesting that ineffective erythropoiesis in the bone marrow of thalassemic mice is not corrected by treatment of mice with mActRIIA-Fc.

Chronic anemia of thalassemia induces stress erythropoiesis compensatory responses. However, these responses are unproductive because of ineffective erythropoiesis. Ineffective erythropoiesis is characterized by a requirement of RBC production which cannot be compensated by an accelerated proliferation and differentiation of immature erythroblasts due to the premature cell death by apoptosis of maturing cells. Therefore, imbalanced immature/mature erythroblasts ratio is a feature of ineffective erythropoiesis of thalassemia. To further study the impact of mActRIIA-Fc on erythroid differentiation and ineffective erythropoiesis, cell suspensions from mActRIIA-Fc-treated mice and their respective controls were labeled with antibodies to TfR1 and TER119. Erythroid precursors differentiation was analyzed by flow cytometry in TER119high gate as previously described (Liu et al., Blood 2006). Accordingly, mActRIIA-Fc-treated mice presented a decreased ratio of immature/mature erythroblasts ratio in spleen indicating the correction of ineffective stress erythropoiesis. In bone marrow the immature/mature erythroblasts ratio did not differ between control and mActRIIA-Fc-treated mice further suggesting that ineffective erythropoiesis in the bone marrow of thalassemic mice was not corrected by mActRIIA-Fc treatment. These results suggest that ActRIIa ligands contribute to erythroblast differentiation but also to ineffective spleen erythropoiesis in β-thalassemia.

Bilirubin is a product of hemoglobin degradation and increased plasma bilirubin resulting from hemolysis is a feature of ineffective erythropoiesis in β-thalassemia 18. In time-course analysis, serum levels of total and direct bilirubin were decreased in thalassemic mice treated with mActRIIA-Fc from 5 days of treatment suggesting that hemolysis resulting from ineffective erythropoiesis was affected by mActRIIA-Fc administration (FIG 2D). In agreement with bilirubin values, levels of serum lactate dehydrogenase (LDH) were also reduced in animals treated with mActRIIA-Fc compared to controls after 60 days of treatment (FIG 2D), further confirming that tissue hemolysis was reduced in mice treated with mActRIIA-Fc.

Late-stage erythropoiesis is largely committed to the production of the oxygen carrier hemoglobin (Hb), a tetrameric protein composed of two α-globin and two β-globin subunits. β-thalassemia is a common inherited hemoglobinopathy characterized by impaired or absent β-globin gene production with subsequent accumulation of unpaired α-subunits. The excess of unbound free α-globin in maturing erythroid cells precipitates and leads to the production of reactive oxygen species (ROS) and cellular oxidative stress damage inducing premature death of erythroid precursors. The impact of mActRIIA-Fc on the generation of globin precipitates was further investigated. ROS generation on primary pro-erythroblast differentiation was evaluated by flow cytometry using dichlorodihydrofluorescein (FIG 2E). Analysis of hemoglobin solubility on primary thalassemic pro-erythroblasts treated for 48 hours with mActRIIA-Fc or PBS (FIG 2F).

To gain insight into the cellular mechanisms associated with treatment with mActRIIA-Fc, spleen-derived proeryhtoblasts were cultured and recovered from Hbbth1/th1 mice in the presence or in the absence of mActRIIA-Fc. A well-established in vitro model of pro-erythroblast differentiation was used (spleen precursors were cultured for 5 days in serum free stem cell expansion medium supplemented with mouse Stem Cell Factor, Epo and dexamethasone). These pro-erythroblast-enriched cultures were then differentiated in the presence of 1U/ml Epo and 1mg/ml Fe-Tf for three days. Similar to in vivo observations, treatment with mActRIIA-Fc increased total amounts of hemoglobin in thalassemic erythroblasts. However, those cells presented decreased amounts of membrane-associated precipitated hemoglobin when compared to control treated cells (FIG 2F). Accordingly, the amount of reactive oxygen species (ROS) detected on those cells decreased in cells treated with mActRIIA-Fc (FIG 2E). Therefore, treatment with mActRIIA-Fc resulted in decreased cytotoxic globin precipitation and its associated ROS production, contributing to ineffective erythropoiesis. Altogether, these data suggested that targeting ActRIIa signaling modulated erythroblast differentiation and trapping of ActRIIa ligands by mActRIIA-Fc corrected ineffective erythropoiesis by favoring the formation of mature erythroblasts and reducing membrane-associated hemoglobin precipitates.

### 6.3.3 mActRIIA-Fc modulates apoptosis in thalassemic mice.

The involvement of TGF-beta family members on the apoptotic process in beta-thalassemia-associated ineffective erythropoiesis was investigated by analyzing the expression of pro-apoptotic proteins on erythrocytes using flow cytometry. Although there were no significant changes in the expression of the apoptotic proteins in bone marrow erythroblasts (FIG 3A), analysis of spleen erythroblasts showed that Fas-L was increased on the Ery-B population of cells (FIG 3B). Multiparametric flow cytometry comparative analysis on spleen cells from mice treated with PBS and mActRIIA-Fc showed that Fas-L expression was increased on immature late basophilic and polychromatic erythroblasts (Ery-B) and decreased on orthochromatic erythroblasts (Ery-C) (FIG 3B). In contrast, Fas-L expression was not modulated on BM erythroblasts (FIG 3A). Together, these results showed that ActRIIa signaling modulated Fas/Fas-L pathway on maturing erythroblasts. Collectively, these data suggested that ActRIIa signaling induced premature cell death of maturing erythroblasts committed to ineffective erythropoiesis. Surprisingly, Fas-L expression was decreased on Ery-A and Ery-C subsets of cells showing that the impact of ActRIIa signaling was more pronounced on maturing erythroblasts (FIG 3B). The number of tunel positive cells was also increased in animals treated with mActRIIA-Fc (FIG 3C). Ineffective erythropoiesis of thalassemia is characterized by a massive apoptosis of maturing erythroblasts. Studying the impact of treatment of mActRIIA-Fc on apoptosis indicated that treatment of mice with mActRIIA-Fc presented decreased numbers of tunnel positive cells compared to their respective controls (FIG 3C), suggesting that ActRIIa signaling trough Smad-2,3 activation could control ineffective erythropoiesis by modulating apoptosis levels of maturing erythroblasts.

### 6.3.4 Activin/GDF11 ligands are overexpressed in spleen from thalassemic mice.

RNA (mRNA) expression levels of ActRII, activin A, activin B and GDF11 were evaluated in spleen from wild-type and thalassemic mice by qPCR. The mRNA levels of ActRII, activin A, activin B and GDF11 were all increased suggesting that mActRIIA-Fc could be acting through one of its ligands in its improvement of ineffective erythropoiesis (FIG 4A). Western blot analysis of protein obtained from the spleen from thalassemic mice treated with mActRIIA-Fc showed a significant decrease in GDF11 protein levels compared to PBS-treated mice (FIG 4B), further implicating GDF11 as the ActRIIA ligand responsible for ineffective erythropoiesis. Further immunohistochemical analysis revealed that GDF11 protein levels (and to a much lesser extent activin A or activin B) were greatly increased on spleen biopsies from thalassemic mice and were abrogated in animals treated with mActRIIA-Fc (FIG 3A). These results were further confirmed by immunoblotting (FIG 4B). In contrast to spleen sections, analysis of ActRIIa ligands on BM showed no accumulation of GDF11 in thalassemic mice (FIG 4B). Therefore, GDF11 overexpression in spleen section of thalassemic mice could be associated with ineffective erythropoiesis.

### 6.3.5 mActRIIA-Fc reduces the increased GDF11 expression levels seen in primary thalassemic pro-erythroblasts.

To determine which TGF-beta family members might be implicated in the treatment of beta-thalassemia with mActRIIA-Fc, immunohistochemical analysis of proteins in the activin/GDF signaling pathway was performed. Thalassemic mice were treated with PBS or mActRIIA-Fc for 30 days and spleen was harvested, fixed and stained for activin A, activin B, GDF8, GDF11, ActRII and p-Smad2 (FIG 5A). Immunohistochemical staining showed increased levels of GDF11, ActRII and p-Smad2 in thalassemic mice. To explore whether proteins in the activin/GDF signaling pathway were overexpressed in other mouse models of anemia, the expression of activin A, activin B and GDF11 in thalassemic mice was compared to normoxia, hypoxia and alphaRBC mice (FIG 5B). FACS analysis of primary thalassemic pro-erythroblasts treated with PBS or mActRIIA-Fc for 48 hours then incubated with specific antibodies against activin A, activin B, the GDF11 propeptide and the GDF8/GDF11 cleaved peptide indicated that mActRIIA-Fc treatment normalized GDF11 expression. Quantification of GDF11 staining indicated that treatment of mice with mActRIIA-Fc significantly reduced GDF11 levels (FIG 5C). This reduction in GDF11 expression upon treatment of thalassemic mice with mActRIIA-Fc was confirmed by immunohistochemical analysis of the spleens of the mice (FIG. 5D). * p<0.05, N=4. Thus, the fact that mActRIIA-Fc decreased GDF11 overexpression in thalassemic tissues was additional evidence that mActRIIA-Fc corrected ineffective erythropoiesis by targeting GDF11.

### 6.3.6 Neutralization of GDF11 restores erythroblast differentiation.

To determine if increased GDF11 expression was responsible for ineffective erythropoiesis, thalassemic pro-erythroblasts were cultured in the presence of blocking antibodies directed against activins A and B and GDF11. Anti-GDFl 1 antibodies (but not activin A and B antibodies) promoted erythropoiesis, further confirming that GDF11 negatively regulated erythropoiesis by inducing ineffective erythropoiesis as quantified by flow cytometry following CD71/TER119 staining (FIG. 6A). ROS generation on primary pro-erythroblast differentiation was evaluated by flow cytometry using dichlorodihydrofluorescein (FIG. 6B). *p<0.05, N=4. Thus, anti-GDFl 1 blocking antibodies restored cell differentiation and reduced hemoglobin aggregates in thalassemic erythroblasts.

### 6.3.7 An ActRIIA ligand-detecting assay

An assay was developed to detect, identify and quantify ActRIIA ligands present in blood serum and, specifically, their abnormal expression in diseases associated with ineffective erythropoiesis (e.g., thalassemia, myelodysplastic syndromes, chronic pernicious anemia and sickle cell anemia). The assay consists of a sandwich ELISA based on an ActRIIA-Fc coating followed by the detection of ActRIIA ligands present in serum. The ActRIIA ELISA assay can be used experimentally (for e.g., animals) or clinically (for e.g., human patients) to identify, detect and/or quantify ligand or receptor levels in blood serum in order to aid in treatment decisions and/or determine the effectiveness of a treatment designed to modulate TGF-beta ligand or receptor levels.

### 6.3.8 GDF11 levels are elevated in the serum of thalassemia patients.

The sandwich ELISA assay described above was developed to measure GDF11 levels in serum. ELISA plates were coated with 5 microg/mL of mActRIIA-Fc (FIG 7A). Increasing doses of recombinant GDF11 were added to the plates (0.1 ng/microliter, 0.5 ng/microliter, 2.5 ng/microliter). The plates were washed with PBS 0.1% Tween and bound proteins detected using anti-GDF8/11 antibodies, followed by detection using a horseradish peroxidase-coupled anti-rabbit IgG. GDF11 bound the plates coated with mActRIIA-Fc in a dose-dependent manner indicating that the assay could effectively be used to detect and quantify GDF11 levels (FIG 7B). The ELISA using mActRIIA-Fc detected as little as 100 pg/mL of recombinant GDF11.

Serum from patients having thalassemia was tested for GDF11 expression using the sandwich ELISA with mActRIIA-Fc. As shown in FIG 8, GDF11 levels were elevated 3-fold in patients with thalassemia compared to levels in healthy controls.

To determine if the levels of other ActRIIA ligands were also elevated in thalassemia patients, activin A and activin B expression levels were also measured in the serum of those patients. An ELISA assay was also developed to detect activin A and activin B. ELISA plates were coated with 5 microgram/mL of ActRIIA-Fc. Increasing doses of recombinant activin A and activin B were added to the plates (0.1 ng/microliter, 0.5 ng/microliter, 2.5 ng/microliter). The plates were washed with PBS 0.1% Tween, and bound proteins detected with anti-activin A (FIG 9A) and anti-activin B (FIG 10A) antibodies (R&D systems), followed by detection using a horseradish peroxidase-coupled anti-rabbit IgG. Both activin A (FIG 9B) and activin B (FIG 10B) bound the ActRIIA-Fc-coated plates in a dose-dependent manner indicating that the assay could effectively be used to detect and quantify both proteins. Like GDF11, activin A and activin B were also detected at a levels as low as 100 pg/mL.

The ActRIIA-Fc ELISA was used to determine the expression levels activin A and activin B in the serum of patients with thalassemia. In contrast to GDF11, neither activin A (FIG 9C), nor activin B (FIG 10C) protein levels were elevated in thalassemic patients, indicating that the ActRIIA ligand GDF11 was uniquely implicated in the thalassemia disease process.

### 6.3.9 mActRIIA-Fc does not change hematological parameters in wild-type mice.

Wild-type C57BL/6 mice were treated for 30 days with mActRIIA-Fc (10mg/Kg BW twice a week) or PBS. Evaluation of red blood cell counts (FIG 11A), hematocrit (FIG 11B), hemoglobin (FIG 11C) indicated no change in the levels of the parameters as a result of treatment of the mice with mActRIIA-Fc. Only a slight decrease in reticulocytosis was observed (FIG 11D). mActRIIA-Fc also did not alter red blood cells (RBC) parameters such as MCV (FIG 11E), MCH (FIG 11F) and MCHC (FIG 12G). *p<0.05, N=3-5 for each independent experiment.

The effect of mActRIIA-Fc on the spleen and bone marrow was also assessed in wildtype C57BL/6 mice. mActRIIA-Fc increased spleen weight in wildtype mice, but had no significant effect on spleen cell number. mActRIIA-Fc also had no effect on bone marrow cell number (FIG 12).

### 6.3.10 mACTRIIA-FC STIMULATES ERYTHROPOIETIC DIFFERENTIATION THROUGH INHIBITION OF GDF11

In order to investigate the cellular mechanism by which mActRIIA-Fc increased red blood cell parameters, a series of in vitro experiments were conducted in which no evidence was found to support direct effects of mActRIIA-Fc on human CD34+ cells as assessed in colony formation assays (FIG. 13A) and in erythroid differentiation in liquid culture (FIGs 13B and 13C). As both clinical and pharmacological findings pointed to a clear role for mActRIIA-Fc in stimulating RBC parameters, it was hypothesized that the effects of mActRIIA-Fc could be mediated by accessory cells in the bone marrow (BM) microenvironment. Human CD36+ cells, which are highly enriched for erythroid progenitors, were co-cultured with long-term BM cultures and then their erythroid differentiation was assessed following 6 days of culture in EPO (2U/mL)- supplemented media. At day 6, the output of the cultures was predominantly characterized as EryA (∼basophilic erythroblast) but with the addition of mActRIIA-Fc (50µM), a significant fraction of CD36+ cells matured into EryB/C cells (polychromatic/orthochromatic erythroblasts), suggesting that factors produced by BM accessory cells mediated erythropoietic effects of mActRIIA-Fc and that, in contrast to EPO, mActRIIA-Fc could play a role in the latter stages or erythroblast maturation (FIGs 13D - 13F). To identify cytokines that might mediate the effects of mActRIIA-Fc, CD36+ cells were treated with several ActRIIA ligands. GDF11 treatment significantly decreased proliferation of glycoprotein A positive (GPA+) cells during the differentiation process and mActRIIA-Fc effectively reversed this effect, while having no consequence on untreated cells (FIGs 13G and 13H). These data show that inhibition of GDF11 mediates the erythropoietic stimulatory effects of mActRIIA-Fc.

### 6.4 CONCLUSION

Altogether, the data demonstrate that activin/BMP signaling controls erythroblast differentiation and that targeting BMP type II/activin type II receptors can decrease ineffective erythropoiesis and improve anemia in beta-thalassemia. In particular, the data show the involvement of GDF11 in beta-thalassemia-associated anemia and indicate that GDF11 levels are important as a biomarker in subjects having anemia.

### 7. DESCRIPTION OF THE SEQUENCES

**Table 1: Sequence Information**

| SEQ ID NO: | Description | Sequence |
|---|---|---|
| 1 | human ActRIIA precursor polypeptide | MGAAAKLAFAVFLISCSSGAILGRSETQECLFFNANWEKDRTNQTGVEPC YGDKDKRRHCFATWKNISGSIEIVKQGCWLDDINCYDRTDCVEKKDSPEV YFCCCEGNMCNEKFSYFPEMEVTQPTSNPVTPKPPYYNILLYSLVPLMLI AGIVICAFWVYRHHKMAYPPVLVPTQDPGPPPPSPLLGLKPLQLLEVKAR GRFGCVWKAQLLNEYVAVKIFPIQDKQSWQNEYEVYSLPGMKHENILQFI GAEKRGTSVDVDLWLITAFHEKGSLSDFLKANVVSWNELCHIAETMARGL AYLHEDIPGLKDGHKPAISHRDIKSKNVLLKNNLTACIADFGLALKFEAG KSAGDTHGQVGTRRYMAPEVLEGAINFQRDAFLRIDMYAMGLVLWELASR CTAADGPVDEYMLPFEEEIGQHPSLEDMQEVVVHKKKRPVLRDYWQKHAG MAMLCETIEECWDHDAEARLSAGCVGERITQMQRLTNIITTEDIVTVVTM VTNVDFPPKESSL |
| 2 | human ActRIIA soluble (extracellular), processed polypeptide sequence | ILGRSETQECLFFNANWEKDRTNQTGVEPCYGDKDKRRHCFATWKNISGS IEIVKQGCWLDDINCYDRTDCVEKKDSPEVYFCCCEGNMCNEKFSYFPEM EVTQPTSNPVTPKPP |
| 3 | human ActRIIA soluble (extracellular), processed polypeptide sequence with the C-terminal 15 amino acids deleted | ILGRSETQECLFFNANWEKDRTNQTGVEPCYGDKDKRRHCFATWKNISGS IEIVKQGCWLDDINCYDRTDCVEKKDSPEVYFCCCEGNMCNEKFSYFPEM |
| 4 | nucleic acid sequence encoding human ActRIIA precursor protein | ATGGGAGCTGCTGCAAAGTTGGCGTTTGCCGTCTTTCTTATCTCCTGTTC TTCAGGTGCTATACTTGGTAGATCAGAAACTCAGGAGTGTCTTTTCTTTA ATGCTAATTGGGAAAAAGACAGAACCAATCAAACTGGTGTTGAACCGTGT TATGGTGACAAAGATAAACGGCGGCATTGTTTTGCTACCTGGAAGAATAT TTCTGGTTCCATTGAAATAGTGAAACAAGGTTGTTGGCTGGATGATATCA ACTGCTATGACAGGACTGATTGTGTAGAAAAAAAAGACAGCCCTGAAGTA TATTTTTGTTGCTGTGAGGGCAATATGTGTAATGAAAAGTTTTCTTATTT |
| | | TCCAGAGATGGAAGTCACACAGCCCACTTCAAATCCAGTTACACCTAAGC CACCCTATTACAACATCCTGCTCTATTCCTTGGTGCCACTTATGTTAATT GCGGGGATTGTCATTTGTGCATTTTGGGTGTACAGGCATCACAAGATGGC CTACCCTCCTGTACTTGTTCCAACTCAAGACCCAGGACCACCCCCACCTT CTCCATTACTAGGGTTGAAACCACTGCAGTTATTAGAAGTGAAAGCAAGG GGAAGATTTGGTTGTGTCTGGAAAGCCCAGTTGCTTAACGAATATGTGGC TGTCAAAATATTTCCAATACAGGACAAACAGTCATGGCAAAATGAATACG AAGTCTACAGTTTGCCTGGAATGAAGCATGAGAACATATTACAGTTCATT GGTGCAGAAAAACGAGGCACCAGTGTTGATGTGGATCTTTGGCTGATCAC AGCATTTCATGAAAAGGGTTCACTATCAGACTTTCTTAAGGCTAATGTGG TCTCTTGGAATGAACTGTGTCATATTGCAGAAACCATGGCTAGAGGATTG GCATATTTACATGAGGATATACCTGGCCTAAAAGATGGCCACAAACCTGC CATATCTCACAGGGACATCAAAAGTAAAAATGTGCTGTTGAAAAACAACC TGACAGCTTGCATTGCTGACTTTGGGTTGGCCTTAAAATTTGAGGCTGGC AAGTCTGCAGGCGATACCCATGGACAGGTTGGTACCCGGAGGTACATGGC TCCAGAGGTATTAGAGGGTGCTATAAACTTCGAAAGGGATGCATTTTTGA GGATAGATATGTATGCCATGGGATTAGTCCTATGGGAACTGGCTTCTCGC TGTACTGCTGCAGATGGACCTGTAGATGAATACATGTTGCCATTTGAGGA GGAAATTGGCCAGCATCCATCTCTTGAAGACATGCAGGAAGTTGTTGTGC ATAAAAAAAAGAGGCCTGTTTTAAGAGATTATTGGCAGAAACATGCTGGA ATGGCAATGCTCTGTGAAACCATTGAAGAATGTTGGGATCACGACGCAGA AGCCAGGTTATCAGCTGGATGTGTAGGTGAAAGAATTACCCAGATGCAGA GACTAACAAATATTATTACCACAGAGGACATTGTAACAGTGGTCACAATG GTGACAAATGTTGACTTTCCTCCCAAAGAATCTAGTCTATGA |
| 5 | nucleic acid sequence encoding a human ActRIIA soluble (extracellular) polypeptide | ATACTTGGTAGATCAGAAACTCAGGAGTGTCTTTTCTTTAATGCTAATTG GGAAAAAGACAGAACCAATCAAACTGGTGTTGAACCGTGTTATGGTGACA AAGATAAACGGCGGCATTGTTTTGCTACCTGGAAGAATATTTCTGGTTCC ATTGAAATAGTGAAACAAGGTTGTTGGCTGGATGATATCAACTGCTATGA CAGGACTGATTGTGTAGAAAAAAAAGACAGCCCTGAAGTATATTTTTGTT GCTGTGAGGGCAATATGTGTAATGAAAAGTTTTCTTATTTTCCAGAGATG GAAGTCACACAGCCCACTTCAAATCCAGTTACACCTAAGCCACCC |
| 6 | fusion protein comprising a soluble extracellular domain of ActRIIA fused to an Fc domain | THTCPPCPAPELLGGPSVFLFPPKPKDTLMISRTPEVTCVVVD(A)VSHE DPEVKFNWYVDGVEVHNAKTKPREEQYNSTYRVVSVLTVLHQDWLNGKEY KCK(A)VSNKALPVPIEKTISKAKGQPREPQVYTLPPSREEMTKNQVSLT |
| | | CLVKGFYPSDIAVEWESNGQPENNYKTTPPVLDSDGPFFLYSKLTVDKSR WQQGNVFSCSVMHEALHN(A)HYTQKSLSLSPGK* |
| 7 | Extracellular domain of human ActRIIA fused to a human Fc domain | ILGRSETQECLFFNANWEKDRTNQTGVEPCYGDKDKRRHCFATWKNISGS IEIVKQGCWLDDINCYDRTDCVEKKDSPEVYFCCCEGNMCNEKFSYFPEM EVTQPTSNPVTPKPPTGGGTHTCPPCPAPELLGGPSVFLFPPKPKDTLMI SRTPEVTCVVVDVSHEDPEVKFNWYVDGVEVHNAKTKPREEQYNSTYRVV SVLTVLHQDWLNGKEYKCKVSNKALPVPIEKTISKAKGQPREPQVYTLPP SREEMTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 8 | Leader sequence of Honey bee mellitin (HBML) | MKFLVNVALVFMVVYISYIYA |
| 9 | Leader sequence of Tissue Plasminogen Activator (TPA) | MDAMKRGLCCVLLLCGAVFVSP |
| 10 | Native ActRIIA leader | MGAAAKLAFAVFLISCSSGA |
| 11 | ActRIIA-hFc and ActRIIA-mFc N-terminal sequence | ILGRSETQE |
| 12 | ActRIIA-Fc Protein with deletion of the C-terminal 15 amino acids of the extracellular domain of ActRIIA | ILGRSETQECLFFNANWEKDRTNQTGVEPCYGDKDKRRHCFATWKNISGS IEIVKQGCWLDDINCYDRTDCVEKKDSPEVYFCCCEGNMCNEKFSYFPEM TGGGTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKE YKCKVSNKALPVPIEKTISKAKGQPREPQVYTLPPSREEMTKNQVSLTCL VKGFYPSDIAVEWESNGQPENNYKTTPPVLDSDGSFFLYSKLTVDKSRWQ QGNVFSCSVMHEALHNHYTQKSLSLSPGK |
| 13 | Unprocessed ActRIIA-hFc with TPA leader sequence | MDAMKRGLCCVLLLCGAVFVSPGAAILGRSETQECLFFNANWEKDRTNQT GVEPCYGDKDKRRHCFATWKNISGSIEIVKQGCWLDDINCYDRTDCVEKK DSPEVYFCCCEGNMCNEKFSYFPEMEVTQPTSNPVTPKPPTGGGTHTCPP CPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKAL |
| | | PVPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVM HEALHNHYTQKSLSLSPGK |
| 14 | Nucleic acid sequence encoding Unprocessed ActRIIA-hFc with TPA leader sequence | ATGGATGCAATGAAGAGAGGGCTCTGCTGTGTGCTGCTGCTGTGTGGAGC AGTCTTCGTTTCGCCCGGCGCCGCTATACTTGGTAGATCAGAAACTCAGG AGTGTCTTTTTTAATGCTAATTGGGAAAAAGACAGAACCAATCAAACTG GTGTTGAACCGTGTTATGGTGACAAAGATAAACGGCGGCATTGTTTTGCT ACCTGGAAGAATATTTCTGGTTCCATTGAATAGTGAAACAAGGTTGTTGG CTGGATGATATCAACTGCTATGACAGGACTGATTGTGTAGAAAAAAAAGA CAGCCCTGAAGTATATTTCTGTTGCTGTGAGGGCAATATGTGTAATGAAA AGTTTTCTTATTTTCCGGAGATGGAAGTCACACAGCCCACTTCAAATCCA GTTACACCTAAGCCACCCACCGGTGGTGGAACTCACACATGCCCACCGTG CCCAGCACCTGAACTCCTGGGGGGACCGTCAGTCTTCCTCTTCCCCCCAA AACCCAAGGACACCCTCATGATCTCCCGGACCCCTGAGGTCACATGCGTG GTGGTGGACGTGAGCCACGAAGACCCTGAGGTCAAGTTCAACTGGTACGT GGACGGCGTGGAGGTGCATAATGCCAAGACAAAGCCGCGGGAGGAGCAGT ACAACAGCACGTACCGTGTGGTCAGCGTCCTCACCGTCCTGCACCAGGAC TGGCTGAATGGCAAGGAGTACAAGTGCAAGGTCTCCAACAAAGCCCTCCC AGTCCCCATCGAGAAAACCATCTCCAAAGCCAAAGGGCAGCCCCGAGAAC CACAGGTGTACACCCTGCCCCCATCCCGGGAGGAGATGACCAAGAACCAG GTCAGCCTGACCTGCCTGGTCAAAGGCTTCTATCCCAGCGACATCGCCGT GGAGTGGGAGAGCAATGGGCAGCCGGAGAACAACTACAAGACCACGCCTC CCGTGCTGGACTCCGACGGCTCCTTCTTCCTCTATAGCAAGCTCACCGTG GACAAGAGCAGGTGGCAGCAGGGGAACGTCTTCTCATGCTCCGTGATGCA TGAGGCTCTGCACAACCACTACACGCAGAAGAGCCTCTCCCTGTCTCCGG GTAAATGAGAATTC |
| 15 | human ActRIIB soluble (extracellular), processed polypeptide sequence with the N-terminal 6 amino acids of the EC domain deleted and the C-terminal 4 amino acids of the EC domain deleted (amino acids 25-130 of SEQ ID NO:28) and with an L79D mutation | ETRECIYYNANWELERTNQSGLERCEGEQDKRLHCYASWRNSSGTIELV KKGCWDDDFNCYDRQECVATEENPQVYFCCCEGNFCNERFTHLPEAGGP EVTYEPPP |
| 16 | human ActRIIB precursor protein sequence (A64) | MTAPWVALALLWGSLW PGSGRGEAETRECIYY NANWELERTNQSGLER CEGEQDKRLHCYASWA NSSGTIELVKKGCWLD DFNCYDRQECVATEEN PQVYFCCCEGNFCNER FTHLPEAGGPEVTYEP PPTAPTLLTVLAYSLL PIGGLSLIVLLAFWMY RHRKPPYGHVDIHEDP GPPPPSPLVGLKPLQL LEIKARGRFGCVWKAQ LMNDFVAVKIFPLQDK QSWQSEREIFSTPGMK HENLLQFIAAEKRGSN LEVELWLITAFHDKGS LTDYLKGNIITWNELC HVAETMSRGLSYLHED VPWCRGEGHKPSIAHR DFKSKNVLLKSDLTAV LADFGLAVRFEPGKPP GDTHGQVGTRRYMAPE VLEGAINFQRDAFLRI DMYAMGLVLWELVSRC KAADGPVDEYMLPFEE EIGQHPSLEELQEVVV HKKMRPTIKDHWLKHP GLAQLCVTIEECWDHD AEARLSAGCVEERVSL IRRSVNGTTSDCLVSL VTSVTNVDLPPKESSI |
| 17 | human ActRIIB soluble (extracellular), processed polypeptide sequence (amino acids 19-134 of SEQ ID NO:16) | SGRGEAETRECIYYNANWELERTNQSGLERCEGEQDKRLHCYASWANSSG TIELVKKGCWLDDFNCYDRQECVATEENPQVYFCCCEGNFCNERFTHLPE AGGPEVTYEPPPTAPT |
| 18 | human ActRIIB soluble (extracellular), processed polypeptide sequence with the C-terminal 15 amino acids deleted (amino acids 19-119 of SEQ ID NO:16) | SGRGEAETRECIYYNANWELERTNQSGLERCEGEQDKRLHCYASWANSSG TIELVKKGCWLDDFNCYDRQECVATEENPQVYFCCCEGNFCNERFTHLPE A |
| 19 | nucleic acid sequence encoding a human ActRIIB (A64) precursor protein | ATGACGGCGCCCTGGGTGGCCCTCGCCCTCCTCTGGGGATCGCTGTGGCC |
| | | CGGCTCTGGGCGTGGGAGGCTGAGACACGGAGTGCATCTACTACAACG CCAACTGGGAGCTGGAGCGCACCAACCAGAGCGGCCTGGAGCGCTGCGAA GGCGAGCAGGACAAGCGGCTGCACTGCTACGCCTCCTGGGCCAACAGCTC TGGCACCATCGAGCTCGTGAAGAAGGGCTGCTGGCTAGATGACTTCAACT GCTACGATAGGCAGGAGTGTGTGGCCACTGAGGAGAACCCCCAGGTGTAC TTCTGCTGCTGTGAAGGCAACTTCTGCAACGAGCGCTTCACTCATTTGCC AGAGGCTGGGGGCCCGGAAGTCACGTACGAGCCACCCCCGACAGCCCCCA CCCTGCTCACGGTGCTGGCCTACTCACTGCTGCCCATCGGGGGCCTTTCC CTCATCGTCCTGCTGGCCTTTTGGATGTACCGGCATCGCAAGCCCCCCTA CGGTCATGTGGACATCCATGAGGACCCTGGGCCTCCACCACCATCCCCTC TGGTGGGCCTGAAGCCACTGCAGCTGCTGGAGATCAAGGCTCGGGGGCGC TTTGGCTGTGTCTGGAAGGCCCAGCTCATGAATGACTTTGTAGCTGTCAA GATCTTCCCACTCCAGGACAAGCAGTCGTGGCAGAGTGAACGGGAGATCT TCAGCACACCTGGCATGAAGCACGAGAACCTGCTACAGTTCATTGCTGCC GAGAAGCGAGGCTCCAACCTCGAAGTAGAGCTGTGGCTCATCACGGCCTT CCATGACAAGGGCTCCCTCACGGATTACCTCAAGGGGAACATCATCACAT GGAACGAACTGTGTCATGTAGCAGAGACGATGTCACGAGGCCTCTCATAC CTGCATGAGGATGTGCCCTGGTGCCGTGGCGAGGGCCACAAGCCGTCTAT TGCCCACAGGGACTTTAAAAGTAAGAATGTATTGCTGAAGAGCGACCTCA CAGCCGTGCTGGCTGACTTTGGCTTGGCTGTTCGATTTGAGCCAGGGAAA CCTCCAGGGGACACCCACGGACAGGTAGGCACGAGACGGTACATGGCTCC TGAGGTGCTCGAGGGAGCCATCAACTTCCAGAGAGATGCCTTCCTGCGCA TTGACATGTATGCCATGGGGTTGGTGCTGTGGGAGCTTGTGTCTCGCTGC AAGGCTGCAGACGGACCCGTGGATGAGTACATGCTGCCCTTTGAGGAAGA GATTGGCCAGCACCCTTCGTTGGAGGAGCTGCAGGAGGTGGTGGTGCACA AGAAGATGAGGCCCACCATTAAAGATCACTGGTTGAAACACCCGGGCCTG GCCCAGCTTTGTGTGACCATCGAGGAGTGCTGGGACCATGATGCAGAGGC TCGCTTGTCCGCGGGCTGTGTGGAGGAGCGGGTGTCCCTGATTCGGAGGT CGGTCAACGGCACTACCTCGGACTGTCTCGTTTCCCTGGTGACCTCTGTC ACCAATGTGGACCTGCCCCCTAAAGAGTCAAGCATCTAA |
| 20 | fusion protein comprising a soluble extracellular domain of ActRIIB (A64; SEQ ID NO:17) fused to an Fc domain | SGRGEAETRECIYYNANWELERTNQSGLERCEGEQDKRLHCYASWANSSG TIELVKKGCWLDDFNCYDRQECVATEENPQVYFCCCEGNFCNERFTHLPE AGGPEVTYEPPPTAPTGGGTHTCPPCPAPELLGGPSVFLFPPKPKDTLMI SRTPEVTCVVVDVSHEDPEVKFNWYVDGVEVHNAKTKPREEQYNSTYRVV |
| | | SVLTVLHQDWLNGKEYKCKVSNKALPVPIEKTISKAKGQPREPQVYTLPP SREEMTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 21 | fusion protein comprising a soluble extracellular domain of ActRIIB (A64) with the C-terminal 15 amino acids deleted (SEQ ID NO:18) fused to an Fc domain | SGRGEAETRECIYYNANWELERTNQSGLERCEGEQDKRLHCYASWANSSG TIELVKKGCWLDDFNCYDRQECVATEENPQVYFCCCEGNFCNERFTHLPE AGGGTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKE YKCKVSNKALPVPIEKTISKAKGQPREPQVYTLPPSREEMTKNQVSLTCL VKGFYPSDIAVEWESNGQPENNYKTTPPVLDSDGSFFLYSKLTVDKSRWQ QGNVFSCSVMHEALHNHYTQKSLSLSPGK |
| 22 | human ActRIIB soluble (extracellular), processed polypeptide sequence with the N-terminal 6 amino acids of the EC domain deleted and the C-terminal 5 amino acids of the EC domain deleted (amino acids 25-129 of SEQ ID NO:28) and with an L79D mutation | ETRECIYYNANWELERTNQSGLERCEGEQDKRLHCYASWRNSSGTIELV KKGCWDDDFNCYDRQECVATEENPQVYFCCCEGNFCNERFTHLPEAGGP EVTYEPP |
| 23 | human ActRIIB soluble (extracellular), processed polypeptide sequence with the N-terminal 6 amino acids of the EC domain deleted and the C-terminal 3 amino acids of the EC domain deleted (amino acids 25-131 of SEQ ID NO:28) and with an L79D mutatioN | ETRECIYYNANWELERTNQSGLERCEGEQDKRLHCYASWRNSSGTIELV KKGCWDDDFNCYDRQECVATEENPQVYFCCCEGNFCNERFTHLPEAGGP EVTYEPPPT |
| 24 | Unprocessed ActRIIB-Fc fusion protein with the N-terminal 6 amino acids of the EC domain deleted and the C-terminal 3 amino acids of the EC domain deleted (amino acids 25-131 of SEQ ID NO:28) and with an L79D mutation and with TPA leader sequence | MDAMKRGLCCVLLLCGAVFVSPGAAETRECIYYNANWELERTNQSGLERC EGEQDKRLHCYASWRNSSGTIELVKKGCWDDDFNCYDRQECVATEENPQV YFCCCEGNFCNERFTHLPEAGGPEVTYEPPPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 25 | Processed ActRIIB-Fc fusion protein with the N-terminal 6 amino acids of the EC domain deleted and the C-terminal 3 amino acids of the EC domain deleted (amino acids 25-131 of SEQ ID NO:28) and with an L79D mutation | ETRECIYYNANWELERTNQSGLERCEGEQDKRLHCYASWRNSSGTIELVK KGCWDDDFNCYDRQECVATEENPQVYFCCCEGNFCNERFTHLPEAGGPEV TYEPPPTGGGTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLTV DKSRWQQGNVFSCSVMHEALHNHYTQKSLSLSPGK* |
| 26 | human ActRIIB soluble (extracellular), processed polypeptide sequence (amino acids 20-134 of SEQ ID NO:16) | GRGEAETRECIYYNANWELERTNQSGLERCEGEQDKRLHCYASWANSSG TIELVKKGCWLDDFNCYDRQECVATEENPQVYFCCCEGNFCNERFTHLPE AGGPEVTYEPPPTAPT |
| 27 | human ActRIIB soluble (extracellular), processed polypeptide sequence with the C-terminal 15 amino acids deleted (amino acids 20-119 of SEQ ID NO:16) | GRGEAETRECIYYNANWELERTNQSGLERCEGEQDKRLHCYASWANSSG TIELVKKGCWLDDFNCYDRQECVATEENPQVYFCCCEGNFCNERFTHLPE A |
| 28 | human ActRIIB precursor protein sequence (R64) | MTAPWVALALLWGSLW PGSGRGEAETRECIYY NANWELERTNQSGLER CEGEQDKRLHCYASWR NSSGTIELVKKGCWLD DFNCYDRQECVATEEN PQVYFCCCEGNFCNER FTHLPEAGGPEVTYEP PPTAPTLLTVLAYSLL PIGGLSLIVLLAFWMY RHRKPPYGHVDIHEDP GPPPPSPLVGLKPLQL LEIKARGRFGCVWKAQ LMNDFVAVKIFPLQDK QSWQSEREIFSTPGMK HENLLQFIAAEKRGSN LEVELWLITAFHDKGS LTDYLKGNIITWNELC HVAETMSRGLSYLHED VPWCRGEGHKPSIAHR DFKSKNVLLKSDLTAV LADFGLAVRFEPGKPP GDTHGQVGTRRYMAPE VLEGAINFQRDAFLRI DMYAMGLVLWELVSRC KAADGPVDEYMLPFEE EIGQHPSLEELQEVVV HKKMRPTIKDHWLKHP GLAQLCVTIEECWDHD AEARLSAGCVEERVSL IRRSVNGTTSDCLVSL VTSVTNVDLPPKESSI |
| 29 | human ActRIIB soluble (extracellular) , processed polypeptide sequence (amino acids 19-134 of SEQ ID NO:28) | SGRGEAETRECIYYNANWELERTNQSGLERCEGEQDKRLHCYASWRNSSG TIELVKKGCWLDDFNCYDRQECVATEENPQVYFCCCEGNFCNERFTHLPE AGGPEVTYEPPPTAPT |
| 30 | human ActRIIB soluble (extracellular), processed polypeptide sequence with the C-terminal 15 amino acids deleted (amino acids 19-119 of SEQ ID NO:28) | SGRGEAETRECIYYNANWELERTNQSGLERCEGEQDKRLHCYASWRNSSG TIELVKKGCWLDDFNCYDRQECVATEENPQVYFCCCEGNFCNERFTHLPE A |
| 31 | human ActRIIB soluble (extracellular), processed polypeptide sequence (amino acids 20-134 of SEQ ID NO:28) | GRGEAETRECIYYNANWELERTNQSGLERCEGEQDKRLHCYASWRNSSG TIELVKKGCWLDDFNCYDRQECVATEENPQVYFCCCEGNFCNERFTHLPE AGGPEVTYEPPPTAPT |
| 32 | human ActRIIB soluble (extracellular) , processed polypeptide sequence with the C-terminal 15 amino acids deleted (amino acids 20-119 of SEQ ID NO:28) | GRGEAETRECIYYNANWELERTNQSGLERCEGEQDKRLHCYASWRNSSG TIELVKKGCWLDDFNCYDRQECVATEENPQVYFCCCEGNFCNERFTHLPE A |
| 33 | human ActRIIB soluble (extracellular) , processed polypeptide sequence with the N-terminal 6 amino acids of the EC domain deleted and the C-terminal 3 amino acids of the EC domain deleted (amino acids 25-131 of SEQ ID NO:16) and with an L79D mutation | ETRECIYYNANWELERTNQSGLERCEGEQDKRLHCYASWANSSGTIELV KKGCWDDDFNCYDRQECVATEENPQVYFCCCEGNFCNERFTHLPEAGGP EVTYEPPPT |
| 34 | Unprocessed ActRIIB-Fc fusion protein with the N-terminal 6 amino acids of the EC domain deleted and the C-terminal 3 amino acids of the EC domain deleted (amino acids 25-131 of SEQ ID NO:16) and with an L79D mutation and with TPA leader sequence | MDAMKRGLCCVLLLCGAVFVSPGAAETRECIYYNANWELERTNQSGLERC EGEQDKRLHCYASWANSSGTIELVKKGCWDDDFNCYDRQECVATEENPQV YFCCCEGNFCNERFTHLPEAGGPEVTYEPPPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT |
| | | QKSLSLSPGK* |
| 35 | Processed ActRIIB-Fc fusion protein with the N-terminal 6 amino acids of the EC domain deleted and the C-terminal 3 amino acids of the EC domain deleted (amino acids 25-131 of SEQ ID NO:16) and with an L79D mutation | ETRECIYYNANWELERTNQSGLERCEGEQDKRLHCYASWANSSGTIELVK KGCWDDDFNCYDRQECVATEENPQVYFCCCEGNFCNERFTHLPEAGGPEV TYEPPPTGGGTHTCPPCPAPELLGGPSVFLFPPKPKDTLMISRTPEVTCV VVDVSHEDPEVKFNWYVDGVEVHNAKTKPREEQYNSTYRVVSVLTVLHQD WLNGKEYKCKVSNKALPAPIEKTISKAKGQPREPQVYTLPPSREEMTKNQ VSLTCLVKGFYPSDIAVEWESNGQPENNYKTTPPVLDSDGSFFLYSKLTV DKSRWQQGNVFSCSVMHEALHNHYTQKSLSLSPGK* |
| 36 | human ActRIIB soluble (extracellular) , processed polypeptide sequence (amino acids 20-134 of SEQ ID NO:28) with L79D mutation | GRGEAETRECIYYNANWELERTNQSGLERCEGEQDKRLHCYASWRNSSG TIELVKKGCWDDDFNCYDRQECVATEENPQVYFCCCEGNFCNERFTHLPE AGGPEVTYEPPPTAPT |
| 37 | human ActRIIB soluble (extracellular) , processed polypeptide sequence (amino acids 20-134 of SEQ ID NO:16) with L79D mutation | GRGEAETRECIYYNANWELERTNQSGLERCEGEQDKRLHCYASWANSSG TIELVKKGCWDDDFNCYDRQECVATEENPQVYFCCCEGNFCNERFTHLPE AGGPEVTYEPPPTAPT |
| 38 | human ActRIIB soluble (extracellular) , processed polypeptide sequence (amino acids 20-134 of SEQ ID NO:28) with L79D mutation fused to an Fc domain with a GGG linker | GRGEAETRECIYYNANWELERTNQSGLERCEGEQDKRLHCYASWRNSSG TIELVKKGCWDDDFNCYDRQECVATEENPQVYFCCCEGNFCNERFTHLPE AGGPEVTYEPPPTAPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS |
| | | KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 39 | human ActRIIB soluble (extracellular) , processed polypeptide sequence (amino acids 20-134 of SEQ ID NO:16) with L79D mutation fused to an Fc domain | GRGEAETRECIYYNANWELERTNQSGLERCEGEQDKRLHCYASWANSSG TIELVKKGCWDDDFNCYDRQECVATEENPQVYFCCCEGNFCNERFTHLPE AGGPEVTYEPPPTAPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 40 | human ActRIIB soluble (extracellular) , processed polypeptide sequence (amino acids 20-134 of SEQ ID NO:28) with L79D mutation fused to an Fc domain and with TPA leader sequence | MDAMKRGLCCVLLLCGAVFVSPGASGRGEAETRECIYYNANWELERTNQSG LERCEGEQDKRLHCYASWRNSSG TIELVKKGCWDDDFNCYDRQECVATEENPQVYFCCCEGNFCNERFTHLPE AGGPEVTYEPPPTAPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 41 | human ActRIIB soluble (extracellular), processed polypeptide sequence (amino acids 20-134 of SEQ ID NO:16) with L79D mutation fused to an Fc domain and with TPA leader sequence | MDAMKRGLCCVLLLCGAVFVSPGASGRGEAETRECIYYNANWELERTNQSG LERCEGEQDKRLHCYASWANSSG TIELVKKGCWDDDFNCYDRQECVATEENPQVYFCCCEGNFCNERFTHLPE AGGPEVTYEPPPTAPTGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 42 | human ActRIIB soluble (extracellular) , processed polypeptide sequence having a variant C-terminal sequence (disclosed in WO2007/053775) | GRGEAETRECIYYNANWELERTNQSGLERCEGEQDKRLHCYASWRNSSGT IELVKKGCWLDDFNCYDRQECVATEENPQVYFCCCEGNFCNERFTHLPEA GGPEGPWASTTIPSGGPEATAAAGDQGSGALWLCLEGPAHE |
| 43 | human ActRIIB soluble (extracellular), processed polypeptide sequence having a variant C-terminal sequence (disclosed in WO2007/053775) having an L79D mutation | GRGEAETRECIYYNANWELERTNQSGLERCEGEQDKRLHCYASWRNSSGT IELVKKGCWDDDFNCYDRQECVATEENPQVYFCCCEGNFCNERFTHLPEA GGPEGPWASTTIPSGGPEATAAAGDQGSGALWLCLEGPAHE |
| 44 | human ActRIIB soluble (extracellular) , processed polypeptide sequence having a variant C-terminal sequence (disclosed in WO2007/053775) having an L79D mutation fused to an Fc domain with a TGGG linker | GRGEAETRECIYYNANWELERTNQSGLERCEGEQDKRLHCYASWRNSSGT IELVKKGCWDDDFNCYDRQECVATEENPQVYFCCCEGNFCNERFTHLPEA GGPEGPWASTTIPSGGPEATAAAGDQGSGALWLCLEGPAHE TGGGTHTCPPCPAPELLGG PSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHYT QKSLSLSPGK* |
| 45 | Nucleic Acid Sequence Encoding SEQ ID NO:24 | ATGGATGCAA TGAAGAGAGG GCTCTGCTGT GTGCTGCTGC TGTGTGGAGC AGTCTTCGTT TCGCCCGGCG CCGCCGAAAC CCGCGAATGT ATTTATTACA ATGCTAATTG GGAACTCGAA CGGACGAACC AATCCGGGCT CGAACGGTGT GAGGGGGAAC AGGATAAACG CCTCCATTGC TATGCGTCGT GGAGGAACTC CTCCGGGACG ATTGAACTGG TCAAGAAAGG GTGCTGGGAC GACGATTTCA ATTGTTATGA CCGCCAGGAA TGTGTCGCGA CCGAAGAGAA TCCGCAGGTC TATTTCTGTT GTTGCGAGGG GAATTTCTGT AATGAACGGT TTACCCACCT CCCCGAAGCC GGCGGGCCCG AGGTGACCTA TGAACCCCCG CCCACCGGTG GTGGAACTCA CACATGCCCA CCGTGCCCAG CACCTGAACT CCTGGGGGGA CCGTCAGTCT TCCTCTTCCC CCCAAAACCC |
| | | AAGGACACCC TCATGATCTC CCGGACCCCT GAGGTCACAT GCGTGGTGGT GGACGTGAGC CACGAAGACC CTGAGGTCAA GTTCAACTGG TACGTGGACG GCGTGGAGGT GCATAATGCC AAGACAAAGC CGCGGGAGGA GCAGTACAAC AGCACGTACC GTGTGGTCAG CGTCCTCACC GTCCTGCACC AGGACTGGCT GAATGGCAAG GAGTACAAGT GCAAGGTCTC CAACAAAGCC CTCCCAGCCC CCATCGAGAA AACCATCTCC AAAGCCAAAG GGCAGCCCCG AGAACCACAG GTGTACACCC TGCCCCCATC CCGGGAGGAG ATGACCAAGA ACCAGGTCAG CCTGACCTGC CTGGTCAAAG GCTTCTATCC CAGCGACATC GCCGTGGAGT GGGAGAGCAA TGGGCAGCCG GAGAACAACT ACAAGACCAC GCCTCCCGTG CTGGACTCCG ACGGCTCCTT CTTCCTCTAT AGCAAGCTCA CCGTGGACAA GAGCAGGTGG CAGCAGGGGA ACGTCTTCTC ATGCTCCGTG ATGCATGAGG CTCTGCACAA CCACTACACG CAGAAGAGCC TCTCCCTGTC CCCGGGTAAA TGA |
| 46 | fusion protein comprising a soluble extracellular domain of ActRIIB (R64; SEQ ID NO:29) fused to an Fc domain | SGRGEAETRECIYYNANWELERTNQSGLERCEGEQDKRLHCYASWRNSSG TIELVKKGCWLDDFNCYDRQECVATEENPQVYFCCCEGNFCNERFTHLPE AGGPEVTYEPPPTAPTGGGTHTCPPCPAPELLGGPSVFLFPPKPKDTLMI SRTPEVTCVVVDVSHEDPEVKFNWYVDGVEVHNAKTKPREEQYNSTYRVV SVLTVLHQDWLNGKEYKCKVSNKALPVPIEKTISKAKGQPREPQVYTLPP SREEMTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 47 | fusion protein comprising a soluble extracellular domain of ActRIIB (R64) with the C-terminal 15 amino acids deleted (SEQ ID NO:30) fused to an Fc domain | SGRGEAETRECIYYNANWELERTNQSGLERCEGEQDKRLHCYASWRNSSG TIELVKKGCWLDDFNCYDRQECVATEENPQVYFCCCEGNFCNERFTHLPE AGGGTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKE YKCKVSNKALPVPIEKTISKAKGQPREPQVYTLPPSREEMTKNQVSLTCL VKGFYPSDIAVEWESNGQPENNYKTTPPVLDSDGSFFLYSKLTVDKSRWQ QGNVFSCSVMHEALHNHYTQKSLSLSPGK |
| 48 | full-length, unprocessed precursor protein GDF11, i.e., GDF11 preproprotein | MVLAAPLLLGFLLLALELRPRGEAAEGPAAAAAAAAA AAAAGVGGERSSRPAPSVAPEPDGCPVCVWRQHSREL RLESIKSQILSKLRLKEAPNISREVVKQLLPKAPPLQQIL DLHDFQGDALQPEDFLEEDEYHATTETVISMAQETDPA VQTDGSPLCCHFHFSPKVMFTKVLKAQLWVYLRPVPR PATVYLQILRLKPLTGEGTAGGGGGGRRHIRIRSLKIEL HSRSGHWQSIDFKQVLHSWFRQPQSNWGIEINAFDPSG TDLAVTSLGPGAEGLHPFMELRVLENTKRSRRNLGLDC DEHSSESRCCRYPLTVDFEAFGWDWIIAPKRYKANYCS GQCEYMFMQKYPHTHLVQQANPRGSAGPCCTPTKMSP INMLYFNDKQQIIYGKIPGMVVDRCGCS |
| 49 | Nucleic acid sequence encoding SEQ ID NO: 48 | ATGGTGCTCGCGGCCCCGCTGCTGCTGGGCTTCCTGC TCCTCGCCCTGGAGCTGCGGCCCCGGGGGGAGGCGG CCGAGGGCCCCGCGGCGGCGGCGGCGGCGGCGGCG GCGGCGGCAGCGGCGGGGTCGGGGGGAGCGCTC CAGCCGGCCAGCCCCGTCCGTGGCGCCCGAGCCGGA CGGCTGCCCCGTGTGCGTTTGGCGGCAGCACAGCCG CGAGCTGCGCCTAGAGAGCATCAAGTCGCAGATCTT GAGCAAACTGCGGCTCAAGGAGGCGCCCAACATCAG CCGCGAGGTGGTGAAGCAGCTGCTGCCCAAGGCGCC GCCGCTGCAGCAGATCCTGGACCTACACGACTTCCA GGGCGACGCGCTGCAGCCCGAGGACTTCCTGGAGGA GGACGAGTACCACGCCACCACCGAGACCGTCATTAG CATGGCCCAGGAGACGGACCCAGCAGTACAGACAGA TGGCAGCCCTCTCTGCTGCCATTTTCACTTCAGCCCC AAGGTGATGTTCACAAAGGTACTGAAGGCCCAGCTG TGGGTGTACCTACGGCCTGTACCCCGCCCAGCCACA GTCTACCTGCAGATCTTGCGACTAAAACCCCTAACTG GGGAAGGGACCGCAGGGGGAGGGGGCGGAGGCCGG CGTCACATCCGTATCCGCTCACTGAAGATTGAGCTGC ACTCACGCTCAGGCCATTGGCAGAGCATCGACTTCA AGCAAGTGCTACACAGCTGGTTCCGCCAGCCACAGA GCAACTGGGGCATCGAGATCAACGCCTTTGATCCCA GTGGCACAGACCTGGCTGTCACCTCCCTGGGGCCGG GAGCCGAGGGGCTGCATCCATTCATGGAGCTTCGAG TCCTAGAGAACACAAAACGTTCCCGGCGGAACCTGG GTCTGGACTGCGACGAGCACTCAAGCGAGTCCCGCT GCTGCCGATATCCCCTCACAGTGGACTTTGAGGCTTT CGGCTGGGACTGGATCATCGCACCTAAGCGCTACAA GGCCAACTACTGCTCCGGCCAGTGCGAGTACATGTTC ATGCAAAAATATCCGCATACCCATTTGGTGCAGCAG GCCAATCCAAGAGGCTCTGCTGGGCCCTGTTGTACCC CCACCAAGATGTCCCCAATCAACATGCTCTACTTCAA TGACAAGCAGCAGATTATCTACGGCAAGATCCCTGG CATGGTGGTGGATCGCTGTGGCTGCTCT |
| 50 | GDF11 propeptide of human GDF11 protein | AEGPAAAAAAAAAAAAAGVGGERSSRPAPSVAPEPDG CPVCVWRQHSRELRLESIKSQILSKLRLKEAPNISREVV KQLLPKAPPLQQILDLHDFQGDALQPEDFLEEDEYHAT TETVISMAQETDPAVQTDGSPLCCHFHFSPKVMFTKVL KAQLWVYLRPVPRPATVYLQILRLKPLTGEGTAGGGG GGRRHIRIRSLKIELHSRSGHWQSIDFKQVLHSWFRQPQ SNWGIEINAFDPSGTDLAVTSLGPGAEGLHPFMELRVLE |
| | | NTKRSRR |
| 51 | Nucleic acid sequence encoding SEQ ID NO: 50 | GCCGAGGGCCCCGCGGCGGCGGCGGCGGCGGCGGC GGCGGCGGCAGCGGCGGGGGTCGGGGGGAGCGCT CCAGCCGGCCAGCCCCGTCCGTGGCGCCCGAGCCGG ACGGCTGCCCCGTGTGCGTTTGGCGGCAGCACAGCC GCGAGCTGCGCCTAGAGAGCATCAAGTCGCAGATCT TGAGCAAACTGCGGCTCAAGGAGGCGCCCAACATCA GCCGCGAGGTGGTGAAGCAGCTGCTGCCCAAGGCGC CGCCGCTGCAGCAGATCCTGGACCTACACGACTTCC AGGGCGACGCGCTGCAGCCCGAGGACTTCCTGGAGG AGGACGAGTACCACGCCACCACCGAGACCGTCATTA GCATGGCCCAGGAGACGGACCCAGCAGTACAGACAG ATGGCAGCCCTCTCTGCTGCCATTTTCACTTCAGCCC CAAGGTGATGTTCACAAAGGTACTGAAGGCCCAGCT GTGGGTGTACCTACGGCCTGTACCCCGCCCAGCCAC AGTCTACCTGCAGATCTTGCGACTAAAACCCCTAACT GGGGAAGGGACCGCAGGGGGAGGGGGCGGAGGCCG GCGTCACATCCGTATCCGCTCACTGAAGATTGAGCTG CACTCACGCTCAGGCCATTGGCAGAGCATCGACTTC AAGCAAGTGCTACACAGCTGGTTCCGCCAGCCACAG AGCAACTGGGGCATCGAGATCAACGCCTTTGATCCC AGTGGCACAGACCTGGCTGTCACCTCCCTGGGGCCG GGAGCCGAGGGGCTGCATCCATTCATGGAGCTTCGA GTCCTAGAGAACACAAAACGTTCCCGGCGG |
| 52 | Mature human GDF11 protein | NLGLDCDEHSSESRCCRYPLTVDFEAFGWDWIIAPKRY KANYCSGQCEYMFMQKYPHTHLVQQANPRGSAGPCC TPTKMSPINMLYFNDKQQIIYGKIPGMVVDRCGCS |
| 53 | Nucleic acid sequence encoding SEQ ID NO: 52 | AACCTGGGTCTGGACTGCGACGAGCACTCAAGCGAG TCCCGCTGCTGCCGATATCCCCTCACAGTGGACTTTG AGGCTTTCGGCTGGGACTGGATCATCGCACCTAAGC GCTACAAGGCCAACTACTGCTCCGGCCAGTGCGAGT ACATGTTCATGCAAAAATATCCGCATACCCATTTGGT GCAGCAGGCCAATCCAAGAGGCTCTGCTGGGCCCTG TTGTACCCCCACCAAGATGTCCCCAATCAACATGCTC TACTTCAATGACAAGCAGCAGATTATCTACGGCAAG ATCCCTGGCATGGTGGTGGATCGCTGTGGCTGCTCT |
| 54 | Extracellular domain of murine ActRIIA fused to a murine Fc domain ("mActRIIA-Fc") | Murine counterpart of SEQ ID NO: 7 |

## Claims

1. An activin receptor type II inhibitor for use in a method for treatment of anemia, beta thalassemia, or for increasing red blood cell levels or orthochromatic erythroblasts (Ery-C) in a subject in need thereof, wherein the method comprises:
(a) assessing the level and/or activity of GDF11 in a tissue sample of the subject, and
(b) if the level and/or activity of GDF11 is elevated over the normal level and/or activity of GDF11, administering an activin receptor type II inhibitor to the subject,
wherein the activin receptor type II inhibitor is a polypeptide comprising an amino acid sequence selected from the group consisting of:
| | |
|---|---|
| a | 90% identical to SEQ ID NO:2; |
| b | 95% identical to SEQ ID NO:2; |
| c | 98% identical to SEQ ID NO:2; |
| d | SEQ ID NO:2; |
| e | 90% identical to SEQ ID NO:3; |
| f | 95% identical to SEQ ID NO:3; |
| g | 98% identical to SEQ ID NO:3; |
| h | SEQ ID NO:3; |
| i | 90% identical to SEQ ID NO:6; |
| j | 95% identical to SEQ ID NO:6; |
| k | 98% identical to SEQ ID NO:6; |
| l | SEQ ID NO:6; |
| m | 90% identical to SEQ ID NO:7; |
| n | 95% identical to SEQ ID NO:7; |
| o | 98% identical to SEQ ID NO:7; |
| p | SEQ ID NO:7; |
| q | 90% identical to SEQ ID NO:12; |
| r | 95% identical to SEQ ID NO:12; |
| s | 98% identical to SEQ ID NO:12; |
| t | SEQ ID NO:12; |
| u | 90% identical to SEQ ID NO:17; |
| v | 95% identical to SEQ ID NO:17; |
| w | 98% identical to SEQ ID NO:17; |
| x | SEQ ID NO:17; |
| y | 90% identical to SEQ ID NO:20; |
| z | 95% identical to SEQ ID NO:20; |
| aa | 98% identical to SEQ ID NO:20; |
| bb | SEQ ID NO:20; |
| cc | 90% identical to SEQ ID NO:21; |
| dd | 95% identical to SEQ ID NO:21; |
| ee | 98% identical to SEQ ID NO:21; |
| ff | SEQ ID NO:21 |
| gg | 90% identical to SEQ ID NO:22; |
| hh | 95% identical to SEQ ID NO:22; |
| ii | 98% identical to SEQ ID NO:22; |
| jj | SEQ ID NO:22; |
| kk | 90% identical to SEQ ID NO:23; |
| ll | 95% identical to SEQ ID NO:23; |
| mm | 98% identical to SEQ ID NO:23; |
| nn | SEQ ID NO:23; |
| oo | 90% identical to SEQ ID NO:24; |
| pp | 95% identical to SEQ ID NO:24; |
| qq | 98% identical to SEQ ID NO:24; |
| rr | SEQ ID NO:24; |
| ss | 90% identical to SEQ ID NO:25; |
| tt | 95% identical to SEQ ID NO:25; |
| uu | 98% identical to SEQ ID NO:25; and |
| vv | SEQ ID NO:25; |
or
wherein the activin receptor type II inhibitor is a polypeptide comprising the extracellular domain of activin receptor type IIA linked to the Fc portion of an antibody;
or
wherein the activin receptor type II inhibitor is a polypeptide comprising the extracellular domain of activin receptor type IIB linked to the Fc portion of an antibody.

2. An activin receptor type II inhibitor for use in a method for treatment of anemia, beta thalassemia, or for increasing red blood cell levels or orthochromatic erythroblasts (Ery-C) in a subject in need thereof, wherein the method comprises:
(a) administering an activin receptor type II inhibitor, and
(b) monitoring the level and/or activity of GDF11 in a tissue sample of the subject,
wherein the activin receptor type II inhibitor is a polypeptide comprising an amino acid sequence selected from the group consisting of:
| | |
|---|---|
| a | 90% identical to SEQ ID NO:2; |
| b | 95% identical to SEQ ID NO:2; |
| c | 98% identical to SEQ ID NO:2; |
| d | SEQ ID NO:2; |
| e | 90% identical to SEQ ID NO:3; |
| f | 95% identical to SEQ ID NO:3; |
| g | 98% identical to SEQ ID NO:3; |
| h | SEQ ID NO:3; |
| i | 90% identical to SEQ ID NO:6; |
| j | 95% identical to SEQ ID NO:6; |
| k | 98% identical to SEQ ID NO:6; |
| l | SEQ ID NO:6; |
| m | 90% identical to SEQ ID NO:7; |
| n | 95% identical to SEQ ID NO:7; |
| o | 98% identical to SEQ ID NO:7; |
| p | SEQ ID NO:7; |
| q | 90% identical to SEQ ID NO:12; |
| r | 95% identical to SEQ ID NO:12; |
| s | 98% identical to SEQ ID NO:12; |
| t | SEQ ID NO:12; |
| u | 90% identical to SEQ ID NO:17; |
| v | 95% identical to SEQ ID NO:17; |
| w | 98% identical to SEQ ID NO:17; |
| x | SEQ ID NO:17; |
| y | 90% identical to SEQ ID NO:20; |
| z | 95% identical to SEQ ID NO:20; |
| aa | 98% identical to SEQ ID NO:20; |
| bb | SEQ ID NO:20; |
| cc | 90% identical to SEQ ID NO:21; |
| dd | 95% identical to SEQ ID NO:21; |
| ee | 98% identical to SEQ ID NO:21; |
| ff | SEQ ID NO:21 |
| gg | 90% identical to SEQ ID NO:22; |
| hh | 95% identical to SEQ ID NO:22; |
| ii | 98% identical to SEQ ID NO:22; |
| jj | SEQ ID NO:22; |
| kk | 90% identical to SEQ ID NO:23; |
| ll | 95% identical to SEQ ID NO:23; |
| mm | 98% identical to SEQ ID NO:23; |
| nn | SEQ ID NO:23; |
| oo | 90% identical to SEQ ID NO:24; |
| pp | 95% identical to SEQ ID NO:24; |
| qq | 98% identical to SEQ ID NO:24; |
| rr | SEQ ID NO:24; |
| ss | 90% identical to SEQ ID NO:25; |
| tt | 95% identical to SEQ ID NO:25; |
| uu | 98% identical to SEQ ID NO:25; and |
| vv | SEQ ID NO:25; |
or
wherein the activin receptor type II inhibitor is a polypeptide comprising the extracellular domain of activin receptor type IIA linked to the Fc portion of an antibody;
or
wherein the activin receptor type II inhibitor is a polypeptide comprising the extracellular domain of activin receptor type IIB linked to the Fc portion of an antibody.

3. The activin receptor type II inhibitor for use of claim 2, wherein the method comprises:
(a) administering a dose amount of an activin receptor type II inhibitor,
(b) assessing the level and/or activity of GDF11 in a tissue sample of the subject, and
(c) adjusting the dose amount of the activin receptor type II inhibitor,
wherein, if the level and/or activity of GDF11 is elevated over the normal level and/or activity of GDF11, the dose amount of the activin receptor type II inhibitor is increased, and
wherein, if the level and/or activity of GDF11 is decreased over the normal level and/or activity of GDF11, the dose amount of the activin receptor type II inhibitor is decreased.

4. The activin receptor type II inhibitor for use of any one of claims 1-3, wherein said administering results in a decreased cell count of late basophilic and polychromatic erythroblasts in the patient.

5. The activin receptor type II inhibitor for use of any one of claims 1-4, wherein the level and/or activity of GDF11 is the protein level of GDF11; or wherein the level and/or activity of GDF11 is mRNA level of GDF11.

6. The activin receptor type II inhibitor for use of any one of claims 1-5, wherein the level and/or activity of GDF11 is elevated over the normal level and/or activity of GDF11 when it is elevated at least 25%, at least 50%, at least 75%, at least 100% or at least 200% over the normal level and/or activity of GDF11.

7. The activin receptor type II inhibitor for use of any one of claims 1-5, wherein the level and/or activity of GDF11 is elevated over the normal level and/or activity of GDF11 when it is elevated at least 2 times over the normal level and/or activity of GDF11.

8. The activin receptor type II inhibitor for use of any one of claims 3-5, wherein the level and/or activity of GDF11 is decreased over the normal level and/or activity of GDF11 when it is decreased at least 25%, at least 50%, at least 75%, or at least 90% over the normal level and/or activity of GDF11.

9. The activin receptor type II inhibitor for use of any one of claims 3-5, wherein the level and/or activity of GDF11 is decreased over the normal level and/or activity of GDF11 when it is decreased at least 2 times over the normal level and/or activity of GDF11.

10. The activin receptor type II inhibitor for use of any one of claims 1-9, wherein the normal level and/or activity of GDF11 is an average level and/or activity of GDF11 in a sample from the same tissue of one or more healthy subjects in the same age category.

11. The activin receptor type II inhibitor for use of any one of claims 1-10, wherein the tissue is serum, bone marrow, liver, or spleen.

12. The activin receptor type II inhibitor for use of any one of claims 1-11, wherein the subject is human.

13. The activin receptor type II inhibitor for use of any one of claims 1-12, wherein the Fc portion of an antibody is Fc portion of an IgG1 antibody.

## Patentansprüche

1. Inhibitor des Aktivin-Rezeptortyps II zur Verwendung in einem Verfahren zur Behandlung von Anämie, Beta Thalassämie oder zur Erhöhung der Anzahl an roten Blutkörperchen oder orthochromatischen Erythroblasten (Ery-C) in einem behandlungsbedürftigen Subjekt, wobei das Verfahren Folgendes umfasst:
(a) Bewerten des Niveaus und/oder der Aktivität von GDF11 in einer Gewebeprobe des Subjekts und,
(b) wenn das Niveau und/oder die Aktivität von GDF11 über das normale Niveau und/oder die normale Aktivität von GDF11 erhöht sind/ist, Verabreichen eines Inhibitors des Aktivin-Rezeptortyps II,
wobei der Inhibitor des Aktivin-Rezeptortyps II ein Polypeptid ist, das eine Aminosäuresequenz umfasst, die aus der Gruppe ausgewählt ist, die aus Folgendem besteht:
| | |
|---|---|
| a | 90 % identisch mit SEQ ID Nr.: 2; |
| b | 95 % identisch mit SEQ ID Nr.: 2; |
| c | 98 % identisch mit SEQ ID Nr.: 2; |
| d | SEQ ID Nr.: 2; |
| e | 90 % identisch mit SEQ ID Nr.: 3; |
| f | 95 % identisch mit SEQ ID Nr.: 3; |
| g | 98 % identisch mit SEQ ID Nr.: 3; |
| h | SEQ ID Nr.: 3; |
| i | 90 % identisch mit SEQ ID Nr.: 6; |
| j | 95 % identisch mit SEQ ID Nr.: 6; |
| k | 98 % identisch mit SEQ ID Nr.: 6; |
| l | SEQ ID Nr.: 6; |
| m | 90 % identisch mit SEQ ID Nr.: 7; |
| n | 95 % identisch mit SEQ ID Nr.: 7; |
| o | 98 % identisch mit SEQ ID Nr.: 7; |
| p | SEQ ID Nr.: 7; |
| q | 90 % identisch mit SEQ ID Nr.: 12; |
| r | 95 % identisch mit SEQ ID Nr.: 12; |
| s | 98 % identisch mit SEQ ID Nr.: 12; |
| t | SEQ ID Nr.: 12; |
| u | 90 % identisch mit SEQ ID Nr.: 17; |
| v | 95 % identisch mit SEQ ID Nr.: 17; |
| w | 98 % identisch mit SEQ ID Nr.: 17; |
| x | SEQ ID Nr.: 17; |
| y | 90 % identisch mit SEQ ID Nr.: 20; |
| z | 95 % identisch mit SEQ ID Nr.: 20; |
| aa | 98 % identisch mit SEQ ID Nr.: 20; |
| bb | SEQ ID Nr.: 20; |
| cc | 90 % identisch mit SEQ ID Nr.: 21; |
| dd | 95 % identisch mit SEQ ID Nr.: 21; |
| ee | 98 % identisch mit SEQ ID Nr.: 21; |
| ff | SEQ ID Nr.: 21; |
| gg | 90 % identisch mit SEQ ID Nr.: 22; |
| hh | 95 % identisch mit SEQ ID Nr.: 22; |
| ii | 98 % identisch mit SEQ ID Nr.: 22; |
| jj | SEQ ID Nr.: 22; |
| kk | 90 % identisch mit SEQ ID Nr.: 23; |
| ll | 95 % identisch mit SEQ ID Nr.: 23; |
| mm | 98 % identisch mit SEQ ID Nr.: 23; |
| nn | SEQ ID Nr.: 23; |
| oo | 90 % identisch mit SEQ ID Nr.: 24; |
| pp | 95 % identisch mit SEQ ID Nr.: 24; |
| qq | 98 % identisch mit SEQ ID Nr.: 24; |
| rr | SEQ ID Nr.: 24; |
| ss | 90 % identisch mit SEQ ID Nr.: 25; |
| tt | 95 % identisch mit SEQ ID Nr.: 25; |
| uu | 98 % identisch mit SEQ ID Nr.: 25; und |
| vv | SEQ ID Nr.: 25; |
oder
wobei der Inhibitor des Aktivin-Rezeptortyps II ein Polypeptid ist, das die extrazelluläre Domäne eines Aktivin-Rezeptortyps IIA umfasst, die mit dem Fc-Abschnitt eines Antikörpers vernetzt ist;
oder
wobei der Inhibitor des Aktivin-Rezeptortyps II ein Polypeptid ist, das die extrazelluläre Domäne eines Aktivin-Rezeptortyps IIB umfasst, die mit dem Fc-Abschnitt eines Antikörpers vernetzt ist.

2. Inhibitor des Aktivin-Rezeptortyps II zur Verwendung bei einem Verfahren zur Behandlung von Anämie, Beta Thalassämie oder zur Erhöhung der Anzahl an roten Blutkörperchen oder orthochromatischen Erythroblasten (Ery-C) in einem behandlungsbedürftigen Subjekt, wobei das Verfahren Folgendes umfasst:
(a) Verabreichen eines Inhibitors des Aktivin-Rezeptortyps II und
(b) Überwachen des Niveaus und/oder der Aktivität von GDF11 in einer Gewebeprobe des Subjekts,
wobei der Inhibitor des Aktivin-Rezeptortyps II ein Polypeptid ist, das eine Aminosäuresequenz umfasst, die aus der Gruppe ausgewählt ist, die aus Folgendem besteht:
| | |
|---|---|
| a | 90 % identisch mit SEQ ID Nr.: 2; |
| b | 95 % identisch mit SEQ ID Nr.: 2; |
| c | 98 % identisch mit SEQ ID Nr.: 2; |
| d | SEQ ID Nr.: 2; |
| e | 90 % identisch mit SEQ ID Nr.: 3; |
| f | 95 % identisch mit SEQ ID Nr.: 3; |
| g | 98 % identisch mit SEQ ID Nr.: 3; |
| h | SEQ ID Nr.: 3; |
| i | 90 % identisch mit SEQ ID Nr.: 6; |
| j | 95 % identisch mit SEQ ID Nr.: 6; |
| k | 98 % identisch mit SEQ ID Nr.: 6; |
| l | SEQ ID Nr.: 6; |
| m | 90 % identisch mit SEQ ID Nr.: 7; |
| n | 95 % identisch mit SEQ ID Nr.: 7; |
| o | 98 % identisch mit SEQ ID Nr.: 7; |
| p | SEQ ID Nr.: 7; |
| q | 90 % identisch mit SEQ ID Nr.: 12; |
| r | 95 % identisch mit SEQ ID Nr.: 12; |
| s | 98 % identisch mit SEQ ID Nr.: 12; |
| t | SEQ ID Nr.: 12; |
| u | 90 % identisch mit SEQ ID Nr.: 17; |
| v | 95 % identisch mit SEQ ID Nr.: 17; |
| w | 98 % identisch mit SEQ ID Nr.: 17; |
| x | SEQ ID Nr.: 17; |
| y | 90 % identisch mit SEQ ID Nr.: 20; |
| z | 95 % identisch mit SEQ ID Nr.: 20; |
| aa | 98 % identisch mit SEQ ID Nr.: 20; |
| bb | SEQ ID Nr.: 20; |
| cc | 90 % identisch mit SEQ ID Nr.: 21; |
| dd | 95 % identisch mit SEQ ID Nr.: 21; |
| ee | 98 % identisch mit SEQ ID Nr.: 21; |
| ff | SEQ ID Nr.: 21; |
| gg | 90 % identisch mit SEQ ID Nr.: 22; |
| hh | 95 % identisch mit SEQ ID Nr.: 22; |
| ii | 98 % identisch mit SEQ ID Nr.: 22; |
| jj | SEQ ID Nr.: 22; |
| kk | 90 % identisch mit SEQ ID Nr.: 23; |
| ll | 95 % identisch mit SEQ ID Nr.: 23; |
| mm | 98 % identisch mit SEQ ID Nr.: 23; |
| nn | SEQ ID Nr.: 23; |
| oo | 90 % identisch mit SEQ ID Nr.: 24; |
| pp | 95 % identisch mit SEQ ID Nr.: 24; |
| qq | 98 % identisch mit SEQ ID Nr.: 24; |
| rr | SEQ ID Nr.: 24; |
| ss | 90 % identisch mit SEQ ID Nr.: 25; |
| tt | 95 % identisch mit SEQ ID Nr.: 25; |
| uu | 98 % identisch mit SEQ ID Nr.: 25; und |
| vv | SEQ ID Nr.: 25; |
oder
wobei der Inhibitor des Aktivin-Rezeptortyps II ein Polypeptid ist, das die extrazelluläre Domäne eines Aktivin-Rezeptortyps IIA umfasst, die mit dem Fc-Abschnitt eines Antikörpers vernetzt ist;
oder
wobei der Inhibitor des Aktivin-Rezeptortyps II ein Polypeptid ist, das die extrazelluläre Domäne eines Aktivin-Rezeptortyps IIB umfasst, die mit dem Fc-Abschnitt eines Antikörpers vernetzt ist.

3. Inhibitor des Aktivin-Rezeptortyps II zur Verwendung nach Anspruch 2, wobei das Verfahren Folgendes umfasst:
(a) Verabreichen einer Dosismenge eines Inhibitors des Aktivin-Rezeptortyps II,
(b) Bewerten des Niveaus und/oder der Aktivität von GDF 11 in einer Gewebeprobe des Subjekts und
(c) Anpassen der Dosismenge des Inhibitors des Aktivin-Rezeptortyps II, wobei die Dosismenge des Inhibitors des Aktivin-Rezeptortyps II erhöht wird, wenn das Niveau und/oder die Aktivität von GDF11 über das normale Niveau und/oder die normale Aktivität von GDF11 erhöht ist/sind, und
wobei die Dosismenge des Inhibitors des Aktivin-Rezeptortyps II verringert wird, wenn das Niveau und/oder die Aktivität von GDF11 über das normale Niveau und/oder die normale Aktivität von GDF11 verringert ist/sind.

4. Inhibitor des Aktivin-Rezeptortyps II zur Verwendung nach einem der Ansprüche 1-3, wobei das Verabreichen zu einer verringerten Zellenzahl später basophiler und polychromatischer Erythroblasten in dem Patienten führt.

5. Inhibitor des Aktivin-Rezeptortyps II zur Verwendung nach einem der Ansprüche 1-4, wobei das Niveau und/oder die Aktivität von GDF11 der Proteingehalt von GDF11 ist/sind; oder wobei das Niveau und/oder die Aktivität von GDF11 die mRNA-Konzentration von GDF11 ist/sind.

6. Inhibitor des Aktivin-Rezeptortyps II zur Verwendung nach einem der Ansprüche 1-5, wobei das Niveau und/oder die Aktivität von GDF 11 über das normale Niveau und/oder die normale Aktivität von GDF11 erhöht ist/sind, wenn dieses/diese um mindestens 25 %, mindestens 50 %, mindestens 75 %, mindestens 100 % oder mindestens 200 % über das normale Niveau und/oder die normale Aktivität von GDF11 erhöht ist/sind.

7. Inhibitor des Aktivin-Rezeptortyps II zur Verwendung nach einem der Ansprüche 1-5, wobei das Niveau und/oder die Aktivität von GDF 11 über das normale Niveau und/oder die normale Aktivität von GDF11 erhöht ist/sind, wenn dieses/diese um mindestens das 2-fache über das normale Niveau und/oder die normale Aktivität von GDF11 erhöht ist/sind.

8. Inhibitor des Aktivin-Rezeptortyps II zur Verwendung nach einem der Ansprüche 3-5, wobei das Niveau und/oder die Aktivität von GDF 11 über das normale Niveau und/oder die normale Aktivität von GDF11 verringert ist/sind, wenn dieses/diese um mindestens 25 %, mindestens 50 %, mindestens 75 % oder mindestens 90 % über das normale Niveau und/oder die normale Aktivität von GDF11 verringert ist/sind.

9. Inhibitor des Aktivin-Rezeptortyps II zur Verwendung nach einem der Ansprüche 3-5, wobei das Niveau und/oder die Aktivität von GDF 11 über das normale Niveau und/oder die normale Aktivität von GDF11 verringert ist/sind, wenn dieses/diese um mindestens das 2-fache über das normale Niveau und/oder die normale Aktivität von GDF11 verringert ist/sind.

10. Inhibitor des Aktivin-Rezeptortyps II zur Verwendung nach einem der Ansprüche 1-9, wobei das normale Niveau und/oder die normale Aktivität von GDF11 ein durchschnittliches Niveau und/oder eine durchschnittliche Aktivität von GDF11 in einer Probe von demselben Gewebe von einem oder mehreren gesunden Subjekten derselben Alterskategorie ist/sind.

11. Inhibitor des Aktivin-Rezeptortyps II zur Verwendung nach einem der Ansprüche 1-10, wobei das Gewebe Serum, Knochenmark, Leber oder Milz ist.

12. Inhibitor des Aktivin-Rezeptortyps II zur Verwendung nach einem der Ansprüche 1-11, wobei das Subjekt menschlich ist.

13. Inhibitor des Aktivin-Rezeptortyps II zur Verwendung nach einem der Ansprüche 1-12, wobei der Fc-Abschnitt eines Antikörpers ein Fc-Abschnitt eines IgG1-Antikörpers ist.

## Revendications

1. Inhibiteur du récepteur de type II de l'activine pour l'utilisation dans un procédé pour le traitement de l'anémie, de la bêta-thalassémie, ou pour augmenter les taux de globules rouges ou d'érythroblastes orthochromatiques (Ery-C) chez un sujet qui en a besoin, dans lequel le procédé comprend:
(a) l'évaluation du taux et/ou de l'activité de GDF11 dans un échantillon de tissu du sujet, et
(b) si le taux et/ou l'activité de GDF11 sont élevés par rapport au taux normal et/ou à l'activité normale de GDF11, l'administration d'un inhibiteur du récepteur de type II de l'activine au sujet,
dans lequel l'inhibiteur du récepteur de type II de l'activine est un polypeptide comprenant une séquence d'acides aminés sélectionnée à partir du groupe constitué par:
| | |
|---|---|
| a | à 90 % identique à SEQ ID N°: 2; |
| b | à 95 % identique à SEQ ID N°: 2; |
| c | à 98 % identique à SEQ ID N°: 2; |
| d | SEQ ID N°: 2; |
| e | à 90 % identique à SEQ ID N°: 3; |
| f | à 95 % identique à SEQ ID N°: 3; |
| g | à 98 % identique à SEQ ID N°: 3; |
| h | SEQ ID N°: 3; |
| i | à 90 % identique à SEQ ID N°: 6; |
| j | à 95 % identique à SEQ ID N°: 6; |
| k | à 98 % identique à SEQ ID N°: 6; |
| 1 | SEQ ID N°: 6; |
| m | à 90 % identique à SEQ ID N°: 7; |
| n | à 95 % identique à SEQ ID N°: 7; |
| o | à 98 % identique à SEQ ID N°: 7; |
| p | SEQ ID N°: 7; |
| q | à 90 % identique à SEQ ID N°: 12; |
| r | à 95 % identique à SEQ ID N°: 12; |
| s | à 98 % identique à SEQ ID N°: 12; |
| t | SEQ ID N°: 12; |
| u | à 90 % identique à SEQ ID N°: 17; |
| v | à 95 % identique à SEQ ID N°: 17; |
| w | à 98 % identique à SEQ ID N°: 17; |
| x | SEQ ID N°: 17; |
| y | à 90 % identique à SEQ ID N°: 20; |
| z | à 95 % identique à SEQ ID N°: 20; |
| aa | à 98 % identique à SEQ ID N°: 20; |
| bb | SEQ ID N°: 20; |
| cc | à 90 % identique à SEQ ID N°: 21; |
| dd | à 95 % identique à SEQ ID N°: 21; |
| ee | à 98 % identique à SEQ ID N°: 21; |
| ff | SEQ ID N°: 21; |
| gg | à 90 % identique à SEQ ID N°: 22; |
| hh | à 95 % identique à SEQ ID N°: 22; |
| ii | à 98 % identique à SEQ ID N°: 22; |
| jj | SEQ ID N°: 22; |
| kk | à 90 % identique à SEQ ID N°: 23; |
| ll | à 95 % identique à SEQ ID N°: 23; |
| mm | à 98 % identique à SEQ ID N°: 23; |
| nn | SEQ ID N°: 23; |
| oo | à 90 % identique à SEQ ID N°: 24; |
| pp | à 95 % identique à SEQ ID N°: 24; |
| qq | à 98 % identique à SEQ ID N°: 24; |
| rr | SEQ ID N°: 24; |
| ss | à 90 % identique à SEQ ID N°: 25; |
| tt | à 95 % identique à SEQ ID N°: 25; |
| uu | à 98 % identique à SEQ ID N°: 25; et |
| vv | SEQ ID N°: 25; |
ou
dans lequel l'inhibiteur du récepteur de type II de l'activine est un polypeptide comprenant le domaine extracellulaire du récepteur de type IIA de l'activine lié au fragment Fc d'un anticorps;
ou
dans lequel l'inhibiteur du récepteur de type II de l'activine est un polypeptide comprenant le domaine extracellulaire du récepteur de type IIB de l'activine lié au fragment Fc d'un anticorps.

2. Inhibiteur du récepteur de type II de l'activine pour l'utilisation dans un procédé pour le traitement de l'anémie, de la bêta-thalassémie, ou pour augmenter les taux de globules rouges ou d'érythroblastes orthochromatiques (Ery-C) chez un sujet qui en a besoin, dans lequel le procédé comprend:
(a) l'administration d'un inhibiteur du récepteur de type II de l'activine, et
(b) la surveillance du taux et/ou de l'activité de GDF11 dans un échantillon de tissu du sujet,
dans lequel l'inhibiteur du récepteur de type II de l'activine est un polypeptide comprenant une séquence d'acides aminés sélectionnée à partir du groupe constitué par:
| | |
|---|---|
| a | à 90 % identique à SEQ ID N°: 2; |
| b | à 95 % identique à SEQ ID N°: 2; |
| c | à 98 % identique à SEQ ID N°: 2; |
| d | SEQ ID N°: 2; |
| e | à 90 % identique à SEQ ID N°: 3; |
| f | à 95 % identique à SEQ ID N°: 3; |
| g | à 98 % identique à SEQ ID N°: 3; |
| h | SEQ ID N°: 3; |
| i | à 90 % identique à SEQ ID N°: 6; |
| j | à 95 % identique à SEQ ID N°: 6; |
| k | à 98 % identique à SEQ ID N°: 6; |
| l | SEQ ID N°: 6; |
| m | à 90 % identique à SEQ ID N°: 7; |
| n | à 95 % identique à SEQ ID N°: 7; |
| o | à 98 % identique à SEQ ID N°: 7; |
| p | SEQ ID N°: 7; |
| q | à 90 % identique à SEQ ID N°: 12; |
| r | à 95 % identique à SEQ ID N°: 12; |
| s | à 98 % identique à SEQ ID N°: 12; |
| t | SEQ ID N°: 12; |
| u | à 90 % identique à SEQ ID N°: 17; |
| v | à 95 % identique à SEQ ID N°: 17; |
| w | à 98 % identique à SEQ ID N°: 17; |
| x | SEQ ID N°: 17; |
| y | à 90 % identique à SEQ ID N°: 20; |
| z | à 95 % identique à SEQ ID N°: 20; |
| aa | à 98 % identique à SEQ ID N°: 20; |
| bb | SEQ ID N°: 20; |
| cc | à 90 % identique à SEQ ID N°: 21; |
| dd | à 95 % identique à SEQ ID N°: 21 ; |
| ee | à 98 % identique à SEQ ID N°: 21; |
| ff | SEQ ID N°: 21; |
| gg | à 90 % identique à SEQ ID N°: 22; |
| hh | à 95 % identique à SEQ ID N°: 22; |
| ii | à 98 % identique à SEQ ID N°: 22; |
| jj | SEQ ID N°: 22; |
| kk | à 90 % identique à SEQ ID N°: 23; |
| ll | à 95 % identique à SEQ ID N°: 23; |
| mm | à 98 % identique à SEQ ID N°: 23; |
| nn | SEQ ID N°: 23; |
| oo | à 90 % identique à SEQ ID N°: 24; |
| pp | à 95 % identique à SEQ ID N°: 24; |
| qq | à 98 % identique à SEQ ID N°: 24; |
| rr | SEQ ID N°: 24; |
| ss | à 90 % identique à SEQ ID N°: 25; |
| tt | à 95 % identique à SEQ ID N°: 25; |
| uu | à 98 % identique à SEQ ID N°: 25; et |
| vv | SEQ ID N°: 25; |
ou
dans lequel l'inhibiteur du récepteur de type II de l'activine est un polypeptide comprenant le domaine extracellulaire du récepteur de type IIA de l'activine lié au fragment Fc d'un anticorps;
ou
dans lequel l'inhibiteur du récepteur de type II de l'activine est un polypeptide comprenant le domaine extracellulaire du récepteur de type IIB de l'activine lié au fragment Fc d'un anticorps.

3. Inhibiteur du récepteur de type II de l'activine pour l'utilisation selon la revendication 2, dans lequel le procédé comprend:
(a) l'administration d'une posologie d'un inhibiteur du récepteur de type II de l'activine,
(b) l'évaluation du taux et/ou de l'activité de GDF11 dans un échantillon de tissu du sujet, et
(c) l'ajustement de la posologie de l'inhibiteur du récepteur de type II de l'activine,
dans lequel, si le taux et/ou l'activité de GDF11 sont élevés par rapport au taux normal et/ou à l'activité normale de GDF11, la posologie de l'inhibiteur du récepteur de type II de l'activine est augmentée, et
dans lequel, si le taux et/ou l'activité de GDF11 sont diminués par rapport au taux normal et/ou à l'activité normale de GDF11, la posologie de l'inhibiteur du récepteur de type II de l'activine est diminuée.

4. Inhibiteur du récepteur de type II de l'activine pour l'utilisation selon l'une quelconque des revendications 1 à 3, dans lequel ladite administration se traduit par un nombre de cellules en baisse des érythroblastes basophiles et polychromes tardifs chez le patient.

5. Inhibiteur du récepteur de type II de l'activine pour l'utilisation selon l'une quelconque des revendications 1 à 4, dans lequel le taux et/ou l'activité de GDF11 sont le taux protéique de GDF11; ou dans lequel le taux et/ou l'activité de GDF11 sont le taux d'ARNm de GDF11.

6. Inhibiteur du récepteur de type II de l'activine pour l'utilisation selon l'une quelconque des revendications 1 à 5, dans lequel le taux et/ou l'activité de GDF11 sont élevés par rapport au taux normal et/ou à l'activité normale de GDF11 lorsqu'ils sont élevés d'au moins 25 %, d'au moins 50 %, d'au moins 75 %, d'au moins 100 % ou d'au moins 200 % par rapport au taux normal et/ou à l'activité normale de GDF11.

7. Inhibiteur du récepteur de type II de l'activine pour l'utilisation selon l'une quelconque des revendications 1 à 5, dans lequel le taux et/ou l'activité de GDF11 sont élevés par rapport au taux normal et/ou à l'activité normale de GDF11 lorsqu'ils sont élevés d'au moins 2 fois par rapport au taux normal et/ou à l'activité normale de GDF11.

8. Inhibiteur du récepteur de type II de l'activine pour l'utilisation selon l'une quelconque des revendications 3 à 5, dans lequel le taux et/ou l'activité de GDF11 sont diminués par rapport au taux normal et/ou à l'activité normale de GDF11 lorsqu'ils sont diminués d'au moins 25 %, d'au moins 50 %, d'au moins 75 % ou d'au moins 90 % par rapport au taux normal et/ou à l'activité normale de GDF11.

9. Inhibiteur du récepteur de type II de l'activine pour l'utilisation selon l'une quelconque des revendications 3 à 5, dans lequel le taux et/ou l'activité de GDF11 sont diminués par rapport au taux normal et/ou à l'activité normale de GDF11 lorsqu'ils sont diminués d'au moins 2 fois par rapport au taux normal et/ou à l'activité normale de GDF11.

10. Inhibiteur du récepteur de type II de l'activine pour l'utilisation selon l'une quelconque des revendications 1 à 9, dans lequel le taux normal et/ou l'activité normale de GDF11 sont un taux moyen et/ou une activité moyenne de GDF11 dans un échantillon issu du même tissu d'un ou plusieurs sujets en bonne santé dans la même tranche d'âge.

11. Inhibiteur du récepteur de type II de l'activine pour l'utilisation selon l'une quelconque des revendications 1 à 10, dans lequel le tissu est le sérum, la moelle osseuse, le foie ou la rate.

12. Inhibiteur du récepteur de type II de l'activine pour l'utilisation selon l'une quelconque des revendications 1 à 11, dans lequel le sujet est humain.

13. Inhibiteur du récepteur de type II de l'activine pour l'utilisation selon l'une quelconque des revendications 1 à 12, dans lequel le fragment Fc d'un anticorps est le fragment Fc d'un anticorps IgGl.
